# EUROPEAN PATENT APPLICATION

(11) **EP 2 700 714 A1**
(43) Date of publication of application: **26.02.2014**
(21) Application number: 12181085.7
(22) Date of filing: 20.08.2012
(51) Int. Cl.: C12P 7/06, C12P 7/16, C12N 15/10, C12N 9/00

(54) **Cell-free and minimized metabolic reaction cascades for the production of chemicals**

(71) Applicant: Clariant Produkte (Deutschland) GmbH, 65929 Frankfurt am Main (DE)
(72) Inventor: Kraus, Michael, 82178 Puchheim (DE); Koltermann, André, 81477 München (DE); Kettling, Ulrich, 81477 München (DE); Garbe, Daniel, 85774 Unterföhring (DE); Brück, Thomas, 85452 Eichenried (DE); Guterl, Jan-Karl, 93053 Regensburg (DE); Sieber, Volker, 85405 Nandlstadt (DE)
(74) Representative: HOFFMANN EITLE

(57) **Abstract**

Provided are enzymatic processes for the production of chemicals from carbon sources. In particular, a process for the production of a target chemical is disclosed using a cell-free enzyme system that converts carbohydrate sources to the intermediate pyruvate and subsequently the intermediate pyruvate to the target chemical.

SEQUENCE LISTING

<110>  Clariant Produkte (Deutschland) GmbH

<120>  Cell-free and minimized metabolic reaction cascades for the
       production of chemicals

<130>  BT-28-I

<160>  69

<170>  PatentIn version 3.5

<210>  1
<211>  1146
<212>  DNA
<213>  Artificial Sequence

<220>
<223>  Glucose dehydrogenase (EC 1.1.1.47), Sulfolobus solfataricus
       DNA-sequence including C-terminal His-Tag

<400>  1
atgaaagcca ttattgtgaa acctccgaat gccggtgttc aggttaaaga tgtggatgaa      60

aaaaaactgg atagctatgg caaaattaaa attcgcacca tttataatgg tatttgcggc     120

accgatcgtg aaattgtgaa tggtaaactg accctgagca ccctgccgaa aggtaaagat     180

tttctggtgc tgggtcatga agcaattggt gttgtggaag aaagctatca tggttttagc     240

cagggtgatc tggttatgcc ggttaatcgt cgtggttgtg gtatttgtcg taattgtctg     300

gttggtcgtc cggatttttg tgaaaccggt gaatttggtg aagccggtat tcataaaatg     360

gatggcttta tgcgtgaatg gtggtatgat gatccgaaat atctggtgaa aattccgaaa     420

agcattgaag atattggtat tctggcacag ccgctggcag atattgaaaa atccattgaa     480

gaaattctgg aagtgcagaa acgtgttccg gtttggacct gtgatgatgg caccctgaat     540

tgtcgtaaag ttctggttgt tggcaccggt ccgattggtg ttctgtttac cctgctgttt     600

cgtacctatg gtctggaagt ttggatggca aatcgtcgtg aaccgaccga agttgaacag     660

accgttattg aagaaaccaa aaccaattat tataatagca gcaatggcta tgataaactg     720

aaagatagcg tgggcaaatt tgatgtgatt attgatgcaa ccggtgccga tgttaatatt     780

ctgggcaatg ttattccgct gctgggtcgt aatggtgttc tgggtctgtt tggttttagc     840

acctctggta gcgttccgct ggattataaa accctgcagg aaattgttca taccaataaa     900

accattattg gcctggtgaa tggtcagaaa ccgcattttc agcaggcagt tgttcatctg     960

gcaagctgga aaaccctgta tccgaaagca gcaaaaatgc tgattaccaa aaccgtgagc    1020

attaatgatg aaaaagaact gctgaaagtg ctgcgtgaaa aagaacatgg cgaaatcaaa    1080

attcgtattc tgtgggaaag cgggagacct gtgctgggca gcagccacca ccaccaccac    1140

cactaa                                                               1146

<210>  2

<211>  381
<212>  PRT
<213>  Artificial Sequence

<220>
<223>  Glucose dehydrogenase (EC 1.1.1.47), Sulfolobus solfataricus
       Protein sequence including C-terminal His-Tag

<400>  2

Met Lys Ala Ile Ile Val Lys Pro Pro Asn Ala Gly Val Gln Val Lys
1               5                   10                  15

Asp Val Asp Glu Lys Lys Leu Asp Ser Tyr Gly Lys Ile Lys Ile Arg
            20                  25                  30

Thr Ile Tyr Asn Gly Ile Cys Gly Thr Asp Arg Glu Ile Val Asn Gly
        35                  40                  45

Lys Leu Thr Leu Ser Thr Leu Pro Lys Gly Lys Asp Phe Leu Val Leu
    50                  55                  60

Gly His Glu Ala Ile Gly Val Val Glu Glu Ser Tyr His Gly Phe Ser
65                  70                  75                  80

Gln Gly Asp Leu Val Met Pro Val Asn Arg Arg Gly Cys Gly Ile Cys
                85                  90                  95

Arg Asn Cys Leu Val Gly Arg Pro Asp Phe Cys Glu Thr Gly Glu Phe
            100                 105                 110

Gly Glu Ala Gly Ile His Lys Met Asp Gly Phe Met Arg Glu Trp Trp
        115                 120                 125

Tyr Asp Asp Pro Lys Tyr Leu Val Lys Ile Pro Lys Ser Ile Glu Asp
    130                 135                 140

Ile Gly Ile Leu Ala Gln Pro Leu Ala Asp Ile Glu Lys Ser Ile Glu
145                 150                 155                 160

Glu Ile Leu Glu Val Gln Lys Arg Val Pro Val Trp Thr Cys Asp Asp
                165                 170                 175

Gly Thr Leu Asn Cys Arg Lys Val Leu Val Val Gly Thr Gly Pro Ile
            180                 185                 190

Gly Val Leu Phe Thr Leu Leu Phe Arg Thr Tyr Gly Leu Glu Val Trp
        195                 200                 205

Met Ala Asn Arg Arg Glu Pro Thr Glu Val Glu Gln Thr Val Ile Glu
    210                 215                 220

Glu Thr Lys Thr Asn Tyr Tyr Asn Ser Ser Asn Gly Tyr Asp Lys Leu
225                 230                 235                 240

Lys Asp Ser Val Gly Lys Phe Asp Val Ile Ile Asp Ala Thr Gly Ala
                245                 250                 255

Asp Val Asn Ile Leu Gly Asn Val Ile Pro Leu Leu Gly Arg Asn Gly
            260                 265                 270

Val Leu Gly Leu Phe Gly Phe Ser Thr Ser Gly Ser Val Pro Leu Asp
        275                 280                 285

Tyr Lys Thr Leu Gln Glu Ile Val His Thr Asn Lys Thr Ile Ile Gly
    290                 295                 300

Leu Val Asn Gly Gln Lys Pro His Phe Gln Gln Ala Val Val His Leu
305                 310                 315                 320

Ala Ser Trp Lys Thr Leu Tyr Pro Lys Ala Ala Lys Met Leu Ile Thr
                325                 330                 335

Lys Thr Val Ser Ile Asn Asp Glu Lys Glu Leu Leu Lys Val Leu Arg
            340                 345                 350

Glu Lys Glu His Gly Glu Ile Lys Ile Arg Ile Leu Trp Glu Ser Gly
        355                 360                 365

Arg Pro Val Leu Gly Ser Ser His His His His His His
    370                 375                 380

<210>  3
<211>  1689
<212>  DNA
<213>  Sulfolobus solfataricus

<400>  3
atgcctgcaa aactgaatag cccgagccgt tatcatggta tttataatgc accgcatcgt      60

gcatttctgc gtagcgttgg tctgaccgat gaagaaattg gtaaaccgct ggttgcaatt     120

gccaccgcat ggtctgaagc cggtccgtgt aattttcata ccctggcact ggcacgtgtt     180

gcaaaagaag gcaccaaaga agccggtctg tctccgctgg catttccgac catggttgtg     240

aatgataata ttggcatggg tagcgaaggt atgcgttata gcctggttag ccgtgatctg     300

attgcagata tggttgaagc acagtttaat gcccatgcat ttgatggtct ggttggtatt     360

ggtggttgtg ataaaaccac accgggtatt ctgatggcaa tggcacgtct gaatgttccg     420

agcatttata tttatggtgg tagcgcagaa ccgggttatt ttatgggtaa acgcctgacc     480

attgaagatg ttcatgaagc cattggtgca tatctggcaa aacgcattac cgaaaatgaa     540

ctgtatgaaa ttgaaaaacg tgcacatccg accctgggca cctgtagcgg tctgtttacc 600

gcaaatacca tgggtagcat gagcgaagca ctgggtatgg cactgcctgg tagcgcatct 660

ccgaccgcaa ccagcagccg tcgtgttatg tatgttaaag aaaccggtaa agccctgggt 720

agcctgattg aaaatggcat taaaagccgt gaaattctga cctttgaagc ctttgaaaat 780

gcaattacaa ccctgatggc gatgggtggt agcaccaatg cagttctgca tctgctggca 840

attgcttatg aagccggtgt taaactgacc ctggatgatt ttaatcgcat tagcaaacgc 900

accccgtata ttgcaagcat gaaaccgggt ggtgattatg ttatggccga tctggatgaa 960

gttggtggtg ttccggttgt tctgaaaaaa ctgctggatg ccggtctgct gcatggtgat 1020

gttctgaccg ttaccggtaa aaccatgaaa cagaatctgg aacagtataa atatccgaat 1080

gtgccgcata gccatattgt tcgtgatgtg aaaaatccga ttaaaccgcg tggtggtatt 1140

gttattctga aaggtagcct ggcaccggaa ggtgcagtta ttaaagttgc agccaccaat 1200

gtggttaaat ttgaaggcaa agccaaagtg tataatagcg aagatgatgc ctttaaaggt 1260

gttcagagcg gtgaagttag cgaaggtgaa gtggtgatta ttcgctatga aggtccgaaa 1320

ggtgcaccgg gtatgccgga aatgctgcgc gttaccgcag cgattatggg tgccggtctg 1380

aataatgttg cactggttac cgatggtcgt tttagcggtg caacccgtgg tccgatggtt 1440

ggtcatgttg caccggaagc aatggttggt ggtccgattg caattgttga agatggcgat 1500

accattgtga ttgatgtgga aagcgaacgt ctggatctga aactgagcga agaagaaatt 1560

aaaaatcgcc tgaaacgttg gagcccgccg tcaccgcgtt ataaaagcgg tctgctggca 1620

aaatatgcaa gcctggtttc tcaggcaagc atgggtgcag ttacccgtcc ggcaagagac 1680

cttaattaa 1689

<210> 4
<211> 558
<212> PRT
<213> Sulfolobus solfataricus

<400> 4

Met Pro Ala Lys Leu Asn Ser Pro Ser Arg Tyr His Gly Ile Tyr Asn
1               5                   10                  15

Ala Pro His Arg Ala Phe Leu Arg Ser Val Gly Leu Thr Asp Glu Glu
            20                  25                  30

Ile Gly Lys Pro Leu Val Ala Ile Ala Thr Ala Trp Ser Glu Ala Gly
        35                  40                  45

Pro Cys Asn Phe His Thr Leu Ala Leu Ala Arg Val Ala Lys Glu Gly
    50                  55                  60

Thr Lys Glu Ala Gly Leu Ser Pro Leu Ala Phe Pro Thr Met Val Val

65                  70                  75                  80

Asn Asp Asn Ile Gly Met Gly Ser Glu Gly Met Arg Tyr Ser Leu Val
                85                  90                  95

Ser Arg Asp Leu Ile Ala Asp Met Val Glu Ala Gln Phe Asn Ala His
            100                 105                 110

Ala Phe Asp Gly Leu Val Gly Ile Gly Gly Cys Asp Lys Thr Thr Pro
        115                 120                 125

Gly Ile Leu Met Ala Met Ala Arg Leu Asn Val Pro Ser Ile Tyr Ile
    130                 135                 140

Tyr Gly Gly Ser Ala Glu Pro Gly Tyr Phe Met Gly Lys Arg Leu Thr
145                 150                 155                 160

Ile Glu Asp Val His Glu Ala Ile Gly Ala Tyr Leu Ala Lys Arg Ile
                165                 170                 175

Thr Glu Asn Glu Leu Tyr Glu Ile Glu Lys Arg Ala His Pro Thr Leu
            180                 185                 190

Gly Thr Cys Ser Gly Leu Phe Thr Ala Asn Thr Met Gly Ser Met Ser
        195                 200                 205

Glu Ala Leu Gly Met Ala Leu Pro Gly Ser Ala Ser Pro Thr Ala Thr
    210                 215                 220

Ser Ser Arg Arg Val Met Tyr Val Lys Glu Thr Gly Lys Ala Leu Gly
225                 230                 235                 240

Ser Leu Ile Glu Asn Gly Ile Lys Ser Arg Glu Ile Leu Thr Phe Glu
                245                 250                 255

Ala Phe Glu Asn Ala Ile Thr Thr Leu Met Ala Met Gly Gly Ser Thr
            260                 265                 270

Asn Ala Val Leu His Leu Leu Ala Ile Ala Tyr Glu Ala Gly Val Lys
        275                 280                 285

Leu Thr Leu Asp Asp Phe Asn Arg Ile Ser Lys Arg Thr Pro Tyr Ile
    290                 295                 300

Ala Ser Met Lys Pro Gly Gly Asp Tyr Val Met Ala Asp Leu Asp Glu
305                 310                 315                 320

Val Gly Gly Val Pro Val Val Leu Lys Lys Leu Leu Asp Ala Gly Leu
                325                 330                 335

Leu His Gly Asp Val Leu Thr Val Thr Gly Lys Thr Met Lys Gln Asn
            340                 345                 350

Leu Glu Gln Tyr Lys Tyr Pro Asn Val Pro His Ser His Ile Val Arg
        355                 360                 365

Asp Val Lys Asn Pro Ile Lys Pro Arg Gly Gly Ile Val Ile Leu Lys
    370                 375                 380

Gly Ser Leu Ala Pro Glu Gly Ala Val Ile Lys Val Ala Ala Thr Asn
385                 390                 395                 400

Val Val Lys Phe Glu Gly Lys Ala Lys Val Tyr Asn Ser Glu Asp Asp
                405                 410                 415

Ala Phe Lys Gly Val Gln Ser Gly Glu Val Ser Glu Gly Glu Val Val
            420                 425                 430

Ile Ile Arg Tyr Glu Gly Pro Lys Gly Ala Pro Gly Met Pro Glu Met
        435                 440                 445

Leu Arg Val Thr Ala Ala Ile Met Gly Ala Gly Leu Asn Asn Val Ala
    450                 455                 460

Leu Val Thr Asp Gly Arg Phe Ser Gly Ala Thr Arg Gly Pro Met Val
465                 470                 475                 480

Gly His Val Ala Pro Glu Ala Met Val Gly Gly Pro Ile Ala Ile Val
                485                 490                 495

Glu Asp Gly Asp Thr Ile Val Ile Asp Val Glu Ser Glu Arg Leu Asp
            500                 505                 510

Leu Lys Leu Ser Glu Glu Glu Ile Lys Asn Arg Leu Lys Arg Trp Ser
        515                 520                 525

Pro Pro Ser Pro Arg Tyr Lys Ser Gly Leu Leu Ala Lys Tyr Ala Ser
    530                 535                 540

Leu Val Ser Gln Ala Ser Met Gly Ala Val Thr Arg Pro Ala
545                 550                 555

<210>  5
<211>  867
<212>  DNA
<213>  Sulfolobus acidocaldarius

<400>  5
atggaaatta ttagcccgat tattaccccg tttgataaac agggtaaagt gaatgttgat        60

gccctgaaaa cccatgcaaa aaatctgctg gaaaaaggca ttgatgccat ttttgttaat 120

ggcaccaccg gtctgggtcc ggcactgagc aaagatgaaa aacgccagaa tctgaatgca 180

ctgtatgatg tgacccataa actgattttt caggtgggta gcctgaatct gaatgatgtt 240

atggaactgg tgaaatttag caatgaaatg gatattctgg gtgttagcag ccatagcccg 300

tattattttc cgcgtctgcc ggaaaaattt ctggccaaat attatgaaga aattgcccgt 360

attagcagcc attccctgta tatttataat tatccggcag ccaccggtta tgatattcca 420

ccgagcattc tgaaaagcct gccggtgaaa ggtattaaag ataccaatca ggatctggca 480

catagcctgg aatacaaact gaatctgccg ggtgtgaaag tttataatgg cagcaatacc 540

ctgatttatt atagcctgct gagcctggat ggtgttgttg caagctttac caatttcatt 600

ccggaagtga ttgtgaaaca gcgcgatctg attaaacagg gcaaactgga tgatgcactg 660

cgtctgcagg aactgattaa tcgtctggca gatattctgc gtaaatatgg tagcattagc 720

gccatttatg tgctggtgaa tgaatttcag ggttatgatg ttggttatcc gcgtccgccg 780

atttttccgc tgaccgatga agaagcactg agcctgaaac gtgaaattga accgctgaaa 840

cgcaaaattc aggaactggt tcattaa 867

<210> 6
<211> 288
<212> PRT
<213> Sulfolobus acidocaldarius

<400> 6

Met Glu Ile Ile Ser Pro Ile Ile Thr Pro Phe Asp Lys Gln Gly Lys
1               5                   10                  15

Val Asn Val Asp Ala Leu Lys Thr His Ala Lys Asn Leu Leu Glu Lys
            20                  25                  30

Gly Ile Asp Ala Ile Phe Val Asn Gly Thr Thr Gly Leu Gly Pro Ala
        35                  40                  45

Leu Ser Lys Asp Glu Lys Arg Gln Asn Leu Asn Ala Leu Tyr Asp Val
    50                  55                  60

Thr His Lys Leu Ile Phe Gln Val Gly Ser Leu Asn Leu Asn Asp Val
65                  70                  75                  80

Met Glu Leu Val Lys Phe Ser Asn Glu Met Asp Ile Leu Gly Val Ser
                85                  90                  95

Ser His Ser Pro Tyr Tyr Phe Pro Arg Leu Pro Glu Lys Phe Leu Ala
            100                 105                 110

Lys Tyr Tyr Glu Glu Ile Ala Arg Ile Ser Ser His Ser Leu Tyr Ile
        115                 120                 125

Tyr Asn Tyr Pro Ala Ala Thr Gly Tyr Asp Ile Pro Pro Ser Ile Leu
    130                 135                 140

Lys Ser Leu Pro Val Lys Gly Ile Lys Asp Thr Asn Gln Asp Leu Ala
145                 150                 155                 160

His Ser Leu Glu Tyr Lys Leu Asn Leu Pro Gly Val Lys Val Tyr Asn
                165                 170                 175

Gly Ser Asn Thr Leu Ile Tyr Tyr Ser Leu Leu Ser Leu Asp Gly Val
            180                 185                 190

Val Ala Ser Phe Thr Asn Phe Ile Pro Glu Val Ile Val Lys Gln Arg
        195                 200                 205

Asp Leu Ile Lys Gln Gly Lys Leu Asp Asp Ala Leu Arg Leu Gln Glu
    210                 215                 220

Leu Ile Asn Arg Leu Ala Asp Ile Leu Arg Lys Tyr Gly Ser Ile Ser
225                 230                 235                 240

Ala Ile Tyr Val Leu Val Asn Glu Phe Gln Gly Tyr Asp Val Gly Tyr
                245                 250                 255

Pro Arg Pro Pro Ile Phe Pro Leu Thr Asp Glu Glu Ala Leu Ser Leu
            260                 265                 270

Lys Arg Glu Ile Glu Pro Leu Lys Arg Lys Ile Gln Glu Leu Val His
        275                 280                 285

<210>  7
<211>  1527
<212>  DNA
<213>  Artificial Sequence

<220>
<223>  Aldehyde Dehydrogenase (EC 1.2.1.3), Thermoplasma acidophilum,
       wildtype enzyme DNA-sequence including C-terminal His-Tag

<400>  7
atggatacca aactgtatat tgatggccag tgggttaata gcagcagcgg taaaaccgtt      60

gataaatatt ctccggttac cggtcaggtt attggtcgtt ttgaagcagc aacccgtgat     120

gatgttgatc gtgcaattga tgcagcagaa gatgcatttt gggcctggaa tgatctgggt     180

agcgttgaac gcagcaaaat tatttatcgt gccaaagaac tgattgaaaa aaatcgtgcc     240

gaactggaaa atattattat ggaagaaaat ggcaaaccgg tgaaagaagc caaagaagaa     300

gtcgacggcg tcattgatca gattcagtat tatgcagaat gggcacgtaa actgaatggt     360

gaagttgttg aaggcaccag cagccatcgt aaaatttttc agtataaagt gccgtatggt 420

attgttgttg cactgacccc gtggaatttt ccggcaggca tggttgcccg taaactggca 480

ccggcactgc tgaccggtaa taccgttgtt ctgaaaccga gcagcgatac accgggtagc 540

gcagaatgga ttgtgcgtaa atttgttgaa gccggtgttc cgaaaggtgt gctgaatttt 600

attaccggtc gtggtagcga aattggcgat tacattgtgg aacataaaaa agtcaatctg 660

attaccatga ccggtagcac cgcaacaggt cagcgcatta tgcagaaagc aagcgcaaat 720

atggcaaaac tgattctgga actgggtggt aaagcaccgt ttatggtttg gaaagatgcc 780

gatatggata atgcactgaa aaccctgctg tgggcaaaat attggaatgc cggtcagagc 840

tgtattgcag cagaacgtct gtatgtgcat gaagatattt atgatacctt tatgagccgt 900

tttgttgaac tgagccgcaa actggcactg ggtgatccga aaaatgcaga tatgggtccg 960

ctgattaata aaggtgcact gcaggcaacc agcgaaattg ttgaagaagc gaaagaatct 1020

ggcgcaaaaa ttctgtttgg tggtagccag ccgagcctga gcggtccgta tcgtaatggc 1080

tatttttttc tgccgaccat tattggtaat gcggatcaga aaagcaaaat ctttcaggaa 1140

gaaatttttg caccggttat tggtgcacgt aaaattagca gcgtggaaga aatgtatgat 1200

ctggccaatg atagcaaata tggtctggcc agctacctgt ttaccaaaga tccgaatatc 1260

atttttgaag ccagcgaacg tattcgtttt ggtgaactgt atgtgaatat gccgggtccg 1320

gaagcaagcc agggttatca caccggtttt cgtatgacag gtcaggcagg cgaaggttct 1380

aaatatggca ttagcgaata tctgaaactg aaaaatattt atgtggatta tagcggcaaa 1440

ccgctgcata ttaataccgt tcgtgatgac ctgtttcaga gcgggagacc tgtgctgggc 1500

agcagccacc accaccacca ccactaa 1527

<210> 8
<211> 508
<212> PRT
<213> Artificial Sequence

<220>
<223> Aldehyde Dehydrogenase (EC 1.2.1.3), Thermoplasma acidophilum,
      wildtype enzyme Protein sequence including C-terminal His-Tag

<400> 8

Met Asp Thr Lys Leu Tyr Ile Asp Gly Gln Trp Val Asn Ser Ser Ser
1               5                   10                  15

Gly Lys Thr Val Asp Lys Tyr Ser Pro Val Thr Gly Gln Val Ile Gly
            20                  25                  30

Arg Phe Glu Ala Ala Thr Arg Asp Asp Val Asp Arg Ala Ile Asp Ala
        35                  40                  45

Ala Glu Asp Ala Phe Trp Ala Trp Asn Asp Leu Gly Ser Val Glu Arg
    50                  55                  60

Ser Lys Ile Ile Tyr Arg Ala Lys Glu Leu Ile Glu Lys Asn Arg Ala
65                  70                  75                  80

Glu Leu Glu Asn Ile Ile Met Glu Glu Asn Gly Lys Pro Val Lys Glu
                85                  90                  95

Ala Lys Glu Glu Val Asp Gly Val Ile Asp Gln Ile Gln Tyr Tyr Ala
            100                 105                 110

Glu Trp Ala Arg Lys Leu Asn Gly Glu Val Val Glu Gly Thr Ser Ser
        115                 120                 125

His Arg Lys Ile Phe Gln Tyr Lys Val Pro Tyr Gly Ile Val Val Ala
    130                 135                 140

Leu Thr Pro Trp Asn Phe Pro Ala Gly Met Val Ala Arg Lys Leu Ala
145                 150                 155                 160

Pro Ala Leu Leu Thr Gly Asn Thr Val Val Leu Lys Pro Ser Ser Asp
                165                 170                 175

Thr Pro Gly Ser Ala Glu Trp Ile Val Arg Lys Phe Val Glu Ala Gly
            180                 185                 190

Val Pro Lys Gly Val Leu Asn Phe Ile Thr Gly Arg Gly Ser Glu Ile
        195                 200                 205

Gly Asp Tyr Ile Val Glu His Lys Lys Val Asn Leu Ile Thr Met Thr
    210                 215                 220

Gly Ser Thr Ala Thr Gly Gln Arg Ile Met Gln Lys Ala Ser Ala Asn
225                 230                 235                 240

Met Ala Lys Leu Ile Leu Glu Leu Gly Gly Lys Ala Pro Phe Met Val
                245                 250                 255

Trp Lys Asp Ala Asp Met Asp Asn Ala Leu Lys Thr Leu Leu Trp Ala
            260                 265                 270

Lys Tyr Trp Asn Ala Gly Gln Ser Cys Ile Ala Ala Glu Arg Leu Tyr
        275                 280                 285

Val His Glu Asp Ile Tyr Asp Thr Phe Met Ser Arg Phe Val Glu Leu
    290                 295                 300

Ser Arg Lys Leu Ala Leu Gly Asp Pro Lys Asn Ala Asp Met Gly Pro

305                 310                 315                 320

Leu Ile Asn Lys Gly Ala Leu Gln Ala Thr Ser Glu Ile Val Glu Glu
                325                 330                 335

Ala Lys Glu Ser Gly Ala Lys Ile Leu Phe Gly Gly Ser Gln Pro Ser
            340                 345                 350

Leu Ser Gly Pro Tyr Arg Asn Gly Tyr Phe Phe Leu Pro Thr Ile Ile
        355                 360                 365

Gly Asn Ala Asp Gln Lys Ser Lys Ile Phe Gln Glu Glu Ile Phe Ala
    370                 375                 380

Pro Val Ile Gly Ala Arg Lys Ile Ser Ser Val Glu Glu Met Tyr Asp
385                 390                 395                 400

Leu Ala Asn Asp Ser Lys Tyr Gly Leu Ala Ser Tyr Leu Phe Thr Lys
                405                 410                 415

Asp Pro Asn Ile Ile Phe Glu Ala Ser Glu Arg Ile Arg Phe Gly Glu
            420                 425                 430

Leu Tyr Val Asn Met Pro Gly Pro Glu Ala Ser Gln Gly Tyr His Thr
        435                 440                 445

Gly Phe Arg Met Thr Gly Gln Ala Gly Glu Gly Ser Lys Tyr Gly Ile
    450                 455                 460

Ser Glu Tyr Leu Lys Leu Lys Asn Ile Tyr Val Asp Tyr Ser Gly Lys
465                 470                 475                 480

Pro Leu His Ile Asn Thr Val Arg Asp Asp Leu Phe Gln Ser Gly Arg
                485                 490                 495

Pro Val Leu Gly Ser Ser His His His His His His
            500                 505

<210>  9
<211>  1527
<212>  DNA
<213>  Artificial Sequence

<220>
<223>  Aldehyde Dehydrogenase (EC 1.2.1.3), Thermoplasma acidophilum,
       variant F34M-Y399C-S405N DNA-sequence including C-terminal
       His-Tag

<400>  9
atggatacca aactgtatat tgatggccag tgggttaata gcagcagcgg taaaaccgtt        60

gataaatatt ctccggttac cggtcaggtt attggtcgta tggaagcagc aacccgtgat       120

gatgttgatc gtgcaattga tgcagcagaa gatgcatttt gggcctggaa tgatctgggt 180

agcgttgaac gcagcaaaat tatttatcgt gccaaagaac tgattgaaaa aaatcgtgcc 240

gaactggaaa atattattat ggaagaaaat ggcaaaccgg tgaaagaagc caaagaagaa 300

gtcgacggcg tcattgatca gattcagtat tatgcagaat gggcacgtaa actgaatggt 360

gaagttgttg aaggcaccag cagccatcgt aaaatttttc agtataaagt gccgtatggt 420

attgttgttg cactgacccc gtggaatttt ccggcaggca tggttgcccg taaactggca 480

ccggcactgc tgaccggtaa taccgttgtt ctgaaaccga gcagcgatac accgggtagc 540

gcagaatgga ttgtgcgtaa atttgttgaa gccggtgttc cgaaaggtgt gctgaatttt 600

attaccggtc gtggtagcga aattggcgat tacattgtgg aacataaaaa agtcaatctg 660

attaccatga ccggtagcac cgcaacaggt cagcgcatta tgcagaaagc aagcgcaaat 720

atggcaaaac tgattctgga actgggtggt aaagcaccgt ttatggtttg gaaagatgcc 780

gatatggata atgcactgaa aaccctgctg tgggcaaaat attggaatgc cggtcagagc 840

tgtattgcag cagaacgtct gtatgtgcat gaagatattt atgatacctt tatgagccgt 900

tttgttgaac tgagccgcaa actggcactg ggtgatccga aaaatgcaga tatgggtccg 960

ctgattaata aaggtgcact gcaggcaacc agcgaaattg ttgaagaagc gaaagaatct 1020

ggcgcaaaaa ttctgtttgg tggtagccag ccgagcctga gcggtccgta tcgtaatggc 1080

tatttttttc tgccgaccat tattggtaat gcggatcaga aaagcaaaat ctttcaggaa 1140

gaaatttttg caccggttat tggtgcacgt aaaattagca gcgtggaaga aatgtgtgat 1200

ctggccaatg ataataaata tggtctggcc agctacctgt ttaccaaaga tccgaatatc 1260

atttttgaag ccagcgaacg tattcgtttt ggtgaactgt atgtgaatat gccgggtccg 1320

gaagcaagcc agggttatca caccggtttt cgtatgacag gtcaggcagg cgaaggttct 1380

aaatatggca ttagcgaata tctgaaactg aaaaatattt atgtggatta tagcggcaaa 1440

ccgctgcata ttaataccgt tcgtgatgac ctgtttcaga gcgggagacc tgtgctgggc 1500

agcagccacc accaccacca ccactaa 1527

<210> 10
<211> 508
<212> PRT
<213> Artificial Sequence

<220>
<223> Aldehyde Dehydrogenase (EC 1.2.1.3), Thermoplasma acidophilum,
variant F34M-Y399C-S405N Protein sequence including C-terminal
His-Tag

<400> 10

Met Asp Thr Lys Leu Tyr Ile Asp Gly Gln Trp Val Asn Ser Ser Ser
1               5                   10                  15

Gly Lys Thr Val Asp Lys Tyr Ser Pro Val Thr Gly Gln Val Ile Gly
            20                  25                  30

Arg Met Glu Ala Ala Thr Arg Asp Asp Val Asp Arg Ala Ile Asp Ala
        35                  40                  45

Ala Glu Asp Ala Phe Trp Ala Trp Asn Asp Leu Gly Ser Val Glu Arg
    50                  55                  60

Ser Lys Ile Ile Tyr Arg Ala Lys Glu Leu Ile Glu Lys Asn Arg Ala
65                  70                  75                  80

Glu Leu Glu Asn Ile Ile Met Glu Glu Asn Gly Lys Pro Val Lys Glu
                85                  90                  95

Ala Lys Glu Glu Val Asp Gly Val Ile Asp Gln Ile Gln Tyr Tyr Ala
            100                 105                 110

Glu Trp Ala Arg Lys Leu Asn Gly Glu Val Val Glu Gly Thr Ser Ser
        115                 120                 125

His Arg Lys Ile Phe Gln Tyr Lys Val Pro Tyr Gly Ile Val Val Ala
    130                 135                 140

Leu Thr Pro Trp Asn Phe Pro Ala Gly Met Val Ala Arg Lys Leu Ala
145                 150                 155                 160

Pro Ala Leu Leu Thr Gly Asn Thr Val Val Leu Lys Pro Ser Ser Asp
                165                 170                 175

Thr Pro Gly Ser Ala Glu Trp Ile Val Arg Lys Phe Val Glu Ala Gly
            180                 185                 190

Val Pro Lys Gly Val Leu Asn Phe Ile Thr Gly Arg Gly Ser Glu Ile
        195                 200                 205

Gly Asp Tyr Ile Val Glu His Lys Lys Val Asn Leu Ile Thr Met Thr
    210                 215                 220

Gly Ser Thr Ala Thr Gly Gln Arg Ile Met Gln Lys Ala Ser Ala Asn
225                 230                 235                 240

Met Ala Lys Leu Ile Leu Glu Leu Gly Gly Lys Ala Pro Phe Met Val
                245                 250                 255

Trp Lys Asp Ala Asp Met Asp Asn Ala Leu Lys Thr Leu Leu Trp Ala
            260                 265                 270

Lys Tyr Trp Asn Ala Gly Gln Ser Cys Ile Ala Ala Glu Arg Leu Tyr
        275                 280                 285

Val His Glu Asp Ile Tyr Asp Thr Phe Met Ser Arg Phe Val Glu Leu
    290                 295                 300

Ser Arg Lys Leu Ala Leu Gly Asp Pro Lys Asn Ala Asp Met Gly Pro
305                 310                 315                 320

Leu Ile Asn Lys Gly Ala Leu Gln Ala Thr Ser Glu Ile Val Glu Glu
                325                 330                 335

Ala Lys Glu Ser Gly Ala Lys Ile Leu Phe Gly Gly Ser Gln Pro Ser
            340                 345                 350

Leu Ser Gly Pro Tyr Arg Asn Gly Tyr Phe Phe Leu Pro Thr Ile Ile
        355                 360                 365

Gly Asn Ala Asp Gln Lys Ser Lys Ile Phe Gln Glu Glu Ile Phe Ala
    370                 375                 380

Pro Val Ile Gly Ala Arg Lys Ile Ser Ser Val Glu Glu Met Cys Asp
385                 390                 395                 400

Leu Ala Asn Asp Asn Lys Tyr Gly Leu Ala Ser Tyr Leu Phe Thr Lys
                405                 410                 415

Asp Pro Asn Ile Ile Phe Glu Ala Ser Glu Arg Ile Arg Phe Gly Glu
            420                 425                 430

Leu Tyr Val Asn Met Pro Gly Pro Glu Ala Ser Gln Gly Tyr His Thr
        435                 440                 445

Gly Phe Arg Met Thr Gly Gln Ala Gly Glu Gly Ser Lys Tyr Gly Ile
    450                 455                 460

Ser Glu Tyr Leu Lys Leu Lys Asn Ile Tyr Val Asp Tyr Ser Gly Lys
465                 470                 475                 480

Pro Leu His Ile Asn Thr Val Arg Asp Asp Leu Phe Gln Ser Gly Arg
                485                 490                 495

Pro Val Leu Gly Ser Ser His His His His His His
            500                 505

<210>  11
<211>  1719
<212>  DNA
<213>  Bacillus subtilis

<400>  11
atgctgacca aagcaaccaa agaacagaaa agcctggtga aaaatcgtgg tgcagaactg      60

gttgttgatt gtctggttga acagggtgtt acccatgttt ttggtattcc gggtgcaaaa     120

attgatgcag tttttgatgc cctgcaggat aaaggtccgg aaattattgt tgcacgccat     180

gaacagaatg cagcatttat ggcacaggca gttggtcgtc tgaccggtaa accgggtgtt     240

gttctggtta ccagcggtcc gggtgcaagc aatctggcaa ccggtctgct gaccgcaaat     300

accgaaggtg atccggttgt tgcactggca ggtaatgtta ttcgtgcaga tcgtctgaaa     360

cgtacccatc agagcctgga taatgcagca ctgtttcagc cgattaccaa atattcagtt     420

gaagtgcagg atgtgaaaaa tattccggaa gcagttacca atgcctttcg tattgcaagc     480

gcaggtcagg caggcgcagc atttgttagc tttccgcagg atgttgttaa tgaagtgacc     540

aataccaaaa atgttcgtgc agttgcagca ccgaaactgg gtccggcagc agatgatgca     600

attagcgcag caattgcaaa aattcagacc gcaaaactgc cggttgttct ggtgggtatg     660

aaaggtggtc gtccggaagc aattaaagca gttcgtaaac tgctgaaaaa agttcagctg     720

ccgtttgttg aaacctatca ggcagcaggc accctgagcc gtgatctgga agatcagtat     780

tttggtcgta ttggtctgtt tcgtaatcag cctggtgatc tgctgctgga acaggcagat     840

gttgttctga ccattggtta tgatccgatt gagtatgatc cgaaattttg gaacattaat     900

ggcgatcgca ccattattca cctggatgaa attattgccg atatcgatca tgcatatcag     960

ccggatctgg aactgattgg tgatattccg agcaccatta accatattga acatgatgcc    1020

gtgaaagtgg aatttgcaga acgtgaacag aaaattctga gcgatctgaa acagtatatg    1080

catgaaggtg aacaggttcc ggcagattgg aaaagcgatc gtgcacatcc gctggaaatt    1140

gttaaagaac tgcgtaatgc cgtggatgat catgttaccg ttacctgtga tattggtagc    1200

catgcaattt ggatgagccg ttattttcgt agctatgaac cgctgaccct gatgattagc    1260

aatggtatgc agaccctggg tgttgcactg ccgtgggcaa ttggtgcaag cctggttaaa    1320

ccgggtgaaa aagttgttag cgttagcggt gatggtggtt ttctgtttag cgcaatggaa    1380

ctggaaaccg cagttcgtct gaaagcaccg attgttcata ttgtttggaa cgatagcacc    1440

tatgatatgg ttgcatttca gcagctgaaa aaatataatc gtaccagcgc agtggatttt    1500

ggcaatattg acattgtgaa atacgccgaa agctttggtg ccaccggtct gcgtgttgaa    1560

agtccggatc agctggcaga tgttctgcgt cagggtatga atgcagaagg tccggttatt    1620

attgatgttc cggttgatta tagcgataac attaatctgg ccagcgataa actgccgaaa    1680

gaatttggtg aactgatgaa aaccaaagcc ctgttataa                           1719

<210>  12
<211>  572
<212>  PRT
<213>  Bacillus subtilis

<400>  12

Met Leu Thr Lys Ala Thr Lys Glu Gln Lys Ser Leu Val Lys Asn Arg
1               5                   10                  15

Gly Ala Glu Leu Val Val Asp Cys Leu Val Glu Gln Gly Val Thr His
            20                  25                  30

Val Phe Gly Ile Pro Gly Ala Lys Ile Asp Ala Val Phe Asp Ala Leu
        35                  40                  45

Gln Asp Lys Gly Pro Glu Ile Ile Val Ala Arg His Glu Gln Asn Ala
    50                  55                  60

Ala Phe Met Ala Gln Ala Val Gly Arg Leu Thr Gly Lys Pro Gly Val
65                  70                  75                  80

Val Leu Val Thr Ser Gly Pro Gly Ala Ser Asn Leu Ala Thr Gly Leu
                85                  90                  95

Leu Thr Ala Asn Thr Glu Gly Asp Pro Val Val Ala Leu Ala Gly Asn
            100                 105                 110

Val Ile Arg Ala Asp Arg Leu Lys Arg Thr His Gln Ser Leu Asp Asn
        115                 120                 125

Ala Ala Leu Phe Gln Pro Ile Thr Lys Tyr Ser Val Glu Val Gln Asp
    130                 135                 140

Val Lys Asn Ile Pro Glu Ala Val Thr Asn Ala Phe Arg Ile Ala Ser
145                 150                 155                 160

Ala Gly Gln Ala Gly Ala Ala Phe Val Ser Phe Pro Gln Asp Val Val
                165                 170                 175

Asn Glu Val Thr Asn Thr Lys Asn Val Arg Ala Val Ala Ala Pro Lys
            180                 185                 190

Leu Gly Pro Ala Ala Asp Asp Ala Ile Ser Ala Ala Ile Ala Lys Ile
        195                 200                 205

Gln Thr Ala Lys Leu Pro Val Val Leu Val Gly Met Lys Gly Gly Arg
    210                 215                 220

Pro Glu Ala Ile Lys Ala Val Arg Lys Leu Leu Lys Lys Val Gln Leu
225                 230                 235                 240

Pro Phe Val Glu Thr Tyr Gln Ala Ala Gly Thr Leu Ser Arg Asp Leu
                245                 250                 255

Glu Asp Gln Tyr Phe Gly Arg Ile Gly Leu Phe Arg Asn Gln Pro Gly
            260                 265                 270

Asp Leu Leu Leu Glu Gln Ala Asp Val Val Leu Thr Ile Gly Tyr Asp
        275                 280                 285

Pro Ile Glu Tyr Asp Pro Lys Phe Trp Asn Ile Asn Gly Asp Arg Thr
    290                 295                 300

Ile Ile His Leu Asp Glu Ile Ile Ala Asp Ile Asp His Ala Tyr Gln
305                 310                 315                 320

Pro Asp Leu Glu Leu Ile Gly Asp Ile Pro Ser Thr Ile Asn His Ile
                325                 330                 335

Glu His Asp Ala Val Lys Val Glu Phe Ala Glu Arg Glu Gln Lys Ile
            340                 345                 350

Leu Ser Asp Leu Lys Gln Tyr Met His Glu Gly Glu Gln Val Pro Ala
        355                 360                 365

Asp Trp Lys Ser Asp Arg Ala His Pro Leu Glu Ile Val Lys Glu Leu
    370                 375                 380

Arg Asn Ala Val Asp Asp His Val Thr Val Thr Cys Asp Ile Gly Ser
385                 390                 395                 400

His Ala Ile Trp Met Ser Arg Tyr Phe Arg Ser Tyr Glu Pro Leu Thr
                405                 410                 415

Leu Met Ile Ser Asn Gly Met Gln Thr Leu Gly Val Ala Leu Pro Trp
            420                 425                 430

Ala Ile Gly Ala Ser Leu Val Lys Pro Gly Glu Lys Val Val Ser Val
        435                 440                 445

Ser Gly Asp Gly Gly Phe Leu Phe Ser Ala Met Glu Leu Glu Thr Ala
    450                 455                 460

Val Arg Leu Lys Ala Pro Ile Val His Ile Val Trp Asn Asp Ser Thr
465                 470                 475                 480

Tyr Asp Met Val Ala Phe Gln Gln Leu Lys Lys Tyr Asn Arg Thr Ser
                485                 490                 495

Ala Val Asp Phe Gly Asn Ile Asp Ile Val Lys Tyr Ala Glu Ser Phe
            500                 505                 510

Gly Ala Thr Gly Leu Arg Val Glu Ser Pro Asp Gln Leu Ala Asp Val
        515                 520                 525

Leu Arg Gln Gly Met Asn Ala Glu Gly Pro Val Ile Ile Asp Val Pro
    530                 535                 540

Val Asp Tyr Ser Asp Asn Ile Asn Leu Ala Ser Asp Lys Leu Pro Lys
545                 550                 555                 560

Glu Phe Gly Glu Leu Met Lys Thr Lys Ala Leu Leu
                565                 570

<210>  13
<211>  1062
<212>  DNA
<213>  Artificial Sequence

<220>
<223>  Ketolacid reductoisomerase (EC 1.1.1.86), Meiothermus ruber
       DNA-sequence including C-terminal His-Tag

<400>  13
atgaagattt actacgacca ggacgcagac atcggcttta tcaaagacaa gactgtggcc      60

attctgggct ttggctcgca gggccatgcc cacgccctta acctgcggga ctccggcatc     120

aaggtggtgg tggggctgcg ccccggcagc cgcaacgagg agaaggcccg taaagcgggg     180

ctcgaggtgc ttccggtagg ggaggcggtg cgcagggccg atgtggtgat gatcctgctc     240

ccggacgaga cccagggggc cgtttacaag gccgaggtgg aacccaacct gaaggaaggg     300

gctgcccttg ccttcgccca cggcttcaac atccatttcg gccagatcaa gccgcgccgc     360

gacctggacg tctggatggt ggcccccaaa ggccccggcc acctggtgcg ctcggagtac     420

gagaaaggct cgggcgtgcc ctcgctggtg gcggtctacc aggacgcctc cgggtcggcc     480

ttccccacgg cgctggccta cgccaaggcc aacggcggca cccgcgccgg caccatcgcc     540

accaccttca aggacgagac cgagaccgac ctgttcggcg agcagaccgt gctgtgcggg     600

ggcctgaccc agctcatcgc cgccggtttc gagaccctgg tggaggccgg ctatcccccc     660

gagatggcct actttgagtg cctgcacgag gtgaagctga tcgtggacct gatctacgag     720

tcaggcttcg ccgggatgcg ctactccatc tccaacaccg ccgagtacgg cgactacacc     780

cgcggcccca tggtgatcaa ccgcgaggag accaaggccc gcatgcgcga ggtgctgcgc     840

cagattcagc agggcgagtt tgcccgcgag tggatgctgg aaaacgtggt gggccagccc     900

accctgaacg ccaaccgcaa ctactggaaa gaccacccca tcgagcaggt gggccccaag     960

ctgcgggcca tgatgccctt cctcaagtcc aggttcacga aggaagaggt cggtagcagc    1020

gggagacctg tgctgggcag cagccaccac caccaccacc ac                       1062

<210>  14
<211>  354

<212>  PRT
<213>  Artificial Sequence

<220>
<223>  Ketolacid reductoisomerase (EC 1.1.1.86), Meiothermus ruber
       Protein sequence including C-terminal His-Tag

<400>  14

Met Lys Ile Tyr Tyr Asp Gln Asp Ala Asp Ile Gly Phe Ile Lys Asp
1               5                   10                  15

Lys Thr Val Ala Ile Leu Gly Phe Gly Ser Gln Gly His Ala His Ala
            20                  25                  30

Leu Asn Leu Arg Asp Ser Gly Ile Lys Val Val Val Gly Leu Arg Pro
        35                  40                  45

Gly Ser Arg Asn Glu Glu Lys Ala Arg Lys Ala Gly Leu Glu Val Leu
    50                  55                  60

Pro Val Gly Glu Ala Val Arg Arg Ala Asp Val Val Met Ile Leu Leu
65                  70                  75                  80

Pro Asp Glu Thr Gln Gly Ala Val Tyr Lys Ala Glu Val Glu Pro Asn
                85                  90                  95

Leu Lys Glu Gly Ala Ala Leu Ala Phe Ala His Gly Phe Asn Ile His
            100                 105                 110

Phe Gly Gln Ile Lys Pro Arg Arg Asp Leu Asp Val Trp Met Val Ala
        115                 120                 125

Pro Lys Gly Pro Gly His Leu Val Arg Ser Glu Tyr Glu Lys Gly Ser
    130                 135                 140

Gly Val Pro Ser Leu Val Ala Val Tyr Gln Asp Ala Ser Gly Ser Ala
145                 150                 155                 160

Phe Pro Thr Ala Leu Ala Tyr Ala Lys Ala Asn Gly Gly Thr Arg Ala
                165                 170                 175

Gly Thr Ile Ala Thr Thr Phe Lys Asp Glu Thr Glu Thr Asp Leu Phe
            180                 185                 190

Gly Glu Gln Thr Val Leu Cys Gly Gly Leu Thr Gln Leu Ile Ala Ala
        195                 200                 205

Gly Phe Glu Thr Leu Val Glu Ala Gly Tyr Pro Pro Glu Met Ala Tyr
    210                 215                 220

Phe Glu Cys Leu His Glu Val Lys Leu Ile Val Asp Leu Ile Tyr Glu
225                 230                 235                 240

Ser Gly Phe Ala Gly Met Arg Tyr Ser Ile Ser Asn Thr Ala Glu Tyr
                245                 250                 255

Gly Asp Tyr Thr Arg Gly Pro Met Val Ile Asn Arg Glu Glu Thr Lys
            260                 265                 270

Ala Arg Met Arg Glu Val Leu Arg Gln Ile Gln Gln Gly Glu Phe Ala
        275                 280                 285

Arg Glu Trp Met Leu Glu Asn Val Val Gly Gln Pro Thr Leu Asn Ala
    290                 295                 300

Asn Arg Asn Tyr Trp Lys Asp His Pro Ile Glu Gln Val Gly Pro Lys
305                 310                 315                 320

Leu Arg Ala Met Met Pro Phe Leu Lys Ser Arg Phe Thr Lys Glu Glu
                325                 330                 335

Val Gly Ser Ser Gly Arg Pro Val Leu Gly Ser Ser His His His His
            340                 345                 350

His His

<210>  15
<211>  1713
<212>  DNA
<213>  Artificial Sequence

<220>
<223>  Branched-chain ketoacid decarboxylase (EC 4.1.1.72), Lactococcus
       lactis DNA-sequence including N-terminal His-Tag

<400>  15
atgggcagca gccatcatca tcatcatcac agcagcggcc tggtgccgcg cggcagccat      60

atggctagca tgtatacagt aggagattac ctgttagacc gattacacga gttgggaatt     120

gaagaaattt ttggagttcc tggtgactat aacttacaat ttttagatca aattatttca     180

cgcgaagata tgaaatggat tggaaatgct aatgaattaa atgcttctta tatggctgat     240

ggttatgctc gtactaaaaa agctgccgca tttctcacca catttggagt cggcgaattg     300

agtgcgatca atggactggc aggaagttat gccgaaaatt taccagtagt agaaattgtt     360

ggttcaccaa cttcaaaagt acaaaatgac ggaaaatttg tccatcatac actagcagat     420

ggtgatttta aacactttat gaagatgcat gaacctgtta cagcagcgcg gactttactg     480

acagcagaaa atgccacata tgaaattgac cgagtacttt ctcaattact aaaagaaaga     540

aaaccagtct atattaactt accagtcgat gttgctgcag caaaagcaga gaagcctgca     600

ttatctttag aaaaagaaag ctctacaaca aatacaactg aacaagtgat tttgagtaag 660

attgaagaaa gtttgaaaaa tgcccaaaaa ccagtagtga ttgcaggaca cgaagtaatt 720

agttttggtt tagaaaaaac ggtaactcag tttgtttcag aaacaaaact accgattacg 780

acactaaatt ttggtaaaag tgctgttgat gaatctttgc cctcattttt aggaatatat 840

aacgggaaac tttcagaaat cagtcttaaa aattttgtgg agtccgcaga ctttatccta 900

atgcttggag tgaagcttac ggactcctca acaggtgcat tcacacatca tttagatgaa 960

aataaaatga tttcactaaa catagatgaa ggaataattt tcaataaagt ggtagaagat 1020

tttgatttta gagcagtggt ttcttcttta tcagaattaa aggaatagaa atatgaagga 1080

caatatattg ataagcaata tgaagaattt attccatcaa gtgctccctt atcacaagac 1140

cgtctatggc aggcagttga aagtttgact caaagcaatg aaacaatcgt tgctgaacaa 1200

ggaacctcat tttttggagc ttcaacaatt ttcttaaaat caaatagtcg ttttattgga 1260

caacctttat ggggttctat tggatatact tttccagcgg ctttaggaag ccaaattgcg 1320

gataaagaga gcagacacct tttatttatt ggtgatggtt cacttcaact taccgtacaa 1380

gaattaggac tatcaatcag agaaaaactc aatccaattt gttttatcat aaataatgat 1440

ggttatacag ttgaaagaga aatccacgga cctactcaaa gttataacga cattccaatg 1500

tggaattact cgaaattacc agaaacattt ggagcaacag aagatcgtgt agtatcaaaa 1560

attgttagaa cagagaatga atttgtgtct gtcatgaaag aagcccaagc agatgtcaat 1620

agaatgtatt ggatagaact agttttggaa aaagaagatg cgccaaaatt actgaaaaaa 1680

atgggtaaat tatttgctga gcaaaataaa taa 1713

<210> 16
<211> 570
<212> PRT
<213> Artificial Sequence

<220>
<223> Branched-chain ketoacid decarboxylase (EC 4.1.1.72), Lactococcus
      lactis Protein sequence including N-terminal His-Tag

<400> 16

Met Gly Ser Ser His His His His His His Ser Ser Gly Leu Val Pro
1               5                   10                  15

Arg Gly Ser His Met Ala Ser Met Tyr Thr Val Gly Asp Tyr Leu Leu
            20                  25                  30

Asp Arg Leu His Glu Leu Gly Ile Glu Glu Ile Phe Gly Val Pro Gly
        35                  40                  45

Asp Tyr Asn Leu Gln Phe Leu Asp Gln Ile Ile Ser Arg Glu Asp Met
    50                  55                  60

Lys Trp Ile Gly Asn Ala Asn Glu Leu Asn Ala Ser Tyr Met Ala Asp
65                  70                  75                  80

Gly Tyr Ala Arg Thr Lys Lys Ala Ala Ala Phe Leu Thr Thr Phe Gly
                85                  90                  95

Val Gly Glu Leu Ser Ala Ile Asn Gly Leu Ala Gly Ser Tyr Ala Glu
            100                 105                 110

Asn Leu Pro Val Val Glu Ile Val Gly Ser Pro Thr Ser Lys Val Gln
        115                 120                 125

Asn Asp Gly Lys Phe Val His His Thr Leu Ala Asp Gly Asp Phe Lys
    130                 135                 140

His Phe Met Lys Met His Glu Pro Val Thr Ala Ala Arg Thr Leu Leu
145                 150                 155                 160

Thr Ala Glu Asn Ala Thr Tyr Glu Ile Asp Arg Val Leu Ser Gln Leu
                165                 170                 175

Leu Lys Glu Arg Lys Pro Val Tyr Ile Asn Leu Pro Val Asp Val Ala
            180                 185                 190

Ala Ala Lys Ala Glu Lys Pro Ala Leu Ser Leu Glu Lys Glu Ser Ser
        195                 200                 205

Thr Thr Asn Thr Thr Glu Gln Val Ile Leu Ser Lys Ile Glu Glu Ser
    210                 215                 220

Leu Lys Asn Ala Gln Lys Pro Val Val Ile Ala Gly His Glu Val Ile
225                 230                 235                 240

Ser Phe Gly Leu Glu Lys Thr Val Thr Gln Phe Val Ser Glu Thr Lys
                245                 250                 255

Leu Pro Ile Thr Thr Leu Asn Phe Gly Lys Ser Ala Val Asp Glu Ser
            260                 265                 270

Leu Pro Ser Phe Leu Gly Ile Tyr Asn Gly Lys Leu Ser Glu Ile Ser
        275                 280                 285

Leu Lys Asn Phe Val Glu Ser Ala Asp Phe Ile Leu Met Leu Gly Val
    290                 295                 300

Lys Leu Thr Asp Ser Ser Thr Gly Ala Phe Thr His His Leu Asp Glu
305                 310                 315                 320

Asn Lys Met Ile Ser Leu Asn Ile Asp Glu Gly Ile Ile Phe Asn Lys
                325                 330                 335

Val Val Glu Asp Phe Asp Phe Arg Ala Val Val Ser Ser Leu Ser Glu
            340                 345                 350

Leu Lys Gly Ile Glu Tyr Glu Gly Gln Tyr Ile Asp Lys Gln Tyr Glu
        355                 360                 365

Glu Phe Ile Pro Ser Ser Ala Pro Leu Ser Gln Asp Arg Leu Trp Gln
    370                 375                 380

Ala Val Glu Ser Leu Thr Gln Ser Asn Glu Thr Ile Val Ala Glu Gln
385                 390                 395                 400

Gly Thr Ser Phe Phe Gly Ala Ser Thr Ile Phe Leu Lys Ser Asn Ser
                405                 410                 415

Arg Phe Ile Gly Gln Pro Leu Trp Gly Ser Ile Gly Tyr Thr Phe Pro
            420                 425                 430

Ala Ala Leu Gly Ser Gln Ile Ala Asp Lys Glu Ser Arg His Leu Leu
        435                 440                 445

Phe Ile Gly Asp Gly Ser Leu Gln Leu Thr Val Gln Glu Leu Gly Leu
    450                 455                 460

Ser Ile Arg Glu Lys Leu Asn Pro Ile Cys Phe Ile Ile Asn Asn Asp
465                 470                 475                 480

Gly Tyr Thr Val Glu Arg Glu Ile His Gly Pro Thr Gln Ser Tyr Asn
                485                 490                 495

Asp Ile Pro Met Trp Asn Tyr Ser Lys Leu Pro Glu Thr Phe Gly Ala
            500                 505                 510

Thr Glu Asp Arg Val Val Ser Lys Ile Val Arg Thr Glu Asn Glu Phe
        515                 520                 525

Val Ser Val Met Lys Glu Ala Gln Ala Asp Val Asn Arg Met Tyr Trp
    530                 535                 540

Ile Glu Leu Val Leu Glu Lys Glu Asp Ala Pro Lys Leu Leu Lys Lys
545                 550                 555                 560

Met Gly Lys Leu Phe Ala Glu Gln Asn Lys
                565                 570

<210>  17
<211>  1044
<212>  DNA
<213>  Artificial Sequence

<220>
<223>  Alcohol dehydrogenase (EC 1.1.1.1), Geobacillus
       stearothermophilus DNA-sequence including C-terminal His-Tag

<400>  17
atgaaagcag cagttgtgga acagtttaaa gaaccgctga aaatcaaaga ggtggaaaaa      60

ccgaccatta gctatggtga agttctggtt cgtattaaag cctgtggtgt ttgtcatacc     120

gatctgcatg cagcacatgg tgattggcct gttaaaccga aactgccgct gattccgggt     180

catgaaggtg ttggtattgt tgaagaggtt ggtccgggtg ttacccatct gaaagttggt     240

gatcgtgttg gtattccgtg gctgtatagc gcatgtggtc attgtgatta ttgtctgagc     300

ggtcaagaaa ccctgtgtga acatcagaaa aatgcaggtt atagcgtgga tggtggttat     360

gcagaatatt gtcgtgcagc agcagattat gttgtgaaaa ttccggataa cctgagcttt     420

gaagaagcag caccgatttt ttgtgccggt gttaccacct ataaagcact gaaagttacc     480

ggtgcaaaac cgggtgaatg ggttgcaatt tatggtattg gtggtctggg ccatgttgca     540

gttcagtatg caaaagcaat gggtctgaat gttgttgcag tggatatcgg tgatgaaaaa     600

ctggaactgg caaaagaact gggtgcagat ctggttgtta atccgctgaa agaagatgca     660

gccaaattta tgaaagaaaa agtgggtggt gttcatgcag cagttgttac cgcagttagc     720

aaaccggcat ttcagagcgc atataatagc attcgtcgtg gtggtgcatg tgttctggtt     780

ggtctgcctc cggaagaaat gccgattccg atttttgata ccgttctgaa cggcattaaa     840

atcattggta gcattgttgg cacccgtaaa gatctgcaag aagcactgca gtttgcagca     900

gaaggtaaag ttaaaaccat cattgaagtt cagccgctgg aaaaaatcaa cgaagttttt     960

gatcgcatgc tgaaaggtca gattaatggt cgtgttgttc tgaccctgga agataaactc    1020

gagcaccacc accaccacca ctga                                           1044

<210>  18
<211>  347
<212>  PRT
<213>  Artificial Sequence

<220>
<223>  Alcohol dehydrogenase (EC 1.1.1.1), Geobacillus
       stearothermophilus Protein sequence including C-terminal His-Tag

<400>  18

Met Lys Ala Ala Val Val Glu Gln Phe Lys Glu Pro Leu Lys Ile Lys
1               5                   10                  15

Glu Val Glu Lys Pro Thr Ile Ser Tyr Gly Glu Val Leu Val Arg Ile
            20                  25                  30

Lys Ala Cys Gly Val Cys His Thr Asp Leu His Ala Ala His Gly Asp
        35                  40                  45

Trp Pro Val Lys Pro Lys Leu Pro Leu Ile Pro Gly His Glu Gly Val
    50                  55                  60

Gly Ile Val Glu Glu Val Gly Pro Gly Val Thr His Leu Lys Val Gly
65                  70                  75                  80

Asp Arg Val Gly Ile Pro Trp Leu Tyr Ser Ala Cys Gly His Cys Asp
                85                  90                  95

Tyr Cys Leu Ser Gly Gln Glu Thr Leu Cys Glu His Gln Lys Asn Ala
            100                 105                 110

Gly Tyr Ser Val Asp Gly Gly Tyr Ala Glu Tyr Cys Arg Ala Ala Ala
        115                 120                 125

Asp Tyr Val Val Lys Ile Pro Asp Asn Leu Ser Phe Glu Glu Ala Ala
    130                 135                 140

Pro Ile Phe Cys Ala Gly Val Thr Thr Tyr Lys Ala Leu Lys Val Thr
145                 150                 155                 160

Gly Ala Lys Pro Gly Glu Trp Val Ala Ile Tyr Gly Ile Gly Gly Leu
                165                 170                 175

Gly His Val Ala Val Gln Tyr Ala Lys Ala Met Gly Leu Asn Val Val
            180                 185                 190

Ala Val Asp Ile Gly Asp Glu Lys Leu Glu Leu Ala Lys Glu Leu Gly
        195                 200                 205

Ala Asp Leu Val Val Asn Pro Leu Lys Glu Asp Ala Ala Lys Phe Met
    210                 215                 220

Lys Glu Lys Val Gly Gly Val His Ala Ala Val Val Thr Ala Val Ser
225                 230                 235                 240

Lys Pro Ala Phe Gln Ser Ala Tyr Asn Ser Ile Arg Arg Gly Gly Ala
                245                 250                 255

Cys Val Leu Val Gly Leu Pro Pro Glu Glu Met Pro Ile Pro Ile Phe
            260                 265                 270

Asp Thr Val Leu Asn Gly Ile Lys Ile Ile Gly Ser Ile Val Gly Thr
        275                 280                 285

Arg Lys Asp Leu Gln Glu Ala Leu Gln Phe Ala Ala Glu Gly Lys Val
    290                 295                 300

Lys Thr Ile Ile Glu Val Gln Pro Leu Glu Lys Ile Asn Glu Val Phe
305                 310                 315                 320

Asp Arg Met Leu Lys Gly Gln Ile Asn Gly Arg Val Val Leu Thr Leu
                325                 330                 335

Glu Asp Lys Leu Glu His His His His His His
            340                 345

<210>  19
<211>  1752
<212>  DNA
<213>  Artificial Sequence

<220>
<223>  Pyruvate decarboxylase (EC 4.1.1.1), Zymomonas mobilis
       DNA-sequence including C-terminal His-Tag

<400>  19
atgagctata ccgttggcac ctatctggca gaacgtctgg ttcagattgg tctgaaacat      60

cattttgcag tggcaggcga ttataatctg gtgctgctgg ataatctgct gctgaataaa     120

aatatggaac aggtgtattg ctgcaatgaa ctgaattgtg gctttagcgc tgaaggttat     180

gcacgtgcaa aaggtgcagc agcagcagtt gttacctata gcgttggtgc actgagcgca     240

tttgatgcca ttggtggtgc ttatgcagaa aatctgccgg ttattctgat ttctggtgca     300

ccgaataata atgatcatgc cgcaggccat gttctgcatc atgcactggg taaaaccgat     360

tatcattatc agctggaaat ggccaaaaat attaccgcag cagccgaagc aatttataca     420

ccggaagaag caccggcaaa aattgatcat gtgattaaaa ccgcactgcg tgaaaaaaaa     480

ccggtgtatc tggaaattgc ctgtaatatt gcaagcatgc cgtgtgcagc accgggtccg     540

gcaagcgcac tgtttaatga tgaagcctct gatgaagcaa gcctgaatgc agcagttgaa     600

gaaaccctga aatttattgc caatcgcgat aaagttgcag ttctggttgg tagcaaactg     660

cgtgcagccg gtgcagaaga agcagcagtt aaatttgcag atgcactggg tggtgcagtt     720

gcaaccatgg cagcagcaaa aagttttttt ccggaagaaa atccgcatta cattggcacc     780

agctggggtg aagttagcta tccgggtgtt gaaaaaacca tgaaagaagc cgacgcagtt     840

attgcactgg caccggtgtt taatgattat agcaccaccg gttggaccga tattccggat     900

ccgaaaaaac tggttctggc cgaaccgcgt agcgttgttg ttaatggtat tcgttttccg     960

agcgtgcatc tgaaagatta tctgacccgt ctggcacaga aagttagcaa aaaaacaggt    1020

gccctggatt tttttaaatc cctgaatgcc ggtgaactga aaaaagcagc accggcagat    1080

ccgagcgcac cgctggttaa tgcagaaatt gcacgtcagg ttgaagcact gctgaccccg    1140

aataccaccg ttattgcaga aaccggtgat agctggttta atgcccagcg tatgaaactg    1200

ccgaatggtg cacgtgttga atatgaaatg cagtggggtc atattggttg gagcgttccg 1260

gcagcatttg gttatgcagt tggtgcaccg gaacgtcgta atattctgat ggttggtgat 1320

ggtagctttc agctgaccgc acaagaggtt gcacagatgg ttcgtctgaa actgccggtg 1380

attatttttc tgattaataa ttatggctat accattgaag tgatgattca tgatggtccg 1440

tataataata ttaaaaattg ggattatgcc ggtctgatgg aagtgtttaa tggcaatggt 1500

ggttatgata gcggtgccgg taaaggtctg aaagcaaaaa ccggtggtga actggcagaa 1560

gcaattaaag ttgcactggc caataccgat ggtccgaccc tgattgaatg ttttattggt 1620

cgcgaagatt gtaccgaaga actggtgaaa tggggtaaac gtgttgcagc agcaaatagc 1680

cgtaaaccgg tgaataaact gctgagcggg agacctgtgc tgggcagcag ccaccaccac 1740

caccaccact aa 1752

<210> 20
<211> 583
<212> PRT
<213> Artificial Sequence

<220>
<223> Pyruvate decarboxylase (EC 4.1.1.1), Zymomonas mobilis Protein
sequence including C-terminal His-Tag

<400> 20

Met Ser Tyr Thr Val Gly Thr Tyr Leu Ala Glu Arg Leu Val Gln Ile
1               5                   10                  15

Gly Leu Lys His His Phe Ala Val Ala Gly Asp Tyr Asn Leu Val Leu
            20                  25                  30

Leu Asp Asn Leu Leu Leu Asn Lys Asn Met Glu Gln Val Tyr Cys Cys
        35                  40                  45

Asn Glu Leu Asn Cys Gly Phe Ser Ala Glu Gly Tyr Ala Arg Ala Lys
    50                  55                  60

Gly Ala Ala Ala Ala Val Val Thr Tyr Ser Val Gly Ala Leu Ser Ala
65                  70                  75                  80

Phe Asp Ala Ile Gly Gly Ala Tyr Ala Glu Asn Leu Pro Val Ile Leu
                85                  90                  95

Ile Ser Gly Ala Pro Asn Asn Asn Asp His Ala Ala Gly His Val Leu
            100                 105                 110

His His Ala Leu Gly Lys Thr Asp Tyr His Tyr Gln Leu Glu Met Ala
        115                 120                 125

Lys Asn Ile Thr Ala Ala Ala Glu Ala Ile Tyr Thr Pro Glu Glu Ala
    130                 135                 140

Pro Ala Lys Ile Asp His Val Ile Lys Thr Ala Leu Arg Glu Lys Lys
145                 150                 155                 160

Pro Val Tyr Leu Glu Ile Ala Cys Asn Ile Ala Ser Met Pro Cys Ala
                165                 170                 175

Ala Pro Gly Pro Ala Ser Ala Leu Phe Asn Asp Glu Ala Ser Asp Glu
            180                 185                 190

Ala Ser Leu Asn Ala Ala Val Glu Glu Thr Leu Lys Phe Ile Ala Asn
        195                 200                 205

Arg Asp Lys Val Ala Val Leu Val Gly Ser Lys Leu Arg Ala Ala Gly
    210                 215                 220

Ala Glu Glu Ala Ala Val Lys Phe Ala Asp Ala Leu Gly Gly Ala Val
225                 230                 235                 240

Ala Thr Met Ala Ala Ala Lys Ser Phe Phe Pro Glu Glu Asn Pro His
                245                 250                 255

Tyr Ile Gly Thr Ser Trp Gly Glu Val Ser Tyr Pro Gly Val Glu Lys
            260                 265                 270

Thr Met Lys Glu Ala Asp Ala Val Ile Ala Leu Ala Pro Val Phe Asn
        275                 280                 285

Asp Tyr Ser Thr Thr Gly Trp Thr Asp Ile Pro Asp Pro Lys Lys Leu
    290                 295                 300

Val Leu Ala Glu Pro Arg Ser Val Val Val Asn Gly Ile Arg Phe Pro
305                 310                 315                 320

Ser Val His Leu Lys Asp Tyr Leu Thr Arg Leu Ala Gln Lys Val Ser
                325                 330                 335

Lys Lys Thr Gly Ala Leu Asp Phe Phe Lys Ser Leu Asn Ala Gly Glu
            340                 345                 350

Leu Lys Lys Ala Ala Pro Ala Asp Pro Ser Ala Pro Leu Val Asn Ala
        355                 360                 365

Glu Ile Ala Arg Gln Val Glu Ala Leu Leu Thr Pro Asn Thr Thr Val
    370                 375                 380

Ile Ala Glu Thr Gly Asp Ser Trp Phe Asn Ala Gln Arg Met Lys Leu

385                 390                 395                 400

Pro Asn Gly Ala Arg Val Glu Tyr Glu Met Gln Trp Gly His Ile Gly
                405                 410                 415

Trp Ser Val Pro Ala Ala Phe Gly Tyr Ala Val Gly Ala Pro Glu Arg
            420                 425                 430

Arg Asn Ile Leu Met Val Gly Asp Gly Ser Phe Gln Leu Thr Ala Gln
        435                 440                 445

Glu Val Ala Gln Met Val Arg Leu Lys Leu Pro Val Ile Ile Phe Leu
    450                 455                 460

Ile Asn Asn Tyr Gly Tyr Thr Ile Glu Val Met Ile His Asp Gly Pro
465                 470                 475                 480

Tyr Asn Asn Ile Lys Asn Trp Asp Tyr Ala Gly Leu Met Glu Val Phe
                485                 490                 495

Asn Gly Asn Gly Gly Tyr Asp Ser Gly Ala Gly Lys Gly Leu Lys Ala
            500                 505                 510

Lys Thr Gly Gly Glu Leu Ala Glu Ala Ile Lys Val Ala Leu Ala Asn
        515                 520                 525

Thr Asp Gly Pro Thr Leu Ile Glu Cys Phe Ile Gly Arg Glu Asp Cys
    530                 535                 540

Thr Glu Glu Leu Val Lys Trp Gly Lys Arg Val Ala Ala Ala Asn Ser
545                 550                 555                 560

Arg Lys Pro Val Asn Lys Leu Leu Ser Gly Arg Pro Val Leu Gly Ser
                565                 570                 575

Ser His His His His His His
            580

<210>  21
<211>  25
<212>  DNA
<213>  Artificial Sequence

<220>
<223>  Primer sequence Fw-Mut (65°C)

<400>  21
gaattgtgag cggataacaa ttccc                                          25

<210>  22
<211>  21

<212>  DNA
<213>  Artificial Sequence

<220>
<223>  Primer sequence Rev-Mut (65°C)

<400>  22
ctttgttagc agccggatct c                                                21


<210>  23
<211>  38
<212>  DNA
<213>  Artificial Sequence

<220>
<223>  Primer sequence Fw-F34 (71°C)


<220>
<221>  misc_feature
<222>  (20)..(21)
<223>  n is a, c, g, or t

<400>  23
cggtcaggtt attggtcgtn nkgaagcagc aacccgtg                              38


<210>  24
<211>  38
<212>  DNA
<213>  Artificial Sequence

<220>
<223>  Primer sequence Rev-F34 (71°C)


<220>
<221>  misc_feature
<222>  (18)..(19)
<223>  n is a, c, g, or t

<400>  24
cacgggttgc tgcttcmnna cgaccaataa cctgaccg                              38


<210>  25
<211>  39
<212>  DNA
<213>  Artificial Sequence

<220>
<223>  Primer sequence Fw-S405 (64°C)


<220>
<221>  misc_feature
<222>  (20)..(21)
<223>  n is a, c, g, or t

<400>  25
gtatgatctg gccaatgatn nkaaatatgg tctggccag                             39


<210>  26

<211>  39
<212>  DNA
<213>  Artificial Sequence

<220>
<223>  Primer sequence Rev-S405 (64°C)


<220>
<221>  misc_feature
<222>  (19)..(20)
<223>  n is a, c, g, or t

<400>  26
ctggccagac catatttmnn atcattggcc agatcatac                              39


<210>  27
<211>  32
<212>  DNA
<213>  Artificial Sequence

<220>
<223>  Primer sequence Fw-W271 (60°C)


<220>
<221>  misc_feature
<222>  (14)..(15)
<223>  n is a, c, g, or t

<400>  27
gaaaaccctg ctgnnkgcaa aatattggaa tg                                     32


<210>  28
<211>  32
<212>  DNA
<213>  Artificial Sequence

<220>
<223>  Primer sequence Rev-W271 (60°C)


<220>
<221>  misc_feature
<222>  (18)..(19)
<223>  n is a, c, g, or t

<400>  28
cattccaata ttttgcmnnc agcagggttt tc                                     32


<210>  29
<211>  28
<212>  DNA
<213>  Artificial Sequence

<220>
<223>  Primer sequence Fw-Y399 (59°C)


<220>
<221>  misc_feature
<222>  (14)..(15)

<223>  n is a, c, g, or t

<400>  29
cgtggaagaa atgnnkgatc tggccaat                                          28


<210>  30
<211>  28
<212>  DNA
<213>  Artificial Sequence

<220>
<223>  Primer sequence Rev-Y399 (59°C)


<220>
<221>  misc_feature
<222>  (14)..(15)
<223>  n is a, c, g, or t

<400>  30
attggccaga tcmnncattt cttccacg                                          28


<210>  31
<211>  37
<212>  DNA
<213>  Artificial Sequence

<220>
<223>  Primer sequence Fw-F34L (75°C)

<400>  31
ggtcaggtta ttggtcgttt agaagcagca acccgtg                                37


<210>  32
<211>  37
<212>  DNA
<213>  Artificial Sequence

<220>
<223>  Primer sequence Rev-F34L (75°C)

<400>  32
cacgggttgc tgcttctaaa cgaccaataa cctgacc                                37


<210>  33
<211>  35
<212>  DNA
<213>  Artificial Sequence

<220>
<223>  Primer sequence Fw-F34M (68°C)

<400>  33
gtcaggttat tggtcgtatg gaagcagcaa cccgt                                  35


<210>  34
<211>  35
<212>  DNA
<213>  Artificial Sequence

<220>
<223>  Primer sequence Rev-F34M (68°C)

<400>  34
acgggttgct gcttccatac gaccaataac ctgac                                35


<210>  35
<211>  32
<212>  DNA
<213>  Artificial Sequence

<220>
<223>  Primer sequence Fw-W271S (65°C)

<400>  35
gaaaaccctg ctgtcggcaa aatattggaa tg                                   32


<210>  36
<211>  32
<212>  DNA
<213>  Artificial Sequence

<220>
<223>  Primer sequence Rev-W271S (65°C)

<400>  36
cattccaata ttttgccgac agcagggttt tc                                   32


<210>  37
<211>  29
<212>  DNA
<213>  Artificial Sequence

<220>
<223>  Primer sequence Fw-Y399C (63°C)

<400>  37
cgtggaagaa atgtgtgatc tggccaatg                                       29


<210>  38
<211>  29
<212>  DNA
<213>  Artificial Sequence

<220>
<223>  Primer sequence Rev-Y399C (63°C)

<400>  38
cattggccag atcacacatt tcttccacg                                       29


<210>  39
<211>  37
<212>  DNA
<213>  Artificial Sequence

<220>
<223>  Primer sequence Fw-S405C (73°C)

<400>  39
gtatgatctg gccaatgatt gcaaatatgg tctggcc                              37

<210>  40
<211>  37
<212>  DNA
<213>  Artificial Sequence

<220>
<223>  Primer sequence Rev-S405C (73°C)

<400>  40
ggccagacca tatttgcaat cattggccag atcatac                                37


<210>  41
<211>  35
<212>  DNA
<213>  Artificial Sequence

<220>
<223>  Primer sequence Fw-S405N (68°C)

<400>  41
tgatctggcc aatgataaca aatatggtct ggcca                                  35


<210>  42
<211>  35
<212>  DNA
<213>  Artificial Sequence

<220>
<223>  Primer sequence Rev-S405N (68°C)

<400>  42
tggccagacc atatttgtta tcattggcca gatca                                  35


<210>  43
<211>  105
<212>  DNA
<213>  Artificial Sequence

<220>
<223>  pCBR multiple cloning site

<400>  43
atatatatat tctagaaata attttgttta actttaagaa ggagatatac atatgatgca       60

ggtatatata tattaataga gacctcctcg gatccatata tatat                      105


<210>  44
<211>  66
<212>  DNA
<213>  Artificial Sequence

<220>
<223>  pCBRHisN multiple cloning site

<400>  44
atatatatat catatgatgc aggtatatat atattaatag agacctcctc gaattcatat       60

atatat                                                                  66

<210>  45
<211>  192
<212>  DNA
<213>  Artificial Sequence

<220>
<223>  pCBRHisC multiple cloning site

<400>  45
atatatatat tctagaaata attttgttta actttaagaa ggagatatac atatgatgca      60

ggtatatata tatagcggga gacctgtgct gggcagcagc caccaccacc accaccacta     120

atgagatccg gctgctaaca aagcccgaaa ggaagctgag ttggctgctg ccaccgctga     180

gcatatatat at                                                         192


<210>  46
<211>  43
<212>  DNA
<213>  Artificial Sequence

<220>
<223>  Primer sequence SsGDH_for

<400>  46
cagcaaggtc tcacatatga aagccattat tgtgaaacct ccg                        43


<210>  47
<211>  29
<212>  DNA
<213>  Artificial Sequence

<220>
<223>  Primer sequence SsGDH_rev

<400>  47
ttcccacaga atacgaattt tgatttcgc                                        29


<210>  48
<211>  39
<212>  DNA
<213>  Artificial Sequence

<220>
<223>  Primer sequence SsDHAD_for

<400>  48
cagcaaggtc tcacatatgc ctgcaaaact gaatagccc                             39


<210>  49
<211>  18
<212>  DNA
<213>  Artificial Sequence

<220>
<223>  Primer sequence SsDHAD_rev

<400>  49
tgccggacgg gtaactgc                                                    18

<210>  50
<211>  44
<212>  DNA
<213>  Artificial Sequence

<220>
<223>  Primer sequence SaKDGA_for

<400>  50
cagcaaggtc tcacatatgg aaattattag cccgattatt accc                        44


<210>  51
<211>  24
<212>  DNA
<213>  Artificial Sequence

<220>
<223>  Primer sequence SaKDGA_rev

<400>  51
atgaaccagt tcctgaattt tgcg                                              24


<210>  52
<211>  43
<212>  DNA
<213>  Artificial Sequence

<220>
<223>  Primer sequence TaALDH_for

<400>  52
cagcaaggtc tcacatatgg ataccaaact gtatattgat ggc                         43


<210>  53
<211>  22
<212>  DNA
<213>  Artificial Sequence

<220>
<223>  Primer sequence TaALDH_rev

<400>  53
ctgaaacagg tcatcacgaa cg                                                22


<210>  54
<211>  44
<212>  DNA
<213>  Artificial Sequence

<220>
<223>  Primer sequence MrKARI_for

<400>  54
cagcaacgtc tcgcatatga agatttacta cgaccaggac gcag                        44


<210>  55
<211>  25
<212>  DNA

<213>  Artificial Sequence

<220>
<223>  Primer sequence MrKARI_rev

<400>  55
gctaccgacc tcttccttcg tgaac                                          25


<210>  56
<211>  1527
<212>  DNA
<213>  Artificial Sequence

<220>
<223>  taALDH-F34L-Chis

<400>  56
atggatacca aactgtatat tgatggccag tgggttaata gcagcagcgg taaaaccgtt      60

gataaatatt ctccggttac cggtcaggtt attggtcgtt tagaagcagc aacccgtgat     120

gatgttgatc gtgcaattga tgcagcagaa gatgcatttt gggcctggaa tgatctgggt     180

agcgttgaac gcagcaaaat tatttatcgt gccaaagaac tgattgaaaa aaatcgtgcc     240

gaactggaaa atattattat ggaagaaaat ggcaaaccgg tgaaagaagc caaagaagaa     300

gtcgacggcg tcattgatca gattcagtat tatgcagaat gggcacgtaa actgaatggt     360

gaagttgttg aaggcaccag cagccatcgt aaaatttttc agtataaagt gccgtatggt     420

attgttgttg cactgacccc gtggaatttt ccggcaggca tggttgcccg taaactggca     480

ccggcactgc tgaccggtaa taccgttgtt ctgaaaccga gcagcgatac accgggtagc     540

gcagaatgga ttgtgcgtaa atttgttgaa gccggtgttc cgaaaggtgt gctgaatttt     600

attaccggtc gtggtagcga aattggcgat tacattgtgg aacataaaaa agtcaatctg     660

attaccatga ccggtagcac cgcaacaggt cagcgcatta tgcagaaagc aagcgcaaat     720

atggcaaaac tgattctgga actgggtggt aaagcaccgt ttatggtttg gaaagatgcc     780

gatatggata atgcactgaa aaccctgctg tgggcaaaat attggaatgc cggtcagagc     840

tgtattgcag cagaacgtct gtatgtgcat gaagatattt atgatacctt tatgagccgt     900

tttgttgaac tgagccgcaa actggcactg ggtgatccga aaaatgcaga tatgggtccg     960

ctgattaata aaggtgcact gcaggcaacc agcgaaattg ttgaagaagc gaaagaatct    1020

ggcgcaaaaa ttctgtttgg tggtagccag ccgagcctga gcggtccgta tcgtaatggc    1080

tatttttttc tgccgaccat tattggtaat gcggatcaga aaagcaaaat ctttcaggaa    1140

gaaatttttg caccggttat tggtgcacgt aaaattagca gcgtggaaga aatgtatgat    1200

ctggccaatg atagcaaata tggtctggcc agctacctgt ttaccaaaga tccgaatatc    1260

atttttgaag ccagcgaacg tattcgtttt ggtgaactgt atgtgaatat gccgggtccg    1320

gaagcaagcc agggttatca caccggtttt cgtatgacag gtcaggcagg cgaaggttct    1380

aaatatggca ttagcgaata tctgaaactg aaaaatattt atgtggatta tagcggcaaa    1440

ccgctgcata ttaataccgt tcgtgatgac ctgtttcaga gcgggagacc tgtgctgggc 1500

agcagccacc accaccacca ccactaa 1527

<210> 57
<211> 508
<212> PRT
<213> Artificial Sequence

<220>
<223> taALDH-F34L-Chis

<400> 57

Met Asp Thr Lys Leu Tyr Ile Asp Gly Gln Trp Val Asn Ser Ser Ser
1               5                   10                  15

Gly Lys Thr Val Asp Lys Tyr Ser Pro Val Thr Gly Gln Val Ile Gly
            20                  25                  30

Arg Leu Glu Ala Ala Thr Arg Asp Asp Val Asp Arg Ala Ile Asp Ala
        35                  40                  45

Ala Glu Asp Ala Phe Trp Ala Trp Asn Asp Leu Gly Ser Val Glu Arg
    50                  55                  60

Ser Lys Ile Ile Tyr Arg Ala Lys Glu Leu Ile Glu Lys Asn Arg Ala
65                  70                  75                  80

Glu Leu Glu Asn Ile Ile Met Glu Glu Asn Gly Lys Pro Val Lys Glu
                85                  90                  95

Ala Lys Glu Glu Val Asp Gly Val Ile Asp Gln Ile Gln Tyr Tyr Ala
            100                 105                 110

Glu Trp Ala Arg Lys Leu Asn Gly Glu Val Val Glu Gly Thr Ser Ser
        115                 120                 125

His Arg Lys Ile Phe Gln Tyr Lys Val Pro Tyr Gly Ile Val Val Ala
    130                 135                 140

Leu Thr Pro Trp Asn Phe Pro Ala Gly Met Val Ala Arg Lys Leu Ala
145                 150                 155                 160

Pro Ala Leu Leu Thr Gly Asn Thr Val Val Leu Lys Pro Ser Ser Asp
                165                 170                 175

Thr Pro Gly Ser Ala Glu Trp Ile Val Arg Lys Phe Val Glu Ala Gly
            180                 185                 190

Val Pro Lys Gly Val Leu Asn Phe Ile Thr Gly Arg Gly Ser Glu Ile

195                 200                 205

Gly Asp Tyr Ile Val Glu His Lys Lys Val Asn Leu Ile Thr Met Thr
    210                 215                 220

Gly Ser Thr Ala Thr Gly Gln Arg Ile Met Gln Lys Ala Ser Ala Asn
225                 230                 235                 240

Met Ala Lys Leu Ile Leu Glu Leu Gly Gly Lys Ala Pro Phe Met Val
                245                 250                 255

Trp Lys Asp Ala Asp Met Asp Asn Ala Leu Lys Thr Leu Leu Trp Ala
            260                 265                 270

Lys Tyr Trp Asn Ala Gly Gln Ser Cys Ile Ala Ala Glu Arg Leu Tyr
        275                 280                 285

Val His Glu Asp Ile Tyr Asp Thr Phe Met Ser Arg Phe Val Glu Leu
    290                 295                 300

Ser Arg Lys Leu Ala Leu Gly Asp Pro Lys Asn Ala Asp Met Gly Pro
305                 310                 315                 320

Leu Ile Asn Lys Gly Ala Leu Gln Ala Thr Ser Glu Ile Val Glu Glu
                325                 330                 335

Ala Lys Glu Ser Gly Ala Lys Ile Leu Phe Gly Gly Ser Gln Pro Ser
            340                 345                 350

Leu Ser Gly Pro Tyr Arg Asn Gly Tyr Phe Phe Leu Pro Thr Ile Ile
        355                 360                 365

Gly Asn Ala Asp Gln Lys Ser Lys Ile Phe Gln Glu Glu Ile Phe Ala
    370                 375                 380

Pro Val Ile Gly Ala Arg Lys Ile Ser Ser Val Glu Glu Met Tyr Asp
385                 390                 395                 400

Leu Ala Asn Asp Ser Lys Tyr Gly Leu Ala Ser Tyr Leu Phe Thr Lys
                405                 410                 415

Asp Pro Asn Ile Ile Phe Glu Ala Ser Glu Arg Ile Arg Phe Gly Glu
            420                 425                 430

Leu Tyr Val Asn Met Pro Gly Pro Glu Ala Ser Gln Gly Tyr His Thr
        435                 440                 445

Gly Phe Arg Met Thr Gly Gln Ala Gly Glu Gly Ser Lys Tyr Gly Ile
    450                 455                 460

Ser Glu Tyr Leu Lys Leu Lys Asn Ile Tyr Val Asp Tyr Ser Gly Lys
465                 470                 475                 480

Pro Leu His Ile Asn Thr Val Arg Asp Asp Leu Phe Gln Ser Gly Arg
                485                 490                 495

Pro Val Leu Gly Ser Ser His His His His His His
            500                 505

<210>  58
<211>  1527
<212>  DNA
<213>  Artificial Sequence

<220>
<223>  TaALDH-S405C-Chis

<400>  58
atggatacca aactgtatat tgatggccag tgggttaata gcagcagcgg taaaaccgtt      60

gataaatatt ctccggttac cggtcaggtt attggtcgtt ttgaagcagc aacccgtgat     120

gatgttgatc gtgcaattga tgcagcagaa gatgcatttt gggcctggaa tgatctgggt     180

agcgttgaac gcagcaaaat tatttatcgt gccaaagaac tgattgaaaa aaatcgtgcc     240

gaactggaaa atattattat ggaagaaaat ggcaaaccgg tgaaagaagc caaagaagaa     300

gtcgacggcg tcattgatca gattcagtat tatgcagaat gggcacgtaa actgaatggt     360

gaagttgttg aaggcaccag cagccatcgt aaaatttttc agtataaagt gccgtatggt     420

attgttgttg cactgacccc gtggaatttt ccggcaggca tggttgcccg taaactggca     480

ccggcactgc tgaccggtaa taccgttgtt ctgaaaccga gcagcgatac accgggtagc     540

gcagaatgga ttgtgcgtaa atttgttgaa gccggtgttc cgaaaggtgt gctgaatttt     600

attaccggtc gtggtagcga aattggcgat tacattgtgg aacataaaaa agtcaatctg     660

attaccatga ccggtagcac cgcaacaggt cagcgcatta tgcagaaagc aagcgcaaat     720

atggcaaaac tgattctgga actgggtggt aaagcaccgt ttatggtttg gaaagatgcc     780

gatatggata atgcactgaa aaccctgctg tgggcaaaat attggaatgc cggtcagagc     840

tgtattgcag cagaacgtct gtatgtgcat gaagatattt atgatacctt tatgagccgt     900

tttgttgaac tgagccgcaa actggcactg ggtgatccga aaaatgcaga tatgggtccg     960

ctgattaata aaggtgcact gcaggcaacc agcgaaattg ttgaagaagc gaaagaatct    1020

ggcgcaaaaa ttctgtttgg tggtagccag ccgagcctga gcggtccgta tcgtaatggc    1080

tatttttttc tgccgaccat tattggtaat gcggatcaga aaagcaaaat ctttcaggaa    1140

gaaatttttg caccggttat tggtgcacgt aaaattagca gcgtggaaga aatgtatgat    1200

ctggccaatg attgcaaata tggtctggcc agctacctgt ttaccaaaga tccgaatatc    1260

atttttgaag ccagcgaacg tattcgtttt ggtgaactgt atgtgaatat gccgggtccg 1320

gaagcaagcc agggttatca caccggtttt cgtatgacag gtcaggcagg cgaaggttct 1380

aaatatggca ttagcgaata tctgaaactg aaaaatattt atgtggatta tagcggcaaa 1440

ccgctgcata ttaataccgt tcgtgatgac ctgtttcaga gcgggagacc tgtgctgggc 1500

agcagccacc accaccacca ccactaa 1527

<210> 59
<211> 508
<212> PRT
<213> Artificial Sequence

<220>
<223> TaALDH-S405C-Chis

<400> 59

Met Asp Thr Lys Leu Tyr Ile Asp Gly Gln Trp Val Asn Ser Ser Ser
1               5                   10                  15

Gly Lys Thr Val Asp Lys Tyr Ser Pro Val Thr Gly Gln Val Ile Gly
            20                  25                  30

Arg Phe Glu Ala Ala Thr Arg Asp Asp Val Asp Arg Ala Ile Asp Ala
        35                  40                  45

Ala Glu Asp Ala Phe Trp Ala Trp Asn Asp Leu Gly Ser Val Glu Arg
    50                  55                  60

Ser Lys Ile Ile Tyr Arg Ala Lys Glu Leu Ile Glu Lys Asn Arg Ala
65                  70                  75                  80

Glu Leu Glu Asn Ile Ile Met Glu Glu Asn Gly Lys Pro Val Lys Glu
                85                  90                  95

Ala Lys Glu Glu Val Asp Gly Val Ile Asp Gln Ile Gln Tyr Tyr Ala
            100                 105                 110

Glu Trp Ala Arg Lys Leu Asn Gly Glu Val Val Glu Gly Thr Ser Ser
        115                 120                 125

His Arg Lys Ile Phe Gln Tyr Lys Val Pro Tyr Gly Ile Val Val Ala
    130                 135                 140

Leu Thr Pro Trp Asn Phe Pro Ala Gly Met Val Ala Arg Lys Leu Ala
145                 150                 155                 160

Pro Ala Leu Leu Thr Gly Asn Thr Val Val Leu Lys Pro Ser Ser Asp
                165                 170                 175

Thr Pro Gly Ser Ala Glu Trp Ile Val Arg Lys Phe Val Glu Ala Gly
            180                 185                 190

Val Pro Lys Gly Val Leu Asn Phe Ile Thr Gly Arg Gly Ser Glu Ile
        195                 200                 205

Gly Asp Tyr Ile Val Glu His Lys Lys Val Asn Leu Ile Thr Met Thr
    210                 215                 220

Gly Ser Thr Ala Thr Gly Gln Arg Ile Met Gln Lys Ala Ser Ala Asn
225                 230                 235                 240

Met Ala Lys Leu Ile Leu Glu Leu Gly Gly Lys Ala Pro Phe Met Val
                245                 250                 255

Trp Lys Asp Ala Asp Met Asp Asn Ala Leu Lys Thr Leu Leu Trp Ala
            260                 265                 270

Lys Tyr Trp Asn Ala Gly Gln Ser Cys Ile Ala Ala Glu Arg Leu Tyr
        275                 280                 285

Val His Glu Asp Ile Tyr Asp Thr Phe Met Ser Arg Phe Val Glu Leu
    290                 295                 300

Ser Arg Lys Leu Ala Leu Gly Asp Pro Lys Asn Ala Asp Met Gly Pro
305                 310                 315                 320

Leu Ile Asn Lys Gly Ala Leu Gln Ala Thr Ser Glu Ile Val Glu Glu
                325                 330                 335

Ala Lys Glu Ser Gly Ala Lys Ile Leu Phe Gly Gly Ser Gln Pro Ser
            340                 345                 350

Leu Ser Gly Pro Tyr Arg Asn Gly Tyr Phe Phe Leu Pro Thr Ile Ile
        355                 360                 365

Gly Asn Ala Asp Gln Lys Ser Lys Ile Phe Gln Glu Glu Ile Phe Ala
    370                 375                 380

Pro Val Ile Gly Ala Arg Lys Ile Ser Ser Val Glu Glu Met Tyr Asp
385                 390                 395                 400

Leu Ala Asn Asp Cys Lys Tyr Gly Leu Ala Ser Tyr Leu Phe Thr Lys
                405                 410                 415

Asp Pro Asn Ile Ile Phe Glu Ala Ser Glu Arg Ile Arg Phe Gly Glu
            420                 425                 430

Leu Tyr Val Asn Met Pro Gly Pro Glu Ala Ser Gln Gly Tyr His Thr

435                 440                 445

Gly Phe Arg Met Thr Gly Gln Ala Gly Glu Gly Ser Lys Tyr Gly Ile
    450                 455                 460

Ser Glu Tyr Leu Lys Leu Lys Asn Ile Tyr Val Asp Tyr Ser Gly Lys
465                 470                 475                 480

Pro Leu His Ile Asn Thr Val Arg Asp Asp Leu Phe Gln Ser Gly Arg
                485                 490                 495

Pro Val Leu Gly Ser Ser His His His His His His
            500                 505

<210>  60
<211>  1527
<212>  DNA
<213>  Artificial Sequence

<220>
<223>  TaALDH-F34L-S405C-Chis

<400>  60
atggatacca aactgtatat tgatggccag tgggttaata gcagcagcgg taaaaccgtt      60

gataaatatt ctccggttac cggtcaggtt attggtcgtt tagaagcagc aacccgtgat     120

gatgttgatc gtgcaattga tgcagcagaa gatgcatttt gggcctggaa tgatctgggt     180

agcgttgaac gcagcaaaat tatttatcgt gccaaagaac tgattgaaaa aaatcgtgcc     240

gaactggaaa atattattat ggaagaaaat ggcaaaccgg tgaaagaagc caaagaagaa     300

gtcgacggcg tcattgatca gattcagtat tatgcagaat gggcacgtaa actgaatggt     360

gaagttgttg aaggcaccag cagccatcgt aaaatttttc agtataaagt gccgtatggt     420

attgttgttg cactgacccc gtggaatttt ccggcaggca tggttgcccg taaactggca     480

ccggcactgc tgaccggtaa taccgttgtt ctgaaaccga gcagcgatac accgggtagc     540

gcagaatgga ttgtgcgtaa atttgttgaa gccggtgttc cgaaaggtgt gctgaatttt     600

attaccggtc gtggtagcga aattggcgat tacattgtgg aacataaaaa agtcaatctg     660

attaccatga ccggtagcac cgcaacaggt cagcgcatta tgcagaaagc aagcgcaaat     720

atggcaaaac tgattctgga actgggtggt aaagcaccgt ttatggtttg gaaagatgcc     780

gatatggata atgcactgaa aaccctgctg tgggcaaaat attggaatgc cggtcagagc     840

tgtattgcag cagaacgtct gtatgtgcat gaagatattt atgatacctt tatgagccgt     900

tttgttgaac tgagccgcaa actggcactg ggtgatccga aaaatgcaga tatgggtccg     960

ctgattaata aaggtgcact gcaggcaacc agcgaaattg ttgaagaagc gaaagaatct    1020

ggcgcaaaaa ttctgtttgg tggtagccag ccgagcctga gcggtccgta tcgtaatggc    1080

tatttttttc tgccgaccat tattggtaat gcggatcaga aaagcaaaat ctttcaggaa    1140

gaaatttttg caccggttat tggtgcacgt aaaattagca gcgtggaaga aatgtatgat 1200

ctggccaatg attgcaaata tggtctggcc agctacctgt ttaccaaaga tccgaatatc 1260

atttttgaag ccagcgaacg tattcgtttt ggtgaactgt atgtgaatat gccgggtccg 1320

gaagcaagcc agggttatca caccggtttt cgtatgacag gtcaggcagg cgaaggttct 1380

aaatatggca ttagcgaata tctgaaactg aaaaatattt atgtggatta tagcggcaaa 1440

ccgctgcata ttaataccgt tcgtgatgac ctgtttcaga gcgggagacc tgtgctgggc 1500

agcagccacc accaccacca ccactaa 1527

<210> 61
<211> 508
<212> PRT
<213> Artificial Sequence

<220>
<223> TaALDH-F34L-S405C-Chis

<400> 61

Met Asp Thr Lys Leu Tyr Ile Asp Gly Gln Trp Val Asn Ser Ser Ser
1               5                   10                  15

Gly Lys Thr Val Asp Lys Tyr Ser Pro Val Thr Gly Gln Val Ile Gly
            20                  25                  30

Arg Leu Glu Ala Ala Thr Arg Asp Asp Val Asp Arg Ala Ile Asp Ala
        35                  40                  45

Ala Glu Asp Ala Phe Trp Ala Trp Asn Asp Leu Gly Ser Val Glu Arg
    50                  55                  60

Ser Lys Ile Ile Tyr Arg Ala Lys Glu Leu Ile Glu Lys Asn Arg Ala
65                  70                  75                  80

Glu Leu Glu Asn Ile Ile Met Glu Glu Asn Gly Lys Pro Val Lys Glu
                85                  90                  95

Ala Lys Glu Glu Val Asp Gly Val Ile Asp Gln Ile Gln Tyr Tyr Ala
            100                 105                 110

Glu Trp Ala Arg Lys Leu Asn Gly Glu Val Val Glu Gly Thr Ser Ser
        115                 120                 125

His Arg Lys Ile Phe Gln Tyr Lys Val Pro Tyr Gly Ile Val Val Ala
    130                 135                 140

Leu Thr Pro Trp Asn Phe Pro Ala Gly Met Val Ala Arg Lys Leu Ala
145                 150                 155                 160

Pro Ala Leu Leu Thr Gly Asn Thr Val Val Leu Lys Pro Ser Ser Asp
                165                 170                 175

Thr Pro Gly Ser Ala Glu Trp Ile Val Arg Lys Phe Val Glu Ala Gly
            180                 185                 190

Val Pro Lys Gly Val Leu Asn Phe Ile Thr Gly Arg Gly Ser Glu Ile
        195                 200                 205

Gly Asp Tyr Ile Val Glu His Lys Lys Val Asn Leu Ile Thr Met Thr
    210                 215                 220

Gly Ser Thr Ala Thr Gly Gln Arg Ile Met Gln Lys Ala Ser Ala Asn
225                 230                 235                 240

Met Ala Lys Leu Ile Leu Glu Leu Gly Gly Lys Ala Pro Phe Met Val
                245                 250                 255

Trp Lys Asp Ala Asp Met Asp Asn Ala Leu Lys Thr Leu Leu Trp Ala
            260                 265                 270

Lys Tyr Trp Asn Ala Gly Gln Ser Cys Ile Ala Ala Glu Arg Leu Tyr
        275                 280                 285

Val His Glu Asp Ile Tyr Asp Thr Phe Met Ser Arg Phe Val Glu Leu
    290                 295                 300

Ser Arg Lys Leu Ala Leu Gly Asp Pro Lys Asn Ala Asp Met Gly Pro
305                 310                 315                 320

Leu Ile Asn Lys Gly Ala Leu Gln Ala Thr Ser Glu Ile Val Glu Glu
                325                 330                 335

Ala Lys Glu Ser Gly Ala Lys Ile Leu Phe Gly Gly Ser Gln Pro Ser
            340                 345                 350

Leu Ser Gly Pro Tyr Arg Asn Gly Tyr Phe Phe Leu Pro Thr Ile Ile
        355                 360                 365

Gly Asn Ala Asp Gln Lys Ser Lys Ile Phe Gln Glu Glu Ile Phe Ala
    370                 375                 380

Pro Val Ile Gly Ala Arg Lys Ile Ser Ser Val Glu Glu Met Tyr Asp
385                 390                 395                 400

Leu Ala Asn Asp Cys Lys Tyr Gly Leu Ala Ser Tyr Leu Phe Thr Lys
                405                 410                 415

Asp Pro Asn Ile Ile Phe Glu Ala Ser Glu Arg Ile Arg Phe Gly Glu
            420                 425                 430

Leu Tyr Val Asn Met Pro Gly Pro Glu Ala Ser Gln Gly Tyr His Thr
        435                 440                 445

Gly Phe Arg Met Thr Gly Gln Ala Gly Glu Gly Ser Lys Tyr Gly Ile
    450                 455                 460

Ser Glu Tyr Leu Lys Leu Lys Asn Ile Tyr Val Asp Tyr Ser Gly Lys
465                 470                 475                 480

Pro Leu His Ile Asn Thr Val Arg Asp Asp Leu Phe Gln Ser Gly Arg
                485                 490                 495

Pro Val Leu Gly Ser Ser His His His His His His
            500                 505

<210>  62
<211>  1527
<212>  DNA
<213>  Artificial Sequence

<220>
<223>  TaALDH-F34M-S405N-Chis

<400>  62
atggatacca aactgtatat tgatggccag tgggttaata gcagcagcgg taaaaccgtt      60

gataaatatt ctccggttac cggtcaggtt attggtcgta tggaagcagc aacccgtgat     120

gatgttgatc gtgcaattga tgcagcagaa gatgcatttt gggcctggaa tgatctgggt     180

agcgttgaac gcagcaaaat tatttatcgt gccaaagaac tgattgaaaa aaatcgtgcc     240

gaactggaaa atattattat ggaagaaaat ggcaaaccgg tgaaagaagc caaagaagaa     300

gtcgacggcg tcattgatca gattcagtat tatgcagaat gggcacgtaa actgaatggt     360

gaagttgttg aaggcaccag cagccatcgt aaaatttttc agtataaagt gccgtatggt     420

attgttgttg cactgacccc gtggaatttt ccggcaggca tggttgcccg taaactggca     480

ccggcactgc tgaccggtaa taccgttgtt ctgaaaccga gcagcgatac accgggtagc     540

gcagaatgga ttgtgcgtaa atttgttgaa gccggtgttc cgaaaggtgt gctgaatttt     600

attaccggtc gtggtagcga aattggcgat tacattgtgg aacataaaaa agtcaatctg     660

attaccatga ccggtagcac cgcaacaggt cagcgcatta tgcagaaagc aagcgcaaat     720

atggcaaaac tgattctgga actgggtggt aaagcaccgt ttatggtttg gaaagatgcc     780

gatatggata atgcactgaa aaccctgctg tgggcaaaat attggaatgc cggtcagagc     840

tgtattgcag cagaacgtct gtatgtgcat gaagatattt atgatacctt tatgagccgt     900

tttgttgaac tgagccgcaa actggcactg ggtgatccga aaaatgcaga tatgggtccg     960

ctgattaata aaggtgcact gcaggcaacc agcgaaattg ttgaagaagc gaaagaatct 1020

ggcgcaaaaa ttctgtttgg tggtagccag ccgagcctga gcggtccgta tcgtaatggc 1080

tatttttttc tgccgaccat tattggtaat gcggatcaga aaagcaaaat ctttcaggaa 1140

gaaatttttg caccggttat tggtgcacgt aaaattagca gcgtggaaga aatgtatgat 1200

ctggccaatg ataacaaata tggtctggcc agctacctgt ttaccaaaga tccgaatatc 1260

atttttgaag ccagcgaacg tattcgtttt ggtgaactgt atgtgaatat gccgggtccg 1320

gaagcaagcc agggttatca caccggtttt cgtatgacag gtcaggcagg cgaaggttct 1380

aaatatggca ttagcgaata tctgaaactg aaaaatattt atgtggatta tagcggcaaa 1440

ccgctgcata ttaataccgt tcgtgatgac ctgtttcaga gcgggagacc tgtgctgggc 1500

agcagccacc accaccacca ccactaa 1527

<210> 63
<211> 508
<212> PRT
<213> Artificial Sequence

<220>
<223> TaALDH-F34M-S405N-Chis

<400> 63

Met Asp Thr Lys Leu Tyr Ile Asp Gly Gln Trp Val Asn Ser Ser Ser
1               5                   10                  15

Gly Lys Thr Val Asp Lys Tyr Ser Pro Val Thr Gly Gln Val Ile Gly
            20                  25                  30

Arg Met Glu Ala Ala Thr Arg Asp Asp Val Asp Arg Ala Ile Asp Ala
        35                  40                  45

Ala Glu Asp Ala Phe Trp Ala Trp Asn Asp Leu Gly Ser Val Glu Arg
    50                  55                  60

Ser Lys Ile Ile Tyr Arg Ala Lys Glu Leu Ile Glu Lys Asn Arg Ala
65                  70                  75                  80

Glu Leu Glu Asn Ile Ile Met Glu Glu Asn Gly Lys Pro Val Lys Glu
                85                  90                  95

Ala Lys Glu Glu Val Asp Gly Val Ile Asp Gln Ile Gln Tyr Tyr Ala
            100                 105                 110

Glu Trp Ala Arg Lys Leu Asn Gly Glu Val Val Glu Gly Thr Ser Ser
        115                 120                 125

His Arg Lys Ile Phe Gln Tyr Lys Val Pro Tyr Gly Ile Val Val Ala
    130                 135                 140

Leu Thr Pro Trp Asn Phe Pro Ala Gly Met Val Ala Arg Lys Leu Ala
145                 150                 155                 160

Pro Ala Leu Leu Thr Gly Asn Thr Val Val Leu Lys Pro Ser Ser Asp
                165                 170                 175

Thr Pro Gly Ser Ala Glu Trp Ile Val Arg Lys Phe Val Glu Ala Gly
            180                 185                 190

Val Pro Lys Gly Val Leu Asn Phe Ile Thr Gly Arg Gly Ser Glu Ile
        195                 200                 205

Gly Asp Tyr Ile Val Glu His Lys Lys Val Asn Leu Ile Thr Met Thr
    210                 215                 220

Gly Ser Thr Ala Thr Gly Gln Arg Ile Met Gln Lys Ala Ser Ala Asn
225                 230                 235                 240

Met Ala Lys Leu Ile Leu Glu Leu Gly Gly Lys Ala Pro Phe Met Val
                245                 250                 255

Trp Lys Asp Ala Asp Met Asp Asn Ala Leu Lys Thr Leu Leu Trp Ala
            260                 265                 270

Lys Tyr Trp Asn Ala Gly Gln Ser Cys Ile Ala Ala Glu Arg Leu Tyr
        275                 280                 285

Val His Glu Asp Ile Tyr Asp Thr Phe Met Ser Arg Phe Val Glu Leu
    290                 295                 300

Ser Arg Lys Leu Ala Leu Gly Asp Pro Lys Asn Ala Asp Met Gly Pro
305                 310                 315                 320

Leu Ile Asn Lys Gly Ala Leu Gln Ala Thr Ser Glu Ile Val Glu Glu
                325                 330                 335

Ala Lys Glu Ser Gly Ala Lys Ile Leu Phe Gly Gly Ser Gln Pro Ser
            340                 345                 350

Leu Ser Gly Pro Tyr Arg Asn Gly Tyr Phe Phe Leu Pro Thr Ile Ile
        355                 360                 365

Gly Asn Ala Asp Gln Lys Ser Lys Ile Phe Gln Glu Glu Ile Phe Ala
    370                 375                 380

Pro Val Ile Gly Ala Arg Lys Ile Ser Ser Val Glu Glu Met Tyr Asp
385                 390                 395                 400

Leu Ala Asn Asp Asn Lys Tyr Gly Leu Ala Ser Tyr Leu Phe Thr Lys
                405                 410                 415

Asp Pro Asn Ile Ile Phe Glu Ala Ser Glu Arg Ile Arg Phe Gly Glu
            420                 425                 430

Leu Tyr Val Asn Met Pro Gly Pro Glu Ala Ser Gln Gly Tyr His Thr
        435                 440                 445

Gly Phe Arg Met Thr Gly Gln Ala Gly Glu Gly Ser Lys Tyr Gly Ile
    450                 455                 460

Ser Glu Tyr Leu Lys Leu Lys Asn Ile Tyr Val Asp Tyr Ser Gly Lys
465                 470                 475                 480

Pro Leu His Ile Asn Thr Val Arg Asp Asp Leu Phe Gln Ser Gly Arg
                485                 490                 495

Pro Val Leu Gly Ser Ser His His His His His His
            500                 505

<210>  64
<211>  1527
<212>  DNA
<213>  Artificial Sequence

<220>
<223>  TaALDH-W271S-Chis

<400>  64
atggatacca aactgtatat tgatggccag tgggttaata gcagcagcgg taaaaccgtt      60

gataaatatt ctccggttac cggtcaggtt attggtcgtt ttgaagcagc aacccgtgat     120

gatgttgatc gtgcaattga tgcagcagaa gatgcatttt gggcctggaa tgatctgggt     180

agcgttgaac gcagcaaaat tatttatcgt gccaaagaac tgattgaaaa aaatcgtgcc     240

gaactggaaa atattattat ggaagaaaat ggcaaaccgg tgaaagaagc caaagaagaa     300

gtcgacggcg tcattgatca gattcagtat tatgcagaat gggcacgtaa actgaatggt     360

gaagttgttg aaggcaccag cagccatcgt aaaatttttc agtataaagt gccgtatggt     420

attgttgttg cactgacccc gtggaatttt ccggcaggca tggttgcccg taaactggca     480

ccggcactgc tgaccggtaa taccgttgtt ctgaaaccga gcagcgatac accgggtagc     540

gcagaatgga ttgtgcgtaa atttgttgaa gccggtgttc cgaaaggtgt gctgaatttt     600

attaccggtc gtggtagcga aattggcgat tacattgtgg aacataaaaa agtcaatctg     660

attaccatga ccggtagcac cgcaacaggt cagcgcatta tgcagaaagc aagcgcaaat     720

atggcaaaac tgattctgga actgggtggt aaagcaccgt ttatggtttg gaaagatgcc     780

gatatggata atgcactgaa aaccctgctg tcggcaaaat attggaatgc cggtcagagc     840

tgtattgcag cagaacgtct gtatgtgcat gaagatattt atgatacctt tatgagccgt 900

tttgttgaac tgagccgcaa actggcactg ggtgatccga aaaatgcaga tatgggtccg 960

ctgattaata aaggtgcact gcaggcaacc agcgaaattg ttgaagaagc gaaagaatct 1020

ggcgcaaaaa ttctgtttgg tggtagccag ccgagcctga gcggtccgta tcgtaatggc 1080

tatttttttc tgccgaccat tattggtaat gcggatcaga aaagcaaaat ctttcaggaa 1140

gaaatttttg caccggttat tggtgcacgt aaaattagca gcgtggaaga aatgtatgat 1200

ctggccaatg atagcaaata tggtctggcc agctacctgt ttaccaaaga tccgaatatc 1260

atttttgaag ccagcgaacg tattcgtttt ggtgaactgt atgtgaatat gccgggtccg 1320

gaagcaagcc agggttatca caccggtttt cgtatgacag gtcaggcagg cgaaggttct 1380

aaatatggca ttagcgaata tctgaaactg aaaaatattt atgtggatta tagcggcaaa 1440

ccgctgcata ttaataccgt tcgtgatgac ctgtttcaga gcgggagacc tgtgctgggc 1500

agcagccacc accaccacca ccactaa 1527

<210> 65
<211> 508
<212> PRT
<213> Artificial Sequence

<220>
<223> TaALDH-W271S-Chis

<400> 65

Met Asp Thr Lys Leu Tyr Ile Asp Gly Gln Trp Val Asn Ser Ser Ser
1               5                   10                  15

Gly Lys Thr Val Asp Lys Tyr Ser Pro Val Thr Gly Gln Val Ile Gly
            20                  25                  30

Arg Phe Glu Ala Ala Thr Arg Asp Asp Val Asp Arg Ala Ile Asp Ala
        35                  40                  45

Ala Glu Asp Ala Phe Trp Ala Trp Asn Asp Leu Gly Ser Val Glu Arg
    50                  55                  60

Ser Lys Ile Ile Tyr Arg Ala Lys Glu Leu Ile Glu Lys Asn Arg Ala
65                  70                  75                  80

Glu Leu Glu Asn Ile Ile Met Glu Glu Asn Gly Lys Pro Val Lys Glu
                85                  90                  95

Ala Lys Glu Glu Val Asp Gly Val Ile Asp Gln Ile Gln Tyr Tyr Ala
            100                 105                 110

Glu Trp Ala Arg Lys Leu Asn Gly Glu Val Val Glu Gly Thr Ser Ser

115                 120                 125

His Arg Lys Ile Phe Gln Tyr Lys Val Pro Tyr Gly Ile Val Val Ala
    130                 135                 140

Leu Thr Pro Trp Asn Phe Pro Ala Gly Met Val Ala Arg Lys Leu Ala
145                 150                 155                 160

Pro Ala Leu Leu Thr Gly Asn Thr Val Val Leu Lys Pro Ser Ser Asp
                165                 170                 175

Thr Pro Gly Ser Ala Glu Trp Ile Val Arg Lys Phe Val Glu Ala Gly
            180                 185                 190

Val Pro Lys Gly Val Leu Asn Phe Ile Thr Gly Arg Gly Ser Glu Ile
        195                 200                 205

Gly Asp Tyr Ile Val Glu His Lys Lys Val Asn Leu Ile Thr Met Thr
    210                 215                 220

Gly Ser Thr Ala Thr Gly Gln Arg Ile Met Gln Lys Ala Ser Ala Asn
225                 230                 235                 240

Met Ala Lys Leu Ile Leu Glu Leu Gly Gly Lys Ala Pro Phe Met Val
                245                 250                 255

Trp Lys Asp Ala Asp Met Asp Asn Ala Leu Lys Thr Leu Leu Ser Ala
            260                 265                 270

Lys Tyr Trp Asn Ala Gly Gln Ser Cys Ile Ala Ala Glu Arg Leu Tyr
        275                 280                 285

Val His Glu Asp Ile Tyr Asp Thr Phe Met Ser Arg Phe Val Glu Leu
    290                 295                 300

Ser Arg Lys Leu Ala Leu Gly Asp Pro Lys Asn Ala Asp Met Gly Pro
305                 310                 315                 320

Leu Ile Asn Lys Gly Ala Leu Gln Ala Thr Ser Glu Ile Val Glu Glu
                325                 330                 335

Ala Lys Glu Ser Gly Ala Lys Ile Leu Phe Gly Gly Ser Gln Pro Ser
            340                 345                 350

Leu Ser Gly Pro Tyr Arg Asn Gly Tyr Phe Phe Leu Pro Thr Ile Ile
        355                 360                 365

Gly Asn Ala Asp Gln Lys Ser Lys Ile Phe Gln Glu Glu Ile Phe Ala
    370                 375                 380

Pro Val Ile Gly Ala Arg Lys Ile Ser Ser Val Glu Glu Met Tyr Asp
385                 390                 395                 400

Leu Ala Asn Asp Ser Lys Tyr Gly Leu Ala Ser Tyr Leu Phe Thr Lys
                405                 410                 415

Asp Pro Asn Ile Ile Phe Glu Ala Ser Glu Arg Ile Arg Phe Gly Glu
            420                 425                 430

Leu Tyr Val Asn Met Pro Gly Pro Glu Ala Ser Gln Gly Tyr His Thr
        435                 440                 445

Gly Phe Arg Met Thr Gly Gln Ala Gly Glu Gly Ser Lys Tyr Gly Ile
    450                 455                 460

Ser Glu Tyr Leu Lys Leu Lys Asn Ile Tyr Val Asp Tyr Ser Gly Lys
465                 470                 475                 480

Pro Leu His Ile Asn Thr Val Arg Asp Asp Leu Phe Gln Ser Gly Arg
                485                 490                 495

Pro Val Leu Gly Ser Ser His His His His His His
            500                 505

<210>  66
<211>  1527
<212>  DNA
<213>  Artificial Sequence

<220>
<223>  TaALDH-Y399R-Chis

<400>  66
atggatacca aactgtatat tgatggccag tgggttaata gcagcagcgg taaaaccgtt      60

gataaatatt ctccggttac cggtcaggtt attggtcgtt ttgaagcagc aacccgtgat     120

gatgttgatc gtgcaattga tgcagcagaa gatgcatttt gggcctggaa tgatctgggt     180

agcgttgaac gcagcaaaat tatttatcgt gccaaagaac tgattgaaaa aaatcgtgcc     240

gaactggaaa atattattat ggaagaaaat ggcaaaccgg tgaaagaagc caaagaagaa     300

gtcgacggcg tcattgatca gattcagtat tatgcagaat gggcacgtaa actgaatggt     360

gaagttgttg aaggcaccag cagccatcgt aaaatttttc agtataaagt gccgtatggt     420

attgttgttg cactgacccc gtggaatttt ccggcaggca tggttgcccg taaactggca     480

ccggcactgc tgaccggtaa taccgttgtt ctgaaaccga gcagcgatac accgggtagc     540

gcagaatgga ttgtgcgtaa atttgttgaa gccggtgttc cgaaaggtgt gctgaatttt     600

attaccggtc gtggtagcga aattggcgat tacattgtgg aacataaaaa agtcaatctg     660

attaccatga ccggtagcac cgcaacaggt cagcgcatta tgcagaaagc aagcgcaaat 720

atggcaaaac tgattctgga actgggtggt aaagcaccgt ttatggtttg gaaagatgcc 780

gatatggata atgcactgaa aaccctgctg tgggcaaaat attggaatgc cggtcagagc 840

tgtattgcag cagaacgtct gtatgtgcat gaagatattt atgatacctt tatgagccgt 900

tttgttgaac tgagccgcaa actggcactg ggtgatccga aaaatgcaga tatgggtccg 960

ctgattaata aaggtgcact gcaggcaacc agcgaaattg ttgaagaagc gaaagaatct 1020

ggcgcaaaaa ttctgtttgg tggtagccag ccgagcctga gcggtccgta tcgtaatggc 1080

tatttttttc tgccgaccat tattggtaat gcggatcaga aaagcaaaat ctttcaggaa 1140

gaaatttttg caccggttat tggtgcacgt aaaattagca gcgtggaaga aatgcgtgat 1200

ctggccaatg atagcaaata tggtctggcc agctacctgt ttaccaaaga tccgaatatc 1260

atttttgaag ccagcgaacg tattcgtttt ggtgaactgt atgtgaatat gccgggtccg 1320

gaagcaagcc agggttatca caccggtttt cgtatgacag gtcaggcagg cgaaggttct 1380

aaatatggca ttagcgaata tctgaaactg aaaaatattt atgtggatta tagcggcaaa 1440

ccgctgcata ttaataccgt tcgtgatgac ctgtttcaga gcgggagacc tgtgctgggc 1500

agcagccacc accaccacca ccactaa 1527

<210> 67
<211> 508
<212> PRT
<213> Artificial Sequence

<220>
<223> TaALDH-Y399R-Chis

<400> 67

Met Asp Thr Lys Leu Tyr Ile Asp Gly Gln Trp Val Asn Ser Ser Ser
1               5                   10                  15

Gly Lys Thr Val Asp Lys Tyr Ser Pro Val Thr Gly Gln Val Ile Gly
            20                  25                  30

Arg Phe Glu Ala Ala Thr Arg Asp Asp Val Asp Arg Ala Ile Asp Ala
        35                  40                  45

Ala Glu Asp Ala Phe Trp Ala Trp Asn Asp Leu Gly Ser Val Glu Arg
    50                  55                  60

Ser Lys Ile Ile Tyr Arg Ala Lys Glu Leu Ile Glu Lys Asn Arg Ala
65                  70                  75                  80

Glu Leu Glu Asn Ile Ile Met Glu Glu Asn Gly Lys Pro Val Lys Glu
                85                  90                  95

Ala Lys Glu Glu Val Asp Gly Val Ile Asp Gln Ile Gln Tyr Tyr Ala
            100                 105                 110

Glu Trp Ala Arg Lys Leu Asn Gly Glu Val Val Glu Gly Thr Ser Ser
        115                 120                 125

His Arg Lys Ile Phe Gln Tyr Lys Val Pro Tyr Gly Ile Val Val Ala
    130                 135                 140

Leu Thr Pro Trp Asn Phe Pro Ala Gly Met Val Ala Arg Lys Leu Ala
145                 150                 155                 160

Pro Ala Leu Leu Thr Gly Asn Thr Val Val Leu Lys Pro Ser Ser Asp
                165                 170                 175

Thr Pro Gly Ser Ala Glu Trp Ile Val Arg Lys Phe Val Glu Ala Gly
            180                 185                 190

Val Pro Lys Gly Val Leu Asn Phe Ile Thr Gly Arg Gly Ser Glu Ile
        195                 200                 205

Gly Asp Tyr Ile Val Glu His Lys Lys Val Asn Leu Ile Thr Met Thr
    210                 215                 220

Gly Ser Thr Ala Thr Gly Gln Arg Ile Met Gln Lys Ala Ser Ala Asn
225                 230                 235                 240

Met Ala Lys Leu Ile Leu Glu Leu Gly Gly Lys Ala Pro Phe Met Val
                245                 250                 255

Trp Lys Asp Ala Asp Met Asp Asn Ala Leu Lys Thr Leu Leu Trp Ala
            260                 265                 270

Lys Tyr Trp Asn Ala Gly Gln Ser Cys Ile Ala Ala Glu Arg Leu Tyr
        275                 280                 285

Val His Glu Asp Ile Tyr Asp Thr Phe Met Ser Arg Phe Val Glu Leu
    290                 295                 300

Ser Arg Lys Leu Ala Leu Gly Asp Pro Lys Asn Ala Asp Met Gly Pro
305                 310                 315                 320

Leu Ile Asn Lys Gly Ala Leu Gln Ala Thr Ser Glu Ile Val Glu Glu
                325                 330                 335

Ala Lys Glu Ser Gly Ala Lys Ile Leu Phe Gly Gly Ser Gln Pro Ser
            340                 345                 350

Leu Ser Gly Pro Tyr Arg Asn Gly Tyr Phe Phe Leu Pro Thr Ile Ile

355                 360                 365

Gly Asn Ala Asp Gln Lys Ser Lys Ile Phe Gln Glu Glu Ile Phe Ala
    370                 375                 380

Pro Val Ile Gly Ala Arg Lys Ile Ser Ser Val Glu Glu Met Arg Asp
385                 390                 395                 400

Leu Ala Asn Asp Ser Lys Tyr Gly Leu Ala Ser Tyr Leu Phe Thr Lys
                405                 410                 415

Asp Pro Asn Ile Ile Phe Glu Ala Ser Glu Arg Ile Arg Phe Gly Glu
            420                 425                 430

Leu Tyr Val Asn Met Pro Gly Pro Glu Ala Ser Gln Gly Tyr His Thr
        435                 440                 445

Gly Phe Arg Met Thr Gly Gln Ala Gly Glu Gly Ser Lys Tyr Gly Ile
    450                 455                 460

Ser Glu Tyr Leu Lys Leu Lys Asn Ile Tyr Val Asp Tyr Ser Gly Lys
465                 470                 475                 480

Pro Leu His Ile Asn Thr Val Arg Asp Asp Leu Phe Gln Ser Gly Arg
                485                 490                 495

Pro Val Leu Gly Ser Ser His His His His His His
            500                 505

<210>  68
<211>  1527
<212>  DNA
<213>  Artificial Sequence

<220>
<223>  TaALDH-F34M-W271S-Y399C-S405N-Chis

<400>  68
atggatacca aactgtatat tgatggccag tgggttaata gcagcagcgg taaaaccgtt      60

gataaatatt ctccggttac cggtcaggtt attggtcgta tggaagcagc aacccgtgat     120

gatgttgatc gtgcaattga tgcagcagaa gatgcatttt gggcctggaa tgatctgggt     180

agcgttgaac gcagcaaaat tatttatcgt gccaaagaac tgattgaaaa aaatcgtgcc     240

gaactggaaa atattattat ggaagaaaat ggcaaaccgg tgaaagaagc caaagaagaa     300

gtcgacggcg tcattgatca gattcagtat tatgcagaat gggcacgtaa actgaatggt     360

gaagttgttg aaggcaccag cagccatcgt aaaatttttc agtataaagt gccgtatggt     420

attgttgttg cactgacccc gtggaatttt ccggcaggca tggttgcccg taaactggca     480

ccggcactgc tgaccggtaa taccgttgtt ctgaaaccga gcagcgatac accgggtagc     540

gcagaatgga ttgtgcgtaa atttgttgaa gccggtgttc cgaaaggtgt gctgaatttt 600

attaccggtc gtggtagcga aattggcgat tacattgtgg aacataaaaa agtcaatctg 660

attaccatga ccggtagcac cgcaacaggt cagcgcatta tgcagaaagc aagcgcaaat 720

atggcaaaac tgattctgga actgggtggt aaagcaccgt ttatggtttg gaaagatgcc 780

gatatggata atgcactgaa aaccctgctg tcggcaaaat attggaatgc cggtcagagc 840

tgtattgcag cagaacgtct gtatgtgcat gaagatattt atgatacctt tatgagccgt 900

tttgttgaac tgagccgcaa actggcactg ggtgatccga aaaatgcaga tatgggtccg 960

ctgattaata aaggtgcact gcaggcaacc agcgaaattg ttgaagaagc gaaagaatct 1020

ggcgcaaaaa ttctgtttgg tggtagccag ccgagcctga gcggtccgta tcgtaatggc 1080

tatttttttc tgccgaccat tattggtaat gcggatcaga aaagcaaaat ctttcaggaa 1140

gaaatttttg caccggttat tggtgcacgt aaaattagca gcgtggaaga aatgtgtgat 1200

ctggccaatg ataacaaata tggtctggcc agctacctgt ttaccaaaga tccgaatatc 1260

atttttgaag ccagcgaacg tattcgtttt ggtgaactgt atgtgaatat gccgggtccg 1320

gaagcaagcc agggttatca caccggtttt cgtatgacag gtcaggcagg cgaaggttct 1380

aaatatggca ttagcgaata tctgaaactg aaaaatattt atgtggatta tagcggcaaa 1440

ccgctgcata ttaataccgt tcgtgatgac ctgtttcaga gcgggagacc tgtgctgggc 1500

agcagccacc accaccacca ccactaa 1527

<210> 69
<211> 508
<212> PRT
<213> Artificial Sequence

<220>
<223> TaALDH-F34M-W271S-Y399C-S405N-Chis

<400> 69

Met Asp Thr Lys Leu Tyr Ile Asp Gly Gln Trp Val Asn Ser Ser Ser
1               5                   10                  15

Gly Lys Thr Val Asp Lys Tyr Ser Pro Val Thr Gly Gln Val Ile Gly
            20                  25                  30

Arg Met Glu Ala Ala Thr Arg Asp Asp Val Asp Arg Ala Ile Asp Ala
        35                  40                  45

Ala Glu Asp Ala Phe Trp Ala Trp Asn Asp Leu Gly Ser Val Glu Arg
    50                  55                  60

Ser Lys Ile Ile Tyr Arg Ala Lys Glu Leu Ile Glu Lys Asn Arg Ala
65                  70                  75                  80

Glu Leu Glu Asn Ile Ile Met Glu Glu Asn Gly Lys Pro Val Lys Glu
                85                  90                  95

Ala Lys Glu Glu Val Asp Gly Val Ile Asp Gln Ile Gln Tyr Tyr Ala
            100                 105                 110

Glu Trp Ala Arg Lys Leu Asn Gly Glu Val Val Glu Gly Thr Ser Ser
        115                 120                 125

His Arg Lys Ile Phe Gln Tyr Lys Val Pro Tyr Gly Ile Val Val Ala
    130                 135                 140

Leu Thr Pro Trp Asn Phe Pro Ala Gly Met Val Ala Arg Lys Leu Ala
145                 150                 155                 160

Pro Ala Leu Leu Thr Gly Asn Thr Val Val Leu Lys Pro Ser Ser Asp
                165                 170                 175

Thr Pro Gly Ser Ala Glu Trp Ile Val Arg Lys Phe Val Glu Ala Gly
            180                 185                 190

Val Pro Lys Gly Val Leu Asn Phe Ile Thr Gly Arg Gly Ser Glu Ile
        195                 200                 205

Gly Asp Tyr Ile Val Glu His Lys Lys Val Asn Leu Ile Thr Met Thr
    210                 215                 220

Gly Ser Thr Ala Thr Gly Gln Arg Ile Met Gln Lys Ala Ser Ala Asn
225                 230                 235                 240

Met Ala Lys Leu Ile Leu Glu Leu Gly Gly Lys Ala Pro Phe Met Val
                245                 250                 255

Trp Lys Asp Ala Asp Met Asp Asn Ala Leu Lys Thr Leu Leu Ser Ala
            260                 265                 270

Lys Tyr Trp Asn Ala Gly Gln Ser Cys Ile Ala Ala Glu Arg Leu Tyr
        275                 280                 285

Val His Glu Asp Ile Tyr Asp Thr Phe Met Ser Arg Phe Val Glu Leu
    290                 295                 300

Ser Arg Lys Leu Ala Leu Gly Asp Pro Lys Asn Ala Asp Met Gly Pro
305                 310                 315                 320

Leu Ile Asn Lys Gly Ala Leu Gln Ala Thr Ser Glu Ile Val Glu Glu
                325                 330                 335

Ala Lys Glu Ser Gly Ala Lys Ile Leu Phe Gly Gly Ser Gln Pro Ser
        340                 345                 350

Leu Ser Gly Pro Tyr Arg Asn Gly Tyr Phe Phe Leu Pro Thr Ile Ile
    355                 360                 365

Gly Asn Ala Asp Gln Lys Ser Lys Ile Phe Gln Glu Glu Ile Phe Ala
370                 375                 380

Pro Val Ile Gly Ala Arg Lys Ile Ser Ser Val Glu Glu Met Cys Asp
385                 390                 395                 400

Leu Ala Asn Asp Asn Lys Tyr Gly Leu Ala Ser Tyr Leu Phe Thr Lys
                405                 410                 415

Asp Pro Asn Ile Ile Phe Glu Ala Ser Glu Arg Ile Arg Phe Gly Glu
            420                 425                 430

Leu Tyr Val Asn Met Pro Gly Pro Glu Ala Ser Gln Gly Tyr His Thr
        435                 440                 445

Gly Phe Arg Met Thr Gly Gln Ala Gly Glu Gly Ser Lys Tyr Gly Ile
    450                 455                 460

Ser Glu Tyr Leu Lys Leu Lys Asn Ile Tyr Val Asp Tyr Ser Gly Lys
465                 470                 475                 480

Pro Leu His Ile Asn Thr Val Arg Asp Asp Leu Phe Gln Ser Gly Arg
                485                 490                 495

Pro Val Leu Gly Ser Ser His His His His His His
        500                 505

## Description

### Field of Invention

This invention pertains to an enzymatic process for the production of chemicals from carbon sources. In particular, a process for the production of a target chemical is disclosed using a cell-free enzyme system that converts carbohydrate sources to the intermediate pyruvate and subsequently the intermediate pyruvate to the target chemical.

### Background of the Invention

The development of sustainable, biomass-based production strategies requires efficient depolymerization into intermediate carbohydrates as well as flexible and efficient technologies to convert such intermediate carbohydrates into chemical products. Presently, biotechnological approaches for conversion of biomass to chemicals focus on well established microbial fermentation processes.

However, these fermentative approaches remain restricted to the physiological limits of cellular production systems. Key barriers for cost effective fermentation processes are their low temperature and solvent tolerance, which result in low conversion efficiencies and yields. Additionally, the multitude of cellular metabolic pathways often leads to unintended substrate redirection into non-productive pathways. Despite advances in genetic engineering, streamlining these metabolic networks for optimal product formation at an organismic level is time-consuming and due to the high complexity continues to be rather unpredictable.

A prominent example is the recombinant fermentative production of isobutanol in E. coli. Titers of 1-2 % (v/v) isobutanol already induce toxic effects in the microbial production host, resulting in low product yields (Nature 2008, 451, 86-89). Additionally, depolymerized biomass often contains inhibitory or non-fermentable components that limit microbial growth and product yields. Therefore, strategies are desired to overcome such limitations of cell-based production.

The cell-free production of chemicals has been shown as early as 1897 when Eduard Buchner used a lysate of yeast cells to convert glucose to ethanol (Ber. Dtsch. Chem. Ges. 1901, 34, 1523-1530). Later Welch und Scopes, 1985 demonstrated cell free production of ethanol, a process which, however, was technically not useful (J. Biotechnol. 1985, 2, 257-273). The system lacked specificity and included side reaction of enzymes and unwanted activities in the lysate.

A number of technical processes have been described that use isolated enzymes for the production of chemicals. For example, alcohol dehydrogenases are used in the production of chiral alcohols from ketons requiring cofactor (NAD) regeneration, for example, by adding glucose and glucose dehydrogenase. Such processes have been designed to produce high-value chemicals but not to provide an enzyme system comprising multiple enzyme reactions that convert carbohydrates into chemicals with high energy and carbon efficiency.

Zhang et al. (Biotechnology: Research, Technology and Applications; 2008) describe the idea for cell free enzymatic conversion of glucose to n-butanol. The concept includes a minimum of 18 enzymes, several different cofactors and coenzymes (e.g. ATP, ADP, NADH, NAD, ferredoxin and coenzyme A). In addition the postulated process results in a net-production of ATP so that it requires in addition an ATPase enzyme to remove the ATP. Under practical terms control of ATPase addition while maintaining a balanced ATP level is very difficult to achieve. To manage balanced ATP cycling, the hydrolysis of excess ATP must be adjusted very carefully or eliminated by using highly toxic arsenate. In summary, the described process would be expensive, inefficient and technically instable.

EP2204453 discloses a process for the conversion of glucose to ethanol, n-butanol and/or isobutanol.

There is thus a need for a cost effective process for the production of chemicals from carbohydrates, in particular for the production of chemicals that can be derived from pyruvate such as ethanol, isobutanol and other C4 alcohols.

### Summary of the Invention

A general objective of the invention is to provide stable and technically feasible cell-free processes by minimizing the number of added components. The present invention addresses this need through a cell free enzymatic system, using only a limited number of enzymes and a limited set of cofactors. As a solution, the invention eliminates the need for cells.

Consequently, cell-associated process limitations such as higher process temperatures, extreme pH, and substrate or product toxicity are avoided. By limiting the enzyme activities to those required for the reaction cascade and chosing enzymes with sufficient reaction selectivity undesired substrate redirection into alternative reaction pathways is eliminated. Due to their reduced molecular complexity and rapid adaptability to harsh industrial reaction conditions, designed biocatalytic processes are superior to their cellular counterparts.

The invention is thus directed to a process for the bioconversion of a carbon source, which is preferably a carbohydrate, into a target chemical by an enzymatic process, preferably in the absence of productive living cells. The target chemical is preferably a hydrophobic, a hydrophilic or an intermediate chemical compound.

In particular, according to a preferred aspect, the inventive process does not result in a net production of ATP/ADP and does not involve ATPase and/or arsenate. The inventive process preferably does not involve the cofactor ATP/ADP and/or any phosphorylation reaction.

According to a further preferred aspect, the inventive process uses only **one redox cofactor** in the process. Such a cofactor is preferably selected from NAD/NADH, NADP/NADPH, FAD/FADH2. Preferably, **NAD/NADH** (i.e. the redox pair NAD+ and NADH + H+) is the only cofactor in the process.

According to a further preferred embodiment, the inventive process comprises the adjustment of the enzyme activity of each enzymatically catalyzed reaction step so that it is the same or greater than the activity of any preceding enzymatically catalyzed reaction step.

According to a further preferred aspect, the inventive process uses an artificial minimized reaction cascade that, preferably, only requires one single cofactor. As a result of the current invention, the cell-free production of target chemical from a carbohydrate, in particular to pyruvate, was achieved. Pyruvate can further be converted to other chemicals, such as ethanol and isobutanol. The invention also includes streamlined cascades which function under conditions where microbial productions cease. The current invention is extendible to an array of industrially relevant molecules. Application of solvent-tolerant biocatalysts allows for high product yields, which significantly simplifies downstream product recovery.

According to another preferred aspect the invention only uses enzymes that withstand the inactivating presence of the produced chemicals.

According to one aspect, the present invention concerns a process for the production of **ethanol** and all enzymes employed in the process withstand 2% (w/w) concentration of the product, preferably 4% (w/w), more preferably 6% (w/w), more preferably 8% (w/w), more preferably 10% (w/w), more preferably 12% (w/w), even more preferably 14% (w/w).

According to another aspect, the present invention concerns a process for the production of **isobutanol** and all enzymes employed in the process withstand 2% (w/w) concentration of the product, preferably 4% (w/w), more preferably 6% (w/w), more preferably 8% (w/w), more preferably 10% (w/w), more preferably 12% (w/w), even more preferably 14% (w/w).

According to one aspect, the present invention concerns a process for the production of a target chemical from glucose and/or galactose, or a glucose- and or galactose-containing dimer, oligomer or polymer, by a cell-free enzyme system, comprising the conversion of glucose to pyruvate as an intermediate product; wherein no net production of ATP occurs and, preferably, wherein no phosphorylation reaction occurs; and wherein the conversion of glucose to pyruvate consists of the use of **three, four or five enzymes** selected from the group of **dehydrogenases, dehydratases, and aldolases,** wherein one or more enzymes is selected from each group. Preferably the conversion from glucose to pyruvate is achieved by three or four enzymes, most preferably by **four** enzymes. More preferably, for the conversion of glucose to pyruvate, this process comprises the use of only one redox cofactor and, preferably, one or more of those enzymes requiring such cofactor are **optimized for greater activity towards this cofactor** as compared to the respective non-optimized enzyme or wildtype enyzme. More preferably such redox cofactor is NAD/NADH and one or more enzymes are **optimized for greater activity towards NAD/NADH** as compared to the respective non-optimized enzyme or wildtype enyzme.

Pyruvate is a central intermediate from which molecules like ethanol or isobutanol can be produced with few additional enzymatic steps. The novel cell-free engineering approach allows production of pyruvate, or target chemicals derived from pyruvate. Particular target chemicals that can be derived from pyruvate are **ethanol, n-butanol, 2-butanol and isobutanol,** under reaction conditions that are prohibitive to any cell-based microbial equivalents. As the reaction cascade is designed as a toolbox-system, other products also serve as target compounds.

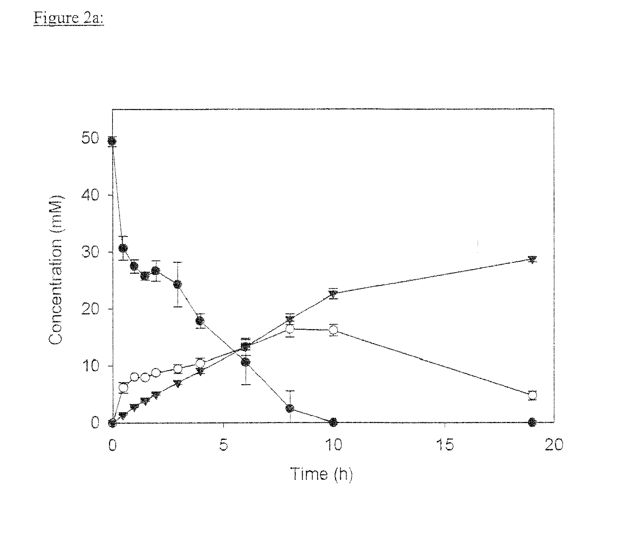

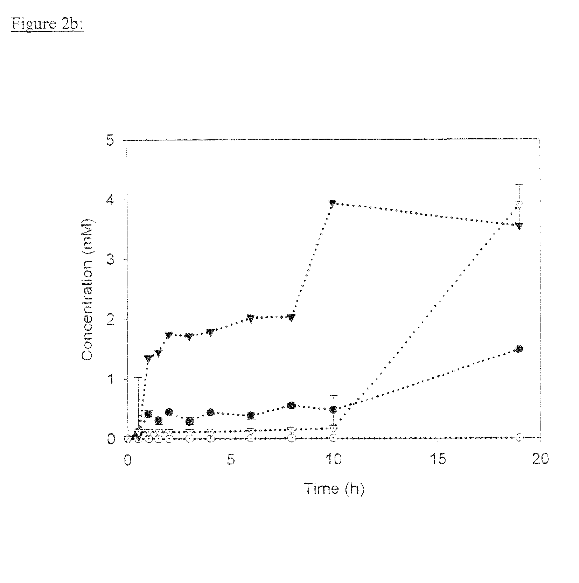

In general, thermostable enzymes from thermophiles are preferred, as they are prone to tolerate higher process temperatures and higher solvent concentrations. Thus, enhanced thermostability allows for increased reaction rates, substrate diffusion, lower viscosities, better phase separation and decreased bacterial contamination of the reaction medium. As demands for substrate selectivity vary at different reaction stages, enzyme fidelity has to be selected accordingly. For example, in the conversion of glucose to the key intermediate pyruvate, DHAD (dihydroxy acid dehydratase) promiscuity allows for parallel conversion of gluconate and glycerate (Fig. 2). In contrast to DHAD, an ALDH (aldehyde dehydrogenase) was chosen that is specific for glyceraldehyde and does not accept other aldehydes such as acetaldehyde or isobutyraldehyde, which are downstream reaction intermediates. These prerequisites were met by an aldehyde dehydrogenase that was able to convert only D-glyceraldehyde to D-glycerate with excellent selectivity. Thus, according to a further preferred aspect, the inventive process can be performed at high temperatures for lengthy periods of time, for example at a temperature range of 40°C - 80°C for at least 30 minutes, preferably 45°C-70°C, and more preferably 50°C-60°C and most preferred at or greater than 50°C. In a particularly preferred embodiment the inventive process employs an ALDH that does not accept acetaldehyde and isobutyraldehyde as substrates.

In order to minimize reaction complexity, the designed pathway may be further consolidated to use coenzyme NADH as the only electron carrier. Provided that subsequent reactions maintain redox-neutrality, pyruvate can be converted to an array of industrial platform chemicals without continuous addition of any electron shuttle.

According to one aspect of the invention, engineered enzymes, for example an ALDH variant with a greater activity for NADH, may also be used, for example resulting from a directed evolution approach. Such optimized enzymes reflecting a greater activity for a specific cofactor, for example NADH, can be applied in combination with minimized enzyme usage during conversion of glucose to pyruvate (e.g. the use of three or four or five enzymes for conversion of glucose to pyruvate), allowing for consolidation of enzyme usage and further improved efficiency and productivity for both conversion of glucose to pyruvate and for the overall conversion of the carbon source to the target organic compound.

Molecular optimization of individual enzymes allows for iterative improvements and extension of the presented cell-free production systems with particular focus on activity, thermal stability and solvent tolerance. In addition, resistance to inhibitors that are present when hydrolysed lignocellulosic biomass is used as feedstock and which can be detrimental to cell-based methods, can be addressed by enzyme engineering.

In regard to and as reflected in the invention, the stability and minimized complexity of the cell-free system eliminate the barriers of current cell-based production, which hamper the wider industrial exploitation of bio-based platform chemicals. Pyruvate is a central intermediate, which may serve as a starting point for cell-free biosynthesis of other commodity compounds. The enzymatic approach demonstrated here is minimized in the number of enzymes and required coenzymes and serves as a highly efficient, cost effective bio-production system.

### Detailed Description of Invention

The present invention concerns a process for the production of a target organic compound from glucose, galactose or mixture of glucose and galactose or a glucose-containing oligomer or polymer and/or a galactose-containing oligomer or polymer by a cell-free enzyme system, comprising the conversion of glucose and/or galactose to pyruvate as an intermediate product; wherein said process comprises the following steps:
(1) oxidation of glucose and/or galactose to gluconate and/or galactonate
(2) conversion of gluconate and/or galactonate to pyruvate and glyceraldehyde
(3) oxidation of glyceraldehyde to glycerate
(4) conversion of glycerate to pyruvate
(5) conversion of pyruvate from steps (2) and (4) to the target compound
wherein steps (1) and (3) are enzymatically catalyzed with one or more enzymes and said steps involve the use of said enzyme(s) to reduce a single cofactor which is added and/or present for electron transport; and wherein step (5) comprises an enzymatically catalyzed reaction involving the reduced form of the cofactor of steps (1) and (3).

Preferred embodiments of this process include the following:

The process of the invention, wherein the process is performed at a temperature of at least 40°C over a period of at least 30 minutes.

The process of the invention, wherein the temperature of the process is maintained in a range from 40-80°C, preferably in a range from 45°-70°C, more preferably in a range from 50°-60°C, and most preferred at or greater than 50°.

The process of the invention, wherein the process is maintained at the given temperature for at least 30 minutes, preferably at least 3 hours, more preferably at least 12 hours, even more preferably for at least 24 hours, most preferred for at least 48 hours, and most highly preferred for at least 72 hours.

The process of the invention, wherein step (1) of the process comprises the use of a single dehydrogenase for the oxidation of glucose and/or galactose to gluconate and/or galactonate.

The process of the invention, wherein step (1) is catalyzed by a single enzyme.

The process of the invention, wherein step (1) comprises the use of a dehydrogenase.

The process of the invention, wherein step (2) comprises the conversion of gluconate to 2-keto-3-deoxygluconate and of 2-keto-3-deoxygluconate to glyceraldehyde and pyruvate.

The process of the invention, wherein step (2) comprises the use of dehydroxy acid dehydratase and keto-3-deoxygluconate aldolase.

The process of the invention, wherein step (3) comprises the use of a dehydrogenase for the oxidation of glyceraldehyde to glycerate.

The process of the invention, wherein steps (1) and (3) are carried out with the use of a single dehydrogenase.

The process of the invention, wherein no net production of ATP occurs.

The process of the invention, wherein no ATPase or arsenate is added.

The process of the invention, wherein said process occurs without ATP and/or ADP as cofactors.

The process of the invention, wherein the single cofactor is selected from the group consisting of NAD/NADH, NADP/NADPH, and FAD/FADH2.

The process of the invention, wherein the single cofactor is NAD/NADH.

The process of the invention, wherein glucose is a preferred starting material.

The process of the invention, wherein the enzyme activity of each enzymatically catalyzed reaction step is adjusted so that it is the same or greater than the activity of any preceding enzymatically catalyzed reaction step.

The process of the invention, wherein the specific enzymatic activity when using glyceraldehyde as a substrate is at least 100 fold greater than using either acetaldehyde or isobutyraldehyde as a substrate, more preferably at least 500 fold greater, even more preferably at least 800 fold greater, most preferably at least 1000 fold greater.

The process of the invention, whereby
- for the conversion of glucose and/or galactose to pyruvate only enzymes selected from the group of dehydrogenases, dehydratases, and aldolases are used; and
- wherein one or more of said enzymes is selected from each group.

Preferred dehydrogenases, dehydratases, and aldolases are dehydrogenases belonging to EC 1.1.1.47 or EC 1.2.1.3, dehydratases belonging to EC 4.2.1.9 or 4.2.1.39, and aldolases belonging to EC 4.1.2.14.

The process of the invention, wherein the conversion of glucose and/or galactose to pyruvate consists of the use of one or two dehydrogenases, one or two dehydratases, and one aldolase.

The process of the invention, wherein the conversion of glucose and/or galactose to pyruvate consists of the use of two dehydrogenases, one dehydratase, and one aldolase.

The process of the invention, wherein the conversion of glucose and/or galactose to pyruvate consists of the use of one dehydrogenases, one dehydratase, and one aldolase.

The process of the invention, wherein one of the enzyme combinations included in any one of the tables P-1, P-2-a, P-2-b, P-2-c, P-2-d, P-3-a, P-3-b, and P-3-c are employed for the conversion of glucose and/or galactose to pyruvate.

The process of the invention, wherein the enzymes used for the conversion of glucose and/or galactose to pyruvate are selected from the group consisting of Glucose dehydrogenase GDH (EC 1.1.1.47) from Sulfolobus solfataricus, NP 344316.1, Seq ID 02; Dihydroxy acid dehydratase DHAD (EC 4.2.1.9) from Sulfolobus solfataricus, NP 344419.1, Seq ID 04; Gluconate dehydratase (EC 4.2.1.39) from Sulfolobus solfataricus, NP_344505; Gluconate dehydratase (EC 4.2.1.39) from Sulfolobus solfataricus, NP_344505, Mutation I9L; Gluconate dehydratase ilvEDD (EC 4.2.1.39) from Achromobacter xylsoxidans; Gluconate dehydratase ilvEDD (EC 4.2.1.39) from Metallosphaera sedula DSM 5348; Gluconate dehydratase ilvEDD (EC 4.2.1.39) from Thermoplasma acidophilum DSM 1728; Gluconate dehydratase ilvEDD (EC 4.2.1.39) from Thermoplasma acidophilum DSM 1728; 2-Keto-3-deoxygluconate aldolase KDGA (EC 4.1.2.14) from Sulfolobus solfataricus, NP 344504.1; 2-Keto-3-deoxygluconate aldolase KDGA (EC 4.1.2.14) from Sulfolobus acidocaidaricus, Seq ID 06; Aldehyde Dehydrogenase ALDH (EC 1.2.1.3) from Flavobacterium frigidimaris, BAB96577.1; Aldehyde Dehydrogenase ALDH (EC 1.2.1.3) from Thermoplasma acidophilum, Seq ID 08; Aldehyde Dehydrogenase ALDH (EC 1.2.1.3) from Thermoplasma acidophilum, Mutations F34M + Y399C + S405N, Seq ID 10; Glycerate kinase (EC 2.7.1.) from Sulfolobus solfataricus, NP_342180.1; Glycerate 2-kinase (EC 2.7.1.165) from Sulfolobus tokodaii, Uniprot Q96YZ3.1; Enolase (EC 4.2.1.11) from Sulfolobus solfataricus, NP 342405.1; Pyruvate Kinase (EC 2.7.1.40) from Sulfolobus solfataricus, NP 342465.1; Glycerate dehydrogenase/hydroxypyruvate reductase (EC 1.1.1.29 / 1.1.1.81) from Picrophilus torridus, YP_023894.1; Serine-pyruvate transaminase (EC 2.6.1.51) from Sulfolobus solfataricus, NCBI Gen ID: NP_343929.1; L-serine ammonia-lyase (EC 4.3.1.17) from Thermus thermophilus, YP_144295.1 and YP_144005.1; and Alanine dehydrogenase (EC 1.4.1.1) from Thermus thermophilus, NCBI-Gen ID: YP_005739.1.

The process of the invention, wherein the conversion of glucose to pyruvate consists of the conversion of one mole of glucose to two moles of pyruvate.

The process of the invention, wherein pyruvate is further converted to a target chemical, and wherein during such conversion 1 (one) molecule NADH is converted to 1 (one) molecule NAD per molecule pyruvate, and wherein such target chemical is preferably selected from ethanol, isobutanol, n-butanol and 2-butanol.

The process of the invention, wherein one of the enzyme combinations included in any one of the tables E-1, I-1, N-1, T-1, T-2, and T-2a are employed for the conversion of pyruvate to the respective target chemical.

The process of the invention, wherein the target chemical is ethanol and the enzymes used for the conversion of pyruvate to ethanol are selected from the group consisting of Pyruvate decarboxylase PDC (EC 4.1.1.1) from Zymomonas mobilis, Seq ID 20; and Alcohol dehydrogenase ADH (EC 1.1.1.1) from Geobacillus stearothermophilus, Seq ID 18.

The process of the invention, wherein the target chemical is isobutanol and the enzymes used for the conversion of pyruvate to isobutanol are selected from the group consisting of Acetolactate synthase ALS (EC 2.2.1.6) from Bacillus subtilis, Seq ID 12; Acetolactate synthase ALS (EC 2.2.1.6) from Sulfolobus solfataricus, NCBI-GenID: NP_342102.1; Acetolactate synthetase ALS (EC: 2.2.1.6) from Thermotoga maritima, NCBI-GeneID: NP_228358.1; Ketol-acid reductoisomerase KARI (EC 1.1.1.86) from Meiothermus ruber, Seq ID 14; Ketol-acid reductoisomerase KARI (EC 1.1.1.86) from Sulfolobus solfataricus, NCBI-GenID: NP_342100.1; Ketol-acid reductoisomerase KARI (EC. 1.1.1.86) from Thermotoga maritima, NCBI-GeneID: NP_228360.1; Branched-chain-2-oxo acid decarboxylase KDC (EC 4.1.1.72) from Lactococcus lactis, Seq ID 16; α-Ketoisovalerate decarboxylase KDC, (EC 4.1.1.-) from Lactococcus lactis, NCBI-GeneID: CAG34226.1; Dihydroxy acid dehydratase DHAD (EC 4.2.1.9) from Sulfolobus solfataricus, NP 344419.1, Seq ID 04; Dihydroxy-acid dehydratase DHAD, (EC: 4.2.1.9) from Thermotoga maritima, NCBI-GeneID: NP_228361.1; Alcohol dehydrogenase ADH (EC 1.1.1.1 from Geobacillus stearothermophilus, Seq ID 18; Alcohol dehydrogenase ADH (EC 1.1.1.1) from Flavobacterium frigidimaris, NCBI-GenID: BAB91411.1; and Alcohol dehydrogenase ADH (EC: 1.1.1.1) from Saccharomyces cerevisiae.

The process of the invention, wherein the product is removed from the reaction continuously or in a batch mode, preferably by extraction, perstraction, distillation, adsorption, gas stripping, pervaporation, membrane extraction or reverse osmosis.

The process of the invention, wherein the solvent tolerance of the used enzymes for the respective target chemical is preferably better than 1% (w/w), more preferably better than 4% (w/w), even more preferably better than 6% (w/w) and most preferably better than 10% (w/w).

The process of the invention, wherein the enzyme or enzymes involved in steps (1) and/or (3) is/are optimized for the single cofactor by having a higher specific activity to said cofactor.

The process of the invention, wherein the optimized enzyme has a sequence identity of at least 50%, preferably 70%, more preferably 80%, even more preferably 90%, even more preferably 95%, most preferably 97%, most highly preferred 99% as compared to either SEQ ID NO. 8 or SEQ ID NO. 2 and has an improved specific activity to said cofactor as compared to SEQ ID NO. 8 or SEQ ID NO. 2, respectively.

The process of the invention, wherein the enzyme has a specific activity of 0.4 U/mg or more to said cofactor at 50°C and pH 7.0 with glyceraldehyde/glycerate as substrates at 1 mM and the said cofactor at 2 mM in a total reaction volume of 0.2 ml. A preferred embodiment is wherein the specific activity is 0.6 U/mg or more, more preferably 0.8 U/mg or more, even more preferably 1.0 U/mg or more, most preferably 1.2 U/mg or more, and most highly preferred 1.5 U/mg or more.

The process of the invention, wherein the specific activity is measured in the presence of 3% isobutanol.

The process of the invention, wherein the enzyme involved in steps (1) and/or (3) is aldehyde dehydrogenase.

The process of the invention, wherein the aldehyde dehydrogenase belongs to the class EC 1.1.1.47.

The process of the invention, wherein the aldehyde dehydrogenase is selected from the group consisting of SEQ ID NO: 10, SEQ ID NO 57, SEQ ID NO 59, SEQ ID NO 61, SEQ ID NO 63, SEQ ID NO 65, SEQ ID NO 67, SEQ ID NO 69.

The above embodiments may be combined to provide further specific embodiments of the invention.

The present invention is directed to a cell-free process for the biotechnological production of target chemicals from carbohydrate sources, in particular of alcohols, including C2 alcohols such as ethanol, and C4 alcohols such as n-butanol, isobutanol or 2-butanol. Products include hydrophobic, hydrohilic, and intermediate chemicals. A most preferred hydrophobic chemical of the current invention is isobutanol. A most preferred hydrophilic and intermediate chemical of the current invention is ethanol.

According to a preferred aspect, the invention discloses a process for the production of a target chemical from a carbohydrate source by a cell-free enzyme system, comprising the conversion of glucose to pyruvate as an intermediate product wherein no phosphorylation reaction and no net production of ATP occurs.

As used herein, the term "enzyme" encompasses also the term "enzyme activity" and may be used interchangeably.

### Selection of chemical routes for the conversion of glucose to pyruvate:

According to a preferred aspect, the invention discloses a process for the production of a target chemical from glucose and/or galactose or a glucose- and/or galactose-containing dimer, oligomer or polymer by a cell-free enzyme system, wherein one molecule glucose or galactose is converted to one molecule pyruvate and one molecule glycerate without net production of ATP, and wherein the glycerate is converted to pyruvate. Preferably such glucose is converted via the intermediate gluconate (or gluconic acid) to 2-keto-3-deoxy-gluconate (or 2-keto-3-deoxy-gluconic acid). Preferably such galactose is converted via the intermediate galactonate (or galactonic acid) to 2-keto-3-deoxy-galactonate (or 2-keto-3-deoxy-galactonic acid). Preferably such 2-keto-3-deoxy-gluconate or 2-keto-3-deoxy-galactonate is converted to one molecule pyruvate and one molecule glyceraldehyde. Preferably such glyceraldehyde is converted to glycerate.

Different options exist for the conversion of glycerate to pyruvate. In one preferred aspect the glycerate is converted via glycerate-2-phosphate and phosphoenolpyruvate to pyruvate. In another preferred aspect glycerate is converted via hydroxypyruvate and serine to pyruvate. In another preferred aspect glycerate is converted directly to pyruvate.

### Selection of enzymes for the conversion of glucose to pyruvate:

According to a preferred aspect, the invention discloses a process for the production of a target chemical from glucose and/or galactose or a glucose- and/or galactose-containing dimer, oligomer or polymer by a cell-free enzyme system, comprising the conversion of glucose and/or galactose to pyruvate as an intermediate product; wherein no net production of ATP occurs; and wherein the conversion of glucose and/or galactose to pyruvate consists of the use of **three, four or five** enzymes, preferably three to four and most preferred four enzymes, selected from the group of **dehydrogenases, dehydratases, and aldolases,** wherein one or more enzymes is be selected from each group.

Different preferred options to minimize the number of enzymes in the conversion of glucose to pyruvate are disclosed:
(1) A particularly preferred aspect of the invention is wherein said enzymes are a dehydrogenase (EC 1.1.1.47) accepting gluconate/glucose and glycerate/glyceraldehyde as substrates, a dihydroxyacid dehydratase (EC 4.2.1.9) accepting gluconate/2-keto-3-deoxygluconate and glycerate/pyruvate as substrates, and a 2-keto-3-deoxy gluconate aldolase (EC 4.1.2.14) accepting 2-keto-3-deoxy gluconate/pyruvate/glyceraldehyde as substrate. (i.e. **3 enzymes)**
(2) A particularly preferred aspect of the invention is wherein said enzymes are a glucose dehydrogenase (EC 1.1.1.47) accepting gluconate/glucose as substrate, an aldehyde dehydrogenase (EC 1.2.1.3) accepting glycerate/glyceraldehyde as substrate, a dihydroxyacid dehydratase (EC 4.2.1.9 or 4.2.1.39) accepting gluconate/2-keto-3-deoxygluconate and glycerate/pyruvate as substrates, and a 2-keto-3-deoxy gluconate aldolase (EC 4.1.2.14) accepting 2-keto-3-deoxy gluconate/pyruvate/glyceraldehyde as substrate. (i.e. **4 enzymes)**
(3) A particularly preferred aspect of the invention is wherein said enzymes are a dehydrogenase (EC 1.1.1.47 or 1.2.1.3) accepting gluconate/glucose and glycerate/glyceraldehyde as substrates, two different dihydroxyacid dehydratases accepting gluconate/2-keto-3-deoxygluconate (EC 4.2.1.39) and glycerate/pyruvate (EC 4.2.1.9) as substrates, and a 2-keto-3-deoxy gluconate aldolase (EC 4.1.2.14) accepting 2-keto-3-deoxy gluconate/pyruvate/glyceraldehyde as substrate. (i.e. **4 enzymes)**
(4) A particularly preferred aspect of the invention is wherein said enzymes are a glucose dehydrogenase (EC 1.1.1.47) accepting gluconate/glucose as substrate, two different dihydroxyacid dehydratases (EC 4.2.1.9) accepting gluconate/2-keto-3-deoxygluconate and glycerate/pyruvate as substrates, an aldehyde dehydrogenase (EC 1.2.1.3) accepting glycerate/glyceraldehyde as substrates, and a 2-keto-3-deoxy gluconate aldolase (EC 4.1.2.14) accepting 2-keto-3-deoxy gluconate/pyruvate and glyceraldehyde as substrate. (i.e. **5 enzymes)**
(5) A particularly preferred aspect of the invention is wherein said enzymes are a glucose dehydrogenase (EC 1.1.1.47) accepting gluconate/glucose as substrate, an aldehyde dehydrogenase (EC 1.2.1.3) accepting gluconate/glucose and glycerate/glyceraldehyde as substrates, two different dihydroxyacid dehydratases (EC 4.2.1.9) accepting gluconate/2-keto-3-deoxygluconate and glycerate/pyruvate as substrates, and a 2-keto-3-deoxy gluconate aldolase (EC 4.1.2.14) accepting 2-keto-3-deoxy gluconate/pyruvate/glyceraldehyde as substrate. (i.e. **5 enzymes)**

Most enzymes can promiscuously catalyze reactions and act on different substrates. These enzymes will not only catalyze one particular reaction, but they are specific for a particular type of chemical bond or functional group. This promiscuity can be utilized in the reaction cascade of the current invention by converting several substrates to the wanted products by one enzyme.

In order to identify suitable promiscuous enzymes the activity of a relevant enzyme for a substrate or a cofactor may be determined where applicable with a photometrical enzyme assay in micro titer plates to allow a high throughput. Enzyme activity is defined as the amount of enzyme necessary to convert 1 µmol substrate to the desired product per minute. An enzyme having a specific activity per mg enzyme of 0.01 or more, preferably 0.1 or more, more preferably 1 or more, even more preferably 10 or more, most preferably 100 or more, most highly preferred 1000 or more for a tested substrate or cofactor is to be considered as an enzyme accepting a particular substrate and/or cofactor.

### Selection of the carbon source and/or carbohydrate source:

**Carbon source** can be any material which can be utilized by microorganisms for growth or production of chemicals. These include carbohydrates and derivatives: polyoses such as cellulose, hemicellulose, starch; bioses such as sucrose, maltose, lactose; hexoses such as glucose, mannose, galactose; pentoses such as xylose, arabinose; uronic acids, glucosamines etc.; polyols such as sorbitol, glycerol; lipids and derivatives, lignin and derivatives. Particularly preferred carbon sources are **carbohydrates,** such as glucose, a glucose-containing oligomer or polymer, a non-glucose monomeric hexose, or mixtures thereof.

According to a preferred aspect, the **carbohydrate source** is glucose, galactose, or a mixture of glucose and galactose, or a hydrolysate of a glucose- and/or galactose containing disaccharide, oligosaccharide, or polysaccharide.

In a particularly preferred aspect, the carbohydrate source is **cellulose or lignocellulose,** whereby the cellulose is hydrolysed first by the use of cellulases to glucose, and/or whereby cellulase activity is added to the enzyme mixture. For one embodiment of the invention, lignocellulosic material is converted to glucose using only 3 to 6 enzymes (preferably 1 or 2 or 3 endocellulase, 1 or 2 exocellulase, 1 beta-glucosidase).

In a particularly preferred aspect, the carbohydrate source is **starch,** whereby the starch is hydrolysed first, and/or whereby amylase and glucoamylase activity is added to the enzyme mixture.

In another particularly preferred aspect, the carbohydrate source is **sucrose,** whereby an invertase is added to the enzyme mixture to hydrolyse the sucrose and an isomerase is added to convert fructose to glucose.

In another particularly preferred aspect, the carbohydrate source is **lactose** whereby the lactose is hydrolysed with a galactosidase first, and/or whereby a galactosidase is added to the enzyme mixture.

### Examples of products that can be produced from pyruvate:

According to a further preferred aspect, the target chemical is ethanol, a four-carbon monoalcohol, in particular n-butanol, iso-butanol, 2-butanol, or another chemical derivable from pyruvate, preferably by an enzymatic pathway. A most preferred hydrophobic chemical of the current invention is isobutanol. A most preferred target chemical of the current invention is ethanol.

**Hydrophobic** chemicals according to the invention comprise, without limitation, C4 alcohols such as n-butanol, 2-butanol, and isobutanol, and other chemicals that have a limited miscibility with water. Limited miscibility means that at room temperature not more than 20% (w/w) can be mixed with water without phase separation. **Hydrophilic** and intermediate chemicals according to the invention comprise, without limitation ethanol and other chemicals.

A **hydrophobic chemical** is a chemical which is only partially soluble in water and which resides in the solid or liquid state at ambient pressure and temperature. Hydrophobic chemicals have a limited miscibiliy with water of not more than 20% (w/w) without phase separation. Particular examples of hydrophobic chemicals according to the present invention include n-butanol, 2-butanol and isobutanol.

**n-Butanol** is a colorless, neutral liquid of medium volatility with restricted miscibility (about 7-8% at 20°C in water. n-Butanol is used as an intermediate in the production of chemicals, as a solvent and as an ingredient in formulated products such as cosmetics. n-Butanol is used in the synthesis of acrylate/methacrylate esters, glycol ethers, n-butyl acetate, amino resins and n-butylamines. n-Butanol can also be used as a fuel in combustion engines due to low vapor pressure, high energy content and the possibility to be blended with gasoline at high concentrations. **n-Butanol** can be produced using solventogenic Clostridia, such as C. acetobuylicum or C. beijerinckii, typically producing a mixture of n-butanol, acetone and ethanol. Butanol production using solventogenic clostridia has several drawbacks: (i) Product isolation from dilute aqueous solution is very expensive as it is either elaborate (e.g. using membrane processes) or energy consuming (e.g. using distillation). (ii) The yield is low as significant parts of the substrate go into the formation of byproducts such as acetone, ethanol, hydrogen and biomass. (iii) The productivity of butanol production is low due to limited cell titers. (iv) The complex metabolism limits metabolical engineering for higher productivity and yield. (v) Limited process stability often leads to production losses and sterility is difficult to maintain. (vi) The biphasic nature of clostridial growth limits process flexibility and productivity.

**2-Butanol** is a colorless, neutral liquid of medium volatility with restricted miscibility (about 12 % at 20°C) in water. 2-Butanol is used as solvent for paints and coatings as well as food ingredients or in the production of 1-buten.

**Isobutanol** is a colorless, neutral liquid of medium volatility with restricted miscibility (about 9-10 % at 20°C) in water. Isobutanol is used as solvent or as plasticizer. It is also used in the production of isobuten which is a precursor for the production of MTBE or ETBE.

### Selection of cofactor requirements:

According to a preferred embodiment of the invention, the conversion of glucose to pyruvate as an intermediate product works completely **without a net production of ATP and/or ADP** as cofactors. More preferably, the overall conversion of the carbon source to the target chemical (the reaction pathway) works completely without a net production of ATP and/or ADP as cofactors.

According to a preferred embodiment of the invention, the conversion of glucose to pyruvate as an intermediate product works completely **without ATP and/or ADP as cofactors.** More preferably, the overall conversion of the carbon source to the target chemical (the reaction pathway) works completely without ATP and/or ADP as cofactors.

According to a preferred embodiment of the invention, the conversion of glucose to pyruvate as an intermediate product works completely **without any phosphorylation reaction.** More preferably, the overall conversion of the carbon source to the target chemical (the reaction pathway) works completely without any phosphorylation reaction.

According to a preferred embodiment of the invention, the conversion of glucose to pyruvate as an intermediate product works completely **without addition of any cofactor except redox cofactors.** More preferably, the overall conversion of the carbon source to the target chemical (the reaction pathway) works completely without addition of any cofactor as metabolic intermediate except redox cofactors. Preferred redox cofactors are FMN/FMNH2, FAD/FADH2, NAD/NADH, and/or NADP/NADPH.

According to a preferred embodiment of the invention, the conversion of glucose to pyruvate as an intermediate product works when only **a single redox cofactor is added** for the redox reactions for either the conversion of glucose to pyruvate and/or for the overall conversion of the carbon source to the target organic compound; a particularly **preferred single cofactor is NAD/NADH.**

### Enzymes optimized for greater activity towards cofactor:

According to one aspect of the invention, for the conversion of glucose to pyruvate, the process of the present invention preferably comprises the use of only one redox cofactor and, preferably, one or more of those enzymes requiring such cofactor are **optimized for greater activity towards this cofactors** as compared to the respective non-optimized enzyme or wildtype enyzme. More preferably such redox cofactor is NAD/NADH and one or more enzymes are **optimized for greater activity towards NAD/NADH** as compared to the respective non-optimized enzyme or wildtype enzyme; even more preferable is that the optimized enzyme is one or more dehydrogenases; even more preferred is that the optimized enzyme is one or more of the following:
- a glucose dehydrogenase (EC 1.1.1.47) accepting glucose or galactose as a substrate, or
- an aldehyde dehydrogenase (EC 1.2.1.3) accepting glyceraldehyde as a substrate, or
- a glucose dehydrogenase (EC 1.1.1.47) accepting glucose or galactose as a substrate and accepting glyceraldehyde as a substrate, or
- an aldehyde dehydrogenase (EC 1.2.1.3) accepting glucose or galactose as a substrate and accepting glyceraldehyde as a substrate,

The combined use of one or more optimized enzymes for greater cofactor activity with a reduced number of enzymes as herein described results in a further improved process for the conversion of glucose to pyruvate and thus ultimately a further improved process for the production of the target chemical.

According to a preferred aspect of the invention, the conversion of glucose to pyruvate comprises the use of one or more enzymes optimized for greater cofactor activity, preferably greater NADH activity, as compared to the respective non-optimized enzyme. A greater cofactor activity includes, for example, improved acceptance of an enzyme of the cofactor as a substrate. An enzyme optimized for greater cofactor activity, preferably greater NADH activity, as compared to the respective non-optimized enzyme is an enzyme which has been engineered as a variant to the non-optimized enzyme resulting in a greater cofactor activity, preferably greater NADH activity. One example of optimization is the use of a directed evolution approach. A variant to the non-optimized enzyme resulting in greater cofactor activity, preferably greater NADH activity, is an enzyme which is encoded by a nucleotide sequence which consists of at least one nucleotide that differs from the nucleotide sequence encoding the enzyme which is non-optimized (i.e. the comparative enzyme), or likewise a variant to the non-optimized enzyme resulting in a greater cofactor activity, preferably greater NADH activity, is an enzyme which consists of at least one amino acid which differs from the amino acid sequence for the enzyme which is non-optimized (i.e. the comparative enzyme). The non-optimized enzyme may be a wildtype enzyme. SEQ ID NO. 8 is a preferred example of a wildtype enzyme enzyme, which is a non-optimized enzyme. Another example is that of SEQ ID NO. 2.

Improved cofactor activity may be measured in absolute amounts, or, alternatively by relative amounts. For example, the specific activity of the purified form of the enzyme may be used. In such a case, in the current invention an improved activity, meaning that which defines an optimized enzyme, to a specific cofactor is when the enzyme has a specific activity of 0.4 U/mg or more to said cofactor at 50°C and pH 7.0 with glyceraldehyde/glycerate as substrates at 1 mM and the specific cofactor at 2 mM in a total reaction volume of 0.2 ml. A preferred cofactor is NAD/NADH. It is a preferred embodiment for the specific activity to be 0.6 U/mg or more, preferably 0.8 U/mg or more, more preferably 1.0 U/mg or more, most preferably 1.2 U/mg or more, and most highly preferred 1.5 U/mg or more under these conditions. A further preferred embodiment is wherein the specific activity as indicated above is measured in the presence of 3% isobutanol. Alternatively, the specific acitivity is measured and normalized to the comparative wildtype (e.g. non-optimized enzyme) and an improved activity, meaning that which defines an optimized enzyme, is one which is 2 fold greater or more than the wildtype value, preferably 4 fold greater or more, even more preferably 8 fold greater or more, most preferably 16 fold greater or more, most highly preferred 32 fold greater or more.

Alternatively, relative values can be made using standard protocols and normalizing to the wildtype values. For example, colonies of cells, (e.g. E. coli) containing a library of enzyme variants (e.g. dehydrogenase variants; Ta-ALDH) are induced, grown, harvested, lysed and insoluble cell debris is removed, supernatants containing the variants can be tested for relative activity under standard conditions (e.g. 2 mM NAD, 1 mM D-glyceraldehyde in 50 mM HEPES (pH 7) at 50°C). After a given period (e.g. 20 min), cofactor formation (e.g. NADH formation) can be detected spectrophotometrically and superior cofactor (e.g. NADH formation) as compared to wild type can be detected and is provided as improved relative activity to the wildtype of the variant of interest. In particular, an improved activity, meaning that which defines an optimized enzyme, is one which is 2 fold greater or more than the wildtype value, preferably 4 fold greater or more, even more preferably 8 fold greater or more, most preferably 16 fold greater or more, most highly preferred 32 fold greater or more.

The optimized enzyme resulting in a greater cofactor activity, preferably greater NADH activity, may be encoded by a nucleotide sequence which consists of substitutions, additions or deletions to the nucleotide sequence encoding the non-optimized enzyme, wherein the substitutions, additions or deletions include 1-60 nucleotides, preferably 1-21 nucleotide, more preferably 1-9 nucleotides, in particularly preferred 1-3 nucleotides, most particularly preferred 1 nucleotide. The substitutions, additions or deletions to the nucleotide sequence encoding the non-optimized enzyme resulting in an optimized enzyme with a greater cofactor activity, preferably greater NADH activity, may be defined as resulting in 80% nucleotide sequence identity to the non-optimized enzyme, preferably 90% nucleotide sequence identity, more preferably 95% nucleotide sequence identity, most preferred 97% nucleotide sequence identity, most highly preferred 99% nucleotide sequence identity. The non-optimized enzyme may be a wildtype enzyme. SEQ ID NO. 8 is a preferred example of a wildtype enzyme enzyme, which is a non-optimized enzyme. Another example is that of SEQ ID NO. 2.

Comparison of sequence identity (alignments) is performed using the ClustalW Algorithm (Larkin M.A., Blackshields G., Brown N.P., Chenna R., McGettigan P.A., McWilliam H., Valentin F., Wallace I.M., Wilm A., Lopez R., Thompson J.D., Gibson T.J. and Higgins D.G. (2007) ClustalW and ClustalX version 2. Bioinformatics 2007 23(21): 2947-2948).

Similarly the optimized enzyme resulting in a greater cofactor activity, preferably greater NADH activity, may be an amino acid sequence which consists of substitutions, additions or deletions to the amino acid sequence of the non-optimized enzyme, where in the substitutions, additions or deletions include 1-20 amino acids, preferably 1-10 amino acids, more preferably 1-5 amino acids, in particularly preferred 1-3 amino acids, most particularly preferred 1 amino acid. The substitutions, additions or deletions to the amino acid sequence of the non-optimized enzyme resulting in an optimized enzyme with a greater cofactor activity, preferably greater NADH activity, may be defined as resulting in an 80% amino acid sequence identity to the non-optimized enzyme, preferably 90% amino acid sequence identity, more preferably 95% amino acid sequence identity, most preferred 97% amino acid sequence identity, most highly preferred 99% amino acid sequence identity. The non-optimized enzyme may be a wildtype enzyme. SEQ ID NO. 8 is a preferred example of a wildtype enzyme enzyme, which is a non-optimized enzyme. Another example is that of SEQ ID NO. 2.

A highly preferred embodiment of the invention is that the optimized enzyme resulting in a greater cofactor activity, preferably greater NADH activity, is an amino acid sequence which consists of 1-10 mutations, preferably 1-8 mutations, more preferably 1-5 mutations, more highly preferred 1-3 mutations, most preferred 1-2 mutations, most highly preferred 1 mutation in the amino acid sequence as compared to the non-optimized enzyme. A mutation is considered as an amino acid which does not correspond to the same amino acid as compared to the non-optimized enzyme. The non-optimized enzyme may be a wildtype enzyme. SEQ ID NO. 8 is a preferred example of a wildtype enzyme enzyme, which is a non-optimized enzyme. Another example is that of SEQ ID NO. 2.

Likewise, the invention includes the nucleotide sequences encoding the optimized aldehyde dehydrogenases with the mutation of F34L as reflected in SEQ ID NO: 56 as well as the amino acid sequences for the optimized aldehyde dehydrogenases with the mutation of F34L as reflected in SEQ ID NO: 57; the nucleotide sequences encoding the optimized aldehyde dehydrogenases with the mutation of S405C as reflected in SEQ ID NO: 58 as well as the amino acid sequences for the optimized aldehyde dehydrogenases with the mutation of S405C as reflected in SEQ ID NO: 59; the nucleotide sequences encoding the optimized aldehyde dehydrogenases with the mutation of F34L and the mutation of S405C as reflected in SEQ ID NO: 60 as well as the amino acid sequences for the optimized aldehyde dehydrogenases with the mutation of F34L and the mutation of S405C as reflected in SEQ ID NO: 61; the nucleotide sequences encoding the optimized aldehyde dehydrogenases with the mutation of F34M and the mutation of S405N as reflected in SEQ ID NO: 62 as well as the amino acid sequences for the optimized aldehyde dehydrogenases with the mutation of F34M and the mutation of S405N as reflected in SEQ ID NO: 63; the nucleotide sequences encoding the optimized aldehyde dehydrogenases with the mutation of W271 S as reflected in SEQ ID NO: 64 as well as the amino acid sequences for the optimized aldehyde dehydrogenases with the mutation of W271 S as reflected in SEQ ID NO: 65; the nucleotide sequences encoding the optimized aldehyde dehydrogenases with the mutation of F34M and the mutation of Y399C and the mutation of S405N as reflected in SEQ ID NO: 9 as well as the amino acid sequences for the optimized aldehyde dehydrogenases with the mutation of F34M and the mutation of Y399C and the mutation of S405N as reflected in SEQ ID NO: 10; the nucleotide sequences encoding the optimized aldehyde dehydrogenases w with the mutation of Y399R as reflected in SEQ ID NO: 66 as well as the amino acid sequences for the optimized aldehyde dehydrogenases with the mutation of Y399R as reflected in SEQ ID NO: 67; the nucleotide sequences encoding the optimized aldehyde dehydrogenases with the mutation of F34M and the mutation of W271S and the mutation of Y399C and the mutation of S405N as reflected in SEQ ID NO: 68 as well as the amino acid sequences for the optimized aldehyde dehydrogenases with the mutation of F34M and the mutation of W271S and the mutation of Y399C and the mutation of S405N as reflected in SEQ ID NO: 69. Exemplary technical effects linked with these specific embodiments are included in table 3.

As a preferred embodiments SEQ ID NO: 2 and SEQ ID NO: 8 may be considered as representative of the wildtype enzyme for glucose dehydrogenase and aldehyde dehydrogenase, respectively. SEQ ID NO: 8 is the preferred sequence used as a comparative sequence to identify if an improved activity for a cofactor, in particular NAD/NADH, is present.

As a preferred embodiments SEQ ID NO: 2 and SEQ ID NO: 8 may be considered as representative of the non-optimized enzyme for glucose dehydrogenase and aldehyde dehydrogenase, respectively. SEQ ID NO: 8 is the preferred sequence used as a comparative sequence to identify if an improved activity for a cofactor, in particular NAD/NADH, is present.

As a preferred embodiment the protein sequence encoded by the nucleotide sequence of SEQ ID NO: 1 and SEQ ID NO: 7 may be considered as representative of the wildtype enzyme for glucose dehydrogenase and aldehyde dehydrogenase, respectively. SEQ ID NO: 7 is the preferred nucleotide sequence encoding the protein sequence used as a comparative sequence to identify if an improved activity for a cofactor, in particular NAD/NADH, is present.

As a preferred embodiment the protein sequence encoded by the nucleotide sequence of SEQ ID NO: 1 and SECT ID NO: 7 may be considered as representative of the non-optimized enzyme for glucose dehydrogenase and aldehyde dehydrogenase, respectively. SEQ ID NO: 7 is the preferred nucleotide sequence encoding the protein sequence used as a comparative sequence to identify if an improved activity for a cofactor, in particular NAD/NADH, is present.

The invention includes both the above mentioned optimized enzymes per se as well as the use of said optimized enzymes in the conversion of glucose to pyruvate in combination with the described process for the production of a target chemical. For example, one or more of the optimized enzymes is used in the process for the production of a target chemical from glucose and/or galactose, or a glucose- and or galactose-containing dimer, oligomer or polymer, by a cell-free enzyme system, comprising the conversion of glucose to pyruvate as an intermediate product; wherein no net production of ATP occurs and, preferably, wherein no phosphorylation reaction occurs; and wherein the conversion of glucose to pyruvate consists of the use of three, four or five enzymes selected from the group of dehydrogenases, dehydratases, and aldolases, wherein one or more enzymes is selected from each group. Preferably the conversion from glucose to pyruvate is achieved by three or four enzymes, most preferably by four enzymes.

### Selection of process conditions:

According to a preferred embodiment of the invention, the conversion of glucose to pyruvate as an intermediate product consists of the conversion of one mole of glucose to two moles of pyruvate.

According to a further preferred embodiment of the invention and as further described herein, the production process is performed in a **liquid system comprising two separate phases,** and the target chemical is mainly present in or forms one of the separate phases, and the target chemical is collected from the separate phase. According to a further preferred embodiment of the invention and as further described herein, an organic solvent is added to establish the two separate phases.

According to a further preferred embodiment of the invention and as further described herein, the carbon source compound is continuously fed to the process and the target chemical is continuously removed **(fed-batch process).**

According to one preferred aspect, the inventive production process comprises the following 4 steps:
Step I: Production of enzymes (the "target enzymes") for the conversion of a carbon source into a chemical (also herein referred to as the "target chemical" or "target organic compound") using microbial cells;
Step II: Release of the target enzymes from the microbial cells used in step I, preferably combined with release of cofactors and with inactivation of further, non-target enzyme activities; or purification of the target enzyme from non-target enzyme activities preferably combined with release of cofactors;
Step III: Bringing the target enzymes of step II in contact with the carbon source under conditions suitable for the conversion of the carbon source into the target chemical;
Step IV: Separating the target chemical from the reaction mixture.

### Enzyme selection and production:

In step I the target enzymes are produced using microbial cells. In one embodiment of the invention, enzyme production is done in **two or more different microbial cell lines,** such that the entire production route or major parts of it are not reconstituted in one microorganism. This avoids the unwanted initiation of substrate conversion towards the chemical and leads to a more efficient enzyme production. Enzyme production can be intracellular or extracellular, recombinant or non-recombinant. If enzyme production is recombinant it can be homologous or heterologous.

In a further embodiment of the invention, the target enzymes are **selective for one substrate and one reaction.** Preferably, the target enzymes have a substrate selectivity (kcat/kM) of at least 10 fold compared to any other naturally present substance and a reaction selectivity of at least 90%. More preferably, the target enzymes have a substrate selectivity of at least 20 fold and a reaction selectivity of at least 95%. Even more preferably, the target enzymes have a substrate selectivity of at least 100 fold and a reaction selectivity of at least 99%.

In a further embodiment of the invention, the target enzymes show **no or low inhibition by the substrate or the product** or other intermediates of the multistep reaction (no or low feedback inhibition). Preferably, the inhibition constants (Kᵢ) for any substrate, product or intermediate of the multistep reaction are at least 10 fold higher than the K_{M} value for the respective enzyme and substrate. More preferably, such inhibition constants are 100 fold higher than the respective K_{M}. In a further, particularly preferred embodiment the target enzymes still have 50% of their maximum activity at concentrations of any substrate, intermediate or product of the multistep reaction of 100 mM or more.

Preferably, the target enzymes have k_{cat} and K_{M} values that are adjusted to the multistep nature of the enzymatic route.

According to one embodiment of the process of the invention, the **target enzymes tolerate elevated levels of the target chemical** and, optionally, other organic solvents that are optionally added to support segregation of the target chemical into a separate phase.

Preferably the target enzymes tolerate concentrations of the target chemical of more than 2 % (w/w), more preferably more than 4 % (w/w), more preferably more than 6 % (w/w), even more preferably more preferably more than 8 % (w/w). In a particularly preferred embodiment, the target enzymes tolerate concentrations of the target chemicals up to the maximum solubility in water.

In a preferred embodiment, the entire cell-free enzyme system tolerates elevated levels of the target chemical, such as isobutanol, butanol, or ethanol. In a particularly preferred embodiment, the entire cell-free enzyme system tolerates isobutanol in concentrations of 2 % (w/w) or more, preferably 4 %(w/w) or more, more preferably 6 % (w/w) or more, even more preferably 8 % (w/w) or more. In a particularly preferred embodiment, the entire cell-free enzyme system tolerates concentrations of the target chemicals such as isobutanol at concentrations at which phase separation occurs, i.e. at concentrations corresponding to the maximum solubility of the target chemical in water at the process temperature.

In a preferred embodiment of the invention, the target enzymes tolerate elevated levels of **chaotropic substances** and **elevated temperatures.** Preferably, the target enzymes tolerate concentrations of guanidinium chloride of more than 1 M, more preferably more than 3 M, and most preferably more than 6 M. Alternatively or in combination, the target enzymes tolerate preferably temperatures of more than 40 - 90 °C, more preferably more than 50-80 °C, and most preferably more than 50-60°C. In such preferred embodiment, target enzyme production is done in a host organism whose endogenous enzyme activities are mostly inactivated at elevated levels of chaotropic substances and/or at elevated temperatures.

Preferably target enzyme production is performed using one or more of the following microbial species: Escherichia coli; Pseudomonas fluorescence; Bacillus subtilis; Saccharomyces cerevisiae; Pichia pastoris; Hansenula polymorpha; Klyuveromyces lactis; Trichoderma reesei; Aspergillus niger. More preferably target enzyme production is performed using **Escherichia coli** as host organism. Preferably, enzyme production and cell growth are separated into separate growth phases. Thereby, no substrate is used for general metabolic activity.

In a preferred aspect of the invention, E. coli, B. subtilis, S. cerevisae and/or Pichia pastoris are used as the expression hosts and one or more enzymes are recombinantly produced in each individual strain. In another preferred aspect, E. coli is the expression host and one enzyme is expressed per individual strain. Preferably, such enzymes have a half-life of more than 12 hours under temperatures and/or pH values that inactivate, when incubated for at least 10 min, preferably at least 30 min, the expression host (i.e. cell growth) and inactivate at least 90%, preferably 95%, more preferably 99% of the enzyme activities that orginate from the expression host and accept any of the intermediates from the carbohydrate source (e.g. glucose or galactose) to the target chemical as substrate. In a particularly preferred aspect, no such enzyme activities can be detected after incubation for 30 min at such temperature. Most preferably the expressed enzymes have a half-life of at least 12 hours when incubated at 50°C in standard buffer at neutral pH.

In a further preferred embodiment of the invention, the target enzymes tolerate elevated levels of **oxygen.** Preferably, the target enzymes tolerate oxygen concentrations of more than 1 ppm, more preferably more than 7 ppm. Most preferably the target enzymes are active and stable under aerobic conditions. Preferably, the multistep reaction does not require oxygen and is not inhibited by oxygen. Thereby, no special precautions for oxygen exclusion have to be taken, making the process more stable with less effort on the production environment and/or equipment.

In step II the target enzymes are released from the cells. In a preferred embodiment, the target enzymes tolerate high temperatures and chaotropic conditions, whereas the background enzymes from the producing microorganism do not tolerate these conditions. According to this embodiment, the target enzymes are produced **intracellularly** in microbial cells, the cells are lysed using high temperature and/or chaotropic conditions, thereby releasing the target enzymes in active form, optionally together with cofactors, while unwanted background enzyme activities are inactivated.

In another preferred embodiment, enzyme production is **extracellular,** the target enzymes tolerate high temperatures and chaotropic conditions, and background enzyme activities (non-target enzymes) do not tolerate these conditions. According to this embodiment, the supernatant from extracellular production is treated under conditions such as high temperatures and/or chaotropic conditions. This leads to inactivation of unwanted background enzyme activities (non-target enzymes) while the target enyzmes remain active.

In one embodiment of the invention, cofactors are required for one or more of the multiple enzymatic conversion steps. In one aspect of the invention such cofactors are added to the enzyme mixture. In another, particularly preferred aspect of this embodiment such **cofactors are also produced by the microbial cells** intracellularly and are released by the same treatment as to release the target enzymes. In another preferred embodiment of the invention, the microbial cells are engineered in order to optimize the level of cofactors produced. Preferably, the microbial cells are inactivated during step II. Thereby, cell growth and enzyme activity are separated in the process, and no carbon source is consumed by undesirable cell growth.

### Process set-up for the enzymatic conversion:

In step III the carbon source is converted by a mixture of enzymes in a multistep enzymatic reaction to pyruvate, and, optionally, further to the target chemical. According to the inventive process, the enzymes are active under the denaturing activity of the target chemical. Preferably, the microbial cells used in step I are inactive and/or are inactivated under the reaction conditions of step III.

Preferably, the **concentration of each enzyme** in the target enzyme mixture is adjusted to the optimal level under process conditions. In a particularly preferred embodiment one or more enzyme concentrations are increased above typical intracellular concentrations in order to improve the yield of the process (no limit by the maximal density of the microorganisms as in classical processes). In another particularly preferred embodiment, one or more enzymes are engineered for maximal catalytic efficiency (leading to lower reactor size and running costs compared to classical processes).

The activity of the enzymes in the target enzyme mixture is adjusted to the optimal level under process conditions. In a preferred embodiment the activity of the first enzyme of the enzymatic cascade is adjusted to a lower level compared to the activities of the enzymes later in the cascade. Such enzyme activities prevent accumulation of intermediates in the reaction. In case an enzyme is used in more than one step of the cascade, the activity may be increased accordingly. In a particularly preferred embodiment the activity of each enzyme of the enzymatic cascade is adjusted to a level which is greater than any preceeding enzyme activity in the enzymatic cascade. Such enzyme activities prevent accumulation of intermediates in the reaction.

In a further preferred embodiment, the **target chemical is added to or present in the reaction mixture** in step III at a concentration **at or slightly above the maximum level** that can be mixed in a single phase with water under process conditions. According to this embodiment, the target chemical continuously segregates into the second phase during the process. In a particular variant of this embodiment, a water soluble substance is added to the reaction mixture that leads to a phase separation of the target chemical at lower concentration than without the added substance. Examples of such substances are salts and are known to the person skilled in the art. In a particularly preferred variant of this embodiment, sodium chloride is added to lower the solubility of the target chemical in the water phase.

In another preferred embodiment, an additional organic solvent is added to the process that forms its own phase and extracts the produced hydrophobic chemical from the water phase. Preferred examples of such additional solvents comprise: n-hexane, cyclohexane, octanol, ethylacetate, methylbutyl ketone, or combinations thereof.

In another preferred embodiment, the yield is improved because the formation of side products is decreased by using target enzymes that are specific for the desired reactions. In another preferred embodiment, host enzymes that would catalyse side reactions are inactivated during step II and/or are inactive under the reaction conditions of step III.

ln yet another preferred embodiment of the invention, contamination of the process by microorganisms is avoided by adjusting reaction conditions in step III that are toxic for typical microbial contaminants. Such conditions comprise elevated temperature, extreme pH, addition of organic chemicals. In a particularly preferred embodiment of the invention, the target chemical itself is toxic at the concentration achieved in the process (more stable process with less effort on production environmaent and/or equipment).

According to another preferred embodiment to the invention, **no additional redox cofactors are added** to the reaction mixture except for those cofactors that are produced by the microorganisms used in step I and that are included in the cell lysate produced in step II. Examples of such cofactors are FAD/FADH2, FMN/FMNH2, NAD/NADH, NADP/NADPH. According to this embodiment, the cofactors that are required are produced by the microbial cells in step I and are regenerated during the process (NADH to NAD and vice versa; NADPH to NADP and vice versa). In one embodiment of the invention, excess reduction equivalents (NADH, NADPH) or energy equivalents (ATP) are regenerated by additional enzymes (e.g. NADH oxidase for NADH; NADPH oxidase for NADPH).

In a particularly preferred embodiment of the invention, neither ATP nor ADP is involved as a cofactor in the conversion from glucose to pyruvate and none of the target enzymes involved in this conversion comprises a phosphorylation step (non-phosphorylative pyruvate production).

In step IV, the one or more target chemicals are separated from the reaction mixture. In a preferred embodiment of the invention the one or more target chemicals are hydrophobic and form a separate phase which preferably contains at least a substantial fraction of the produced chemicals. In a particularly preferred embodiment the one or more target chemicals are continuously removed from the reaction mixture.

In a further preferred embodiment of the invention, the carbon source is continuously fed to the reaction mixture to be converted into the target chemical. Likewise, the target chemical is preferably continuously removed as a separate phase and further purified by methods known in the art. Thereby, product isolation is simplified as the product is collected in a separate phase from which it can be purified further. Thereby, the yield is improved and product purification is simplified.

In a further preferred embodiment, the inventive process does not require ADP or ATP as cofactors. Other processes (Welch and Scopes, 1985; Algar and Scopes, 1985) require cofactors such as ADP/ATP and NAD/NADH. The postulated conversion of glucose to Butanol (Zhang et al, 2008) requires the cofactors ADP/ATP, NAD/NADH, Ferredoxin and Coenzyme A. A major problem of the described cell free enzymatic processes (Zhang et al., 2008, Welch and Scopes 1985) is the accumulation of ATP. In the known processes, the undesired accumulation of ATP is circumvented by the addition of an ATPase. To find the right concentration of ATPase, however, is difficult as it depends on the concentration of the substrate and different intermediates as well as on the activity of the enzymes. With either too much ATPase or too little ATPase, an ATP imbalance results and the conversion completely ceases (Welch and Scopes, 1985). As an alternative to ATPase, arsenate may also be used, with similar disadvantages as for ATPase. In contrast, according to a particularly preferred aspect, the inventive cell-free process converts a carbon source such as glucose to pyruvate, and more preferably glucose to the target chemical, without net production of ATP and without using an ATPase and/or arsenate.

### Production of pyruvate

Several preferred embodiments are hereinafter described regarding the production of pyruvate from glucose. Two molecules pyruvate are produced from one molecule glucose. The pyruvate can subsequently be converted to target chemicals such as n-butanol, isobutanol, ethanol and 2-butanol.

According to one aspect of the invention glucose is converted to pyruvate by the use of the following enzymes:

### Enzyme combination P-1:

| **#** | **Enzyme** | **EC #** | **Substrate** | **Product** |
|---|---|---|---|---|
| 1 | Glucose dehydrogenase | 1.1.1.47 | Glucose | Gluconate |
| 2 | Gluconate dehydratase | 4.2.1.39 | Gluconate | 2-keto-3-deoxy gluconate |
| 3 | 2-keto-3-deoxy gluconate aldolase | 4.1.2.14 | 2-keto-3-deoxy gluconate | Pyruvate, Glyceraldehyde |
| 4 | Aldehyde dehydrogenase | 1.2.1.3 | Glyceraldehyde | Glycerate |
| 5 | Glycerate 2-kinase | 2.7.1.165_ | Glycerate | Glycerate-2-Phosphate |
| 6 | Enolase | 4.2.1.11 | Glycerate-2-Phosphate | Phosphoenolpyruvate |
| 7 | Pyruvate Kinase | 2.7.1.40 | Phosphoenolpyruvate | Pyruvate |

According to another preferred aspect of the invention glucose is converted to pyruvate by the use of the following enzymes:

### Enzyme combination P-2-a:

| **#** | **Enzyme** | **EC #** | **Substrate** | **Product** |
|---|---|---|---|---|
| 1 | Glucose dehydrogenase | 1.1.1.47 | Glucose | Gluconate |
| 2 | Gluconate dehydratase | 4.2.1.39 | Gluconate | 2-keto-3-deoxy gluconate |
| 3 | 2-keto-3-deoxy gluconate aldolase | 4.1.2.14 | 2-keto-3-deoxy gluconate | Pyruvate, Glyceraldehyde |
| 4 | Aldehyde dehydrogenase | 1.2.1.3 | Glyceraldehyde | Glycerate |
| 5 | Glycerate dehydrogenase | 1.1.1.29/ 1.1.1.81 | Glycerate | Hydroxypyruvate |
| 6 | Serine-pyruvate transaminase | 2.6.1.51 | Hydroxypyruvate + Alanine | Serine + Pyruvate |
| 7 | L-Serine ammonia-lyase | 4.3.1.17 | Serine | Pyruvate + Ammonia |
| 8 | Alanine dehydrogenase | 1.4.1.1 | Pyruvate + Ammonia | Alanine |

According to another preferred aspect of the invention glucose is converted to pyruvate by the use of the following enzymes:

### Enzyme combination P-2-b:

| **#** | **Enzyme** | **EC #** | **Substrate** | **Product** |
|---|---|---|---|---|
| 1 | Glucose dehydrogenase | 1.1.1.47 | Glucose | Gluconate |
| 2 | Gluconate dehydratase | 4.2.1.39 | Gluconate | 2-keto-3-deoxy gluconate |
| 3 | 2-keto-3-deoxy gluconate aldolase | 4.1.2.14 | 2-keto-3-deoxy gluconate | Pyruvate, Glyceraldehyde |
| 4 | Aldehyde dehydrogenase | 1.2.1.3 | Glyceraldehyde | Glycerate |
| 5 | Glycerate dehydrogenase | 1.1.1.29/ 1.1.1.81 | Glycerate | Hydroxypyruvate |
| 6 | Serine-pyruvate transam inase | 2.6.1.51 | Hydroxypyruvate + Glycine | Serine + Glyoxylate |
| 7 | L-Serine ammonia-lyase | 4.3.1.17 | Serine | Pyruvate + Ammonia |
| 8 | Glycine dehydrogenase | 1.4.1.1 | Glyoxylate + Ammonia | Glycine |

According to another preferred aspect of the invention glucose is converted to pyruvate by the use of the following enzymes:

### Enzyme combination P-2-c:

| **#** | **Enzyme** | **EC#** | **Substrate** | **Product** |
|---|---|---|---|---|
| 1 | Glucose dehydrogenase | 1.1.1.47 | Glucose | Gluconolacton |
| 2 | Gluconate dehydratase | 4.2.1.39 | Gluconate | 2-keto-3-deoxy gluconate |
| 3 | 2-keto-3-deoxy gluconate aldolase | 4.1.2.14 | 2-keto-3-deoxy gluconate | Pyruvate, Glyceraldehyde |
| 4 | Aldehyde dehydrogenase | 1.2.1.3 | Glyceraldehyde | Glycerate |
| 5 | Glycerate dehydrogenase | 1.1.1.29/ 1.1.1.81 | Glycerate | Hydroxypyruvate |
| 6 | Serine-pyruvate transaminase | 2.6.1.51 | Hydroxypyruvate + L-Glutamate | Serine + 2-Ketoglutarate |
| 7 | L-Serine ammonia-lyase | 4.3.1.17 | Serine | Pyruvate + Ammonia |
| 8 | L-Glutamate | 1.4.1.1 | 2-Ketoglutarate + Ammonia | L-Glutamate |

According to another preferred aspect of the invention glucose is converted to pyruvate by the use of the following enzymes:

### Enzyme combination P-2-d:

| **#** | **Enzyme** | **EC #** | **Substrate** | **Product** |
|---|---|---|---|---|
| 1 | Glucose dehydrogenase | 1.1.1.47 | Glucose | Gluconate |
| 2 | Gluconate dehydratase | 4.2.1.39 | Gluconate | 2-keto-3-deoxy gluconate |
| 3 | 2-keto-3-deoxy gluconate aldolase | 4.1.2.14 | 2-keto-3-deoxy gluconate | Pyruvate, Glyceraldehyde |
| 4 | Aldehyde dehydrogenase | 1.2.1.3 | Glyceraldehyde | Glycerate |
| 5 | Glycerate dehydrogenase | 1.1.1.29/ 1.1.1.81 | Glycerate | Hydroxypyruvate |
| 6 | Serine-pyruvate transaminase | 2.6.1.51 | Hydroxypyruvate + L-Phenylalanine | Serine + Phenylpyruvate |
| 7 | L-Serine ammonia-lyase | 4.3.1.17 | Serine | Pyruvate + Ammonia |
| 8 | L-Phenylalanine dehydrogenase | 1.4.1.20 | Phenylpyruvate + Ammonia | L-Phenylalanine |

According to another preferred aspect of the invention glucose is converted to pyruvate by the use of the following enzymes:

### Enzyme combination P-3-a:

| **#** | **Enzyme** | **EC #** | **Substrate** | **Product** |
|---|---|---|---|---|
| 1 | Glucose dehydrogenase | 1.1.1.47 | Glucose | Gluconate |
| 2 | Gluconate dehydratase | 4.2.1.39 | Gluconate | 2-keto-3-deoxy gluconate |
| 3 | 2-keto-3-deoxy gluconate aldolase | 4.1.2.14 | 2-keto-3-deoxy gluconate | Pyruvate, Glyceraldehyde |
| 4 | Aldehyde dehydrogenase | 1.2.1.3 | Glyceraldehyde | Glycerate |
| 5 | Dihydroxyacid dehydratase | 4.2.1.9 | Glycerate | Pyruvate |

According to another preferred aspect of the invention glucose is converted to pyruvate by the use of the following enzymes:

### Enzyme combination P-3-b:

| **#** | **Enzyme** | **EC #** | **Substrate** | **Product** |
|---|---|---|---|---|
| 1 | Glucose dehydrogenase | 1.1.1.47 | Glucose | Gluconate |
| 2 | Dihydroxyacid dehydratase | 4.2.1.9 or 4.2.1.39 | Gluconate | 2-keto-3-deoxy gluconate |
| 3 | 2-keto-3-deoxy gluconate aldolase | 4.1.2.14 | 2-keto-3-deoxy gluconate | Pyruvate, Glyceraldehyde |
| 4 | Aldehyde dehydrogenase | 1.2.1.3 | Glyceraldehyde | Glycerate |
| - | (Enzyme #2:) | | Glycerate | Pyruvate |

According to another preferred aspect of the invention glucose is converted to pyruvate by the use of the following enzymes:

### Enzyme combination P-3-c:

| **#** | **Enzyme** | **EC#** | **Substrate** | **Product** |
|---|---|---|---|---|
| 1 | Glucose/aldehyde dehydrogenase | 1.1.1.47 or 1.2.1.3 | Glucose | Gluconate |
| 2 | Dihydroxyacid dehydratase | 4.2.1.9 or 4.2.1.39 | Gluconate | 2-keto-3-deoxy gluconate |
| 3 | 2-keto-3-deoxy gluconate aldolase | 4.1.2.14 | 2-keto-3-deoxy gluconate | Pyruvate, Glyceraldehyde |
| - | (Enzyme #1:) | | Glyceraldehyde | Glycerate |
| - | (Enzyme #2:) | | Glycerate | Pyruvate |

In all enzyme combinations P-x (i.e. P-1, P-2-a, P-2-b, P-2-c, P-2-d, P-3-a, P-3-b, and P-3-c) one mol glucose is converted into two moles pyruvate, coupled with the reduction of two NAD equivalents. To eliminate phosphorylation and dephosphorylation steps of natural pathways and thus reduce the number of required enzymes, the invention exploits, for example, the substrate promiscuity of an archaeal dihydroxy acid dehydratase (DHAD) which catalyzes both, the transformation of glycerate to pyruvate and of gluconate to 2-keto-3-deoxygluconate. The molecular efficiency of DHAD allows for the consolidated conversion of glucose to pyruvate with just 4 enzymes, comprising glucose dehydrogenase (GDH) (J. Biol. Chem. 2006, 281, 14796-14804), gluconate/ glycerate/ dihydroxyacid dehydratase (DHAD) (J. Biochem. 2006, 139, 591-596), 2-keto-3-deoxygluconate aldolase (KDGA) (Biochem. J. 2007, 403, 421-430) and glyceraldehyde dehydrogenase (ALDH) (Biochem J. 2006, 397, 131-138). ALDH together with DHAD redirects glyceraldehyde produced via aldol cleavage towards pyruvate formation. Enzymes of the cell-free reaction cascade are chosen based on their stability and selectivity.

A preferred embodiment of the invention is the use of optimized enzymes for improved NADH activity.

Preferably, enzymes for the conversion of glucose to pyruvate are selected from the following **list of enzymes** (Information: Enzyme name, E.C. number in brackets, Source organism, NCBI / Gene Number if applicable, Mutations if applicable, Seq ID if applicable):
- Glucose dehydrogenase GDH (EC 1.1.1.47), Sulfolobus solfataricus, NP 344316.1, **Seq ID 02**
- Dihydroxy acid dehydratase DHAD (EC 4.2.1.9), Sulfolobus solfataricus, NP 344419.1, **Seq ID 04**
- Gluconate dehydratase (EC 4.2.1.39), Sulfolobus solfataricus, NP_344505
- Gluconate dehydratase (EC 4.2.1.39), Sulfolobus solfataricus, NP_344505, Mutation I9L
- Gluconate dehydratase ilvEDD (EC 4.2.1.39), Achromobacter xylsoxidans
- Gluconate dehydratase ilvEDD (EC 4.2.1.39), Metallosphaera sedula DSM 5348
- Gluconate dehydratase ilvEDD (EC 4.2.1.39), Thermoplasma acidophilum DSM 1728
- Gluconate dehydratase ilvEDD (EC 4.2.1.39), Thermoplasma acidophilum DSM 1728
- 2-Keto-3-deoxygluconate aldolase KDGA (EC 4.1.2.14), Sulfolobus solfataricus, NP 344504.1
- 2-Keto-3-deoxygluconate aldolase KDGA (EC 4.1.2.14), Sulfolobus acidocaldaricus, **Seq ID 06**
- Aldehyde Dehydrogenase ALDH (EC 1.2.1.3), Flavobacterium frigidimaris, BAB96577.1
- Aldehyde Dehydrogenase ALDH (EC 1.2.1.3), Thermoplasma acidophilum, **Seq ID 08**
- Aldehyde Dehydrogenase ALDH (EC 1.2.1.3), Thermoplasma acidophilum, Mutations F34M + Y399C + S405N, **Seq ID 10**
- Glycerate kinase (EC 2.7.1.), Sulfolobus solfataricus, NP_342180.1
- Glycerate 2-kinase (EC 2.7.1.165), Sulfolobus tokodaii, Uniprot Q96YZ3.1
- Enolase (EC 4.2.1.11), Sulfolobus solfataricus, NP 342405.1
- Pyruvate Kinase (EC 2.7.1.40), Sulfolobus solfataricus, NP 342465.1
- Glycerate dehydrogenase/hydroxypyruvate reductase (EC 1.1.1.29/1.1.1.81), Picrophilus torridus, YP_023894.1
- Serine-pyruvate transaminase (EC 2.6.1.51), Sulfolobus solfataricus, NCBI Gen ID: NP_343929.1
- L-serine ammonia-lyase (EC 4.3.1.17), EC 4.3.1.17, Thermus thermophilus, YP_144295.1 and YP_144005.1
- Alanine dehydrogenase (EC 1.4.1.1), Thermus thermophilus, NCBI-Gen ID: YP_005739.1

In a preferred embodiment of the invention, enzymes for the **conversion of glucose to pyruvate** are selected from the following **list of enzymes:**
- Glucose dehydrogenase GDH (EC 1.1.1.47), Sulfolobus solfataricus, NP 344316.1
- Dihydroxy acid dehydratase DHAD (EC 4.2.1.9), Sulfolobus solfataricus, NP 344419.1
- Gluconate dehydratase (EC 4.2.1.39), Sulfolobus solfataricus, NP_344505, Mutation I9L
- KDGA (EC 4.1.2.14), Sulfolobus acidocaidaricus
- ALDH (EC 1.2.1.3), Thermoplasma acidophilum

The enzyme combinations listed above for the conversion of glucose to pyruvate can also be employed for the conversion of galactose or a mixture of glucose and galactose to pyruvate. Thereby, galactose is converted via galactonate, and 2-keto-3-deoxy-galactanate, to pyruvate and glycerate. The glycerate is then converted as described above.

The conversion of galactose to galactonate is preferably done by a dehydrogenase accepting galactose, more preferably by a dehydrogenase accepting both, glucose and galactose. The conversion of galactonate to 2-keto-3-deoxy-galactanate is preferably done by a dehydratase accepting galactonate, more preferably by a dehydratase accepting both, gluconate and galactonate. The conversion of 2-keto-3-deoxy-galactanate is preferably done by an aldolase accepting 2-keto-3-deoxy-galactanate, more preferably by an aldolase accepting both, 2-keto-3-deoxy-gluconate and 2-keto-3-deoxy-galactanate.

In a particularly preferred aspect such enzymes are selected from the following enzymes:
- Glucose dehydrogenase GdhA, Picrophilus torridus (Liebl, W. et al. 2005 FEBS J. 272(4):1054)
- Dihydroxy acid dehydratase DHAD, Sulfolobus solfatoricus (Kim, S. J. Biochem. 2006 139(3):591)
- KDGal aldolase, E. coli (Uniprot P75682)

### Production of n-butanol:

In a particularly preferred embodiment, n-butanol is produced from pyruvate.

Various options exist for the **conversion of pyruvate to acetyl CoA.** In one embodiment of the invention, one or more of the following enzymes is used for the conversion: (i) pyruvate oxidoreductase using ferredoxin as cofactor; (ii) pyruvate dehydrogenase using NAD(P)H as cofactor; (iii) pyruvate formate lyase; (iv) pyruvate dehydrogenase enzyme complex.

In a peferred embodiment, pyruvate dehydrogenase is used as the enzyme for this conversion, using NADH as cofactor. Pyruvate dehydrogenases are usually part of a multi enzyme complex (Pyruvate dehydrogenase complex, PDHC) which consists of three enzymatic activities and has a molecular weight of ca. 1 Mio Da. For application in a cell-free reaction system it is beneficial to have small and robust non-complexed enzymes. It has been found that the pyruvate dehydrogenase from *Euglena gracilis* can be used therefore. This enzyme is singular and complex-free. Furthermore it uses NADH as cofactor.

Alternatively, pyruvate formate lyase can be combined with a formate dehydrogenase using NADH as cofactor.

Various options exist for the **conversion of acetyl CoA to n-butanol,** employing enzymes from n-butanol producing bacteria, such as *C. acetobutylicum, C. saccharobutylicum, C. saccharoperbutylacetonicum, C. beijerinckii.*

According to a preferred aspect of the invention pyruvate is converted to n-butanol by the use of the following enzymes:

### Enzyme combination N-1:

| **#** | **Enzyme** | **EC #** | **Substrate** | **Product** |
|---|---|---|---|---|
| 1 | Thiolase | 2.3.1.16 | **AcetylCoA** | AcetoacetylCoA |
| 2 | β-HydroxybutyrylCoA dehydrogenase | 1.1.1.157 | AcetoacetylCoA | β-HydroxybutyrylCoA |
| 3 | Crotonase | 4.2.1.55 | β-HydroxybutyrylCoA | CrotonylCoA |
| 4 | ButyrylCoA Dehydrogenase | 1.3.99.2 | CrotonylCoA | **ButyrylCoA** |
| 5 | CoA acylating Butanal Dehydrogenase | 1.2.1.57 | **Butyrat** | Butanal |
| 6 | Butanol Dehydrogenase | 1.1.1.- | Butanal | **Butanol** |

When any of the enzyme combinations for the production of pyruvate (P-1, P-2-a, P-2-b, P-2-c, P-2-d, P-3-a, P-3-b, and P-3-c) is combined with any of the enzyme combinations for the production of n-butanol (N-1) a net conversion of one molecule glucose to two molecules of CO₂, one molecule of water and one molecule of n-butanol is achieved.

Preferably, enzymes for the **conversion of pyruvate to n-butanol** are selected from the following **list of enzymes** (Information: Enzyme name, E.C. number in brackets, Source organism, NCBI / Gene Number if applicable, Mutations if applicable, Seq ID if applicable):
- Thiolase (EC 2.3.1.16), Clostridium acetobutylicum, NCBI-GenID NP_349476.1
- 3-hydroxybutyryl-CoA dehydrogenase (EC 1.1.1.157), NP_349314.1
- Crotonase (EC 4.2.1.55), Clostridium acetobutylicum, NP_349318.1
- Butyryl-CoA dehydrogenase (EC 1.3.99.2), Clostridium acetobutylicum, NCBI-GenID NP_349317.1,
- Coenzyme A acylating aldehyde dehydrogenase (EC 1.2.1.57), Clostridium beijerinckii, NCBI-GenID AF132754_1)
- NADH-dependent butanol dehydrogenase B (BDH II) (EC 1.1.1.-), Clostridium acetobutylicum, NCBI-GenID NP_349891.1
- electron transfer flavoproteins (etfA and/or B), Clostridium acetobutylicum, NCBI-GenID NP_349315.1 and NP_349316.1

### Production of isobutanol

In another particularly preferred embodiment, isobutanol is produced from pyruvate. Various options exist for the **conversion of pyruvate to isobutanol.**

According to a preferred aspect of the invention pyruvate is converted to isobutanol by the use of the following enzymes:

Enzyme combination I-1:

| **#** | **Enzyme** | **EC #** | **Substrate** | **Product** |
|---|---|---|---|---|
| 1 | acetolactate synthase (ALS) | 2.2.1.6 | Pyruvate | Acetolactate |
| 2 | ketol-acid reductoisomerase (KARI) | 1.1.1.86 | Acetolactate | 2,3 dihydroxy isovalerate |
| 3 | Dihydroxyacid dehydratase (DHAD) | 4.2.1.9 | 2,3 dihydroxy isovalerate | 2-keto-isovalerate |
| 4 | Branched-chain-2-oxo acid decarboxylase (KDC) | 4.1.1.72 | a-keto-isovalerate | isobutanal |
| 5 | alcohol dehydrogenase (ADH) | 1.1.1.1 | Isobutanal | isobutanol |

When any of the enzyme combinations for the production of pyruvate (P-1, P-2-a, P-2-b, P-2-c, P-2-d, P-3-a, P-3-b, and P-3-c) is combined with any of the enzyme combinations for the production of isobutanol (I-1) a net conversion of one molecule glucose to two molecules of CO₂, one molecule of water and one molecule of isobutanol is achieved.

Preferably, enzymes for the **conversion of pyruvate to isobutanol** are selected from the following **list of enzymes** (Information: Enzyme name, E.C. number in brackets, Source organism, NCBI / Gene Number if applicable, Mutations if applicable, Seq ID if applicable):
- Acetolactate synthase ALS (EC 2.2.1.6), Bacillus subtilis, **Seq ID 12**
- Acetolactate synthase ALS (EC 2.2.1.6), Sulfolobus solfataricus, NCBI-GenID: NP_342102.1
- Acetolactate synthetase ALS (EC: 2.2.1.6), Thermotoga maritima, NCBI-GeneID: NP_228358.1
- Ketol-acid reductoisomerase KARI (EC 1.1.1.86), Meiothermus ruber, **Seq ID 14**
- Ketol-acid reductoisomerase KARI (EC 1.1.1.86), Sulfolobus solfataricus, NCBI-GenID: NP_342100.1
- Ketol-acid reductoisomerase KARI (EC. 1.1.1.86), Thermotoga maritima, NCBI-GeneID: NP_228360.1
- Branched-chain-2-oxo acid decarboxylase KDC (EC 4.1.1.72), Lactococcus lactis, **Seq ID 16**
- α-Ketoisovalerate decarboxylase KDC, (EC 4.1.1.-), Lactococcus lactis, NCBI-GeneID: CAG34226.1
- Dihydroxy acid dehydratase DHAD (EC 4.2.1.9), Sulfolobus solfataricus, NP 344419.1, **Seq ID 04**
- Dihydroxy-acid dehydratase DHAD, (EC: 4.2.1.9), Thermotoga maritima, NCBI-GeneID: NP_228361.1
- Alcohol dehydrogenase ADH (EC 1.1.1.1), Geobacillus stearothermophilus, **Seq ID 18**
- Alcohol dehydrogenase ADH (EC 1.1.1.1), Flavobacterium frigidimaris, NCBI-GenID: BAB91411.1
- Alcohol dehydrogenase ADH (EC: 1.1.1.1), S. cerevisiae

In a preferred embodiment of the invention, enzymes for the **conversion of pyruvate to isobutanol** are selected from the following **list of enzymes:**
- Acetolactate synthase ALS (EC 2.2.1.6), Bacillus subtilis, **Seq ID 12**
- Ketol-acid reductoisomerase KARI (EC 1.1.1.86), Meiothermus ruber, **Seq ID 14**
- Branched-chain-2-oxo acid decarboxylase KDC (EC 4.1.1.72), Lactococcus lactis, **Seq ID 16**
- Dihydroxy acid dehydratase DHAD (EC 4.2.1.9), Sulfolobus solfataricus, NP 344419.1, **Seq ID 04**
- Alcohol dehydrogenase ADH (EC 1.1.1.1), Geobacillus stearothermophilus, **Seq ID 18**

In a further particularly preferred embodiment of the invention a single dehydratase can be employed for the conversion of gluconate to 2-keto-3-deoxygluconate and glycerate to pyruvate and 2,3-dihydroxyisovalerate to 2-keto-isovalerate. Therefore a single enzyme can be employed for enzyme activity #2 in enzyme combination P-1, P-2-a, P-2-b, P-2-c, P-2-d, P-3-a, P-3-b, and P-3-c and for enzyme activity #3 in enzyme combination I-1.

### Production of ethanol

In another embodiment of the invention, the target chemical is ethanol. Various options exist for the **conversion of pyruvate to ethanol.**

According to a preferred aspect of the invention pyruvate is converted to ethanol by the use of the following enzymes:

### Enzyme combination E-1:

| **#** | **Enzyme** | **EC #** | **Substrate** | **Product** |
|---|---|---|---|---|
| 1 | Pyruvate decarboxylase | 4.1.1.1 | Pyruvate | Acetaldehyde |
| 2 | Alcohol dehydrogenase | 1.1.1.1 | Acetaldehyde | Ethanol |

When any of the enzyme combinations for the production of pyruvate (P-1, P-2-a, P-2-b, P-2-c, P-2-d, P-3-a, P-3-b, and P-3-c) is combined with any of the enzyme combinations for the production of ethanol (E-1) a net conversion of one molecule glucose to two molecules of CO₂ and two molecules of ethanol is achieved.

A preferred embodiment of the invention is the use of optimized enzymes for improved NADH activity.

Preferably, enzymes for the **conversion of pyruvate to ethanol** are selected from the following **list of enzymes** (Information: Enzyme name, E.C. number in brackets, Source organism, NCBI / Gene Number if applicable, Mutations if applicable, Seq ID if applicable):
- Pyruvate decarboxylase PDC (EC 4.1.1.1), Zymomonas mobilis, **Seq ID 20**
- Alcohol dehydrogenase ADH (EC 1.1.1.1), Geobacillus stearothermophilus, **Seq ID 18**

### Production of 2-butanol

In another embodiment of the invention the target chemical is 2-butanol. Various options exist for the **conversion of pyruvate to 2-butanol.**

According to a preferred aspect of the invention pyruvate is converted to 2-butanol by the use of the following enzymes:

### Enzyme combination T-1:

| **#** | **Enzyme** | **EC#** | **Substrate** | **Product** |
|---|---|---|---|---|
| 1 | Acetolactate synthase | 2.2.1.6 | Pyruvate | Acetolactate |
| 2 | Acetolactate decarboxylase | 4.1.1.5 | Acetolactate | Acetoin |
| 3 | Alcohol (Butanediol) dehydrogenase | 1.1.1.4 | Acetoin | Butane-2,3-diol |
| 4 | Diol dehydratase | 4.2.1.28 | Butane-2,3-diol | 2-butanon |
| 5 | Alcohol dehydrogenase | 1.1.1.1 | 2-butanon | 2-butanol |

In a further preferred embodiment an alcohole dehydrogenase is used that uses acetoin as well as 2-butanon as substrate. Therefore, pyruvate is converted to 2-butanol by the use of the following enzymes:

### Enzyme combination T-2:

| **#** | **Enzyme** | **EC #** | **Substrate** | **Product** |
|---|---|---|---|---|
| 1 | Acetolactate synthase | 2.2.1.6 | Pyruvate | Acetolactate |
| 2 | Acetolactate decarboxylase | 4.1.1.5 | Acetolactate | Acetoin |
| 3 | Alcohol dehydrogenase (ADH) | 1.1.1.4 or 1.1.1.1 | Acetoin | Butane-2,3-diol |
| 4 | Diol dehydratase | 4.2.1.28 | Butane-2,3-diol | 2-butanon |
| - | (enzyme 3:) | | 2-butanon | 2-butanol |

### Enzyme combination T-2a:

| **#** | **Enzyme** | **EC #** | **Substrate** | **Product** |
|---|---|---|---|---|
| 1 | Acetolactate synthase | 2.2.1.6 | Pyruvate | Acetolactate |
| 2 | Alcohol dehydrogenase (ADH) | 1.1.1.4 or 1.1.1.1 | Acetoin | Butane-2,3-diol |
| 3 | Diol dehydratase | 4.2.1.28 | Butane-2,3-diol | 2-butanon |
| - | (enzyme 2:) | | 2-butanon | 2-butanol |

When any of the enzyme combinations for the production of pyruvate (P-1, P-2-a, P-2-b, P-2-c, P-2-d, P-3-a, P-3-b, and P-3-c) is combined with any of the enzyme combinations for the production of 2-butanol (T-1, T-2, T-2a) a net conversion of one molecule glucose to two molecules CO₂, one molecule water and one molecule 2-butanol is achieved.

A preferred embodiment of the invention is the use of optimized enzymes for improved NADH activity..

### Description of figures:

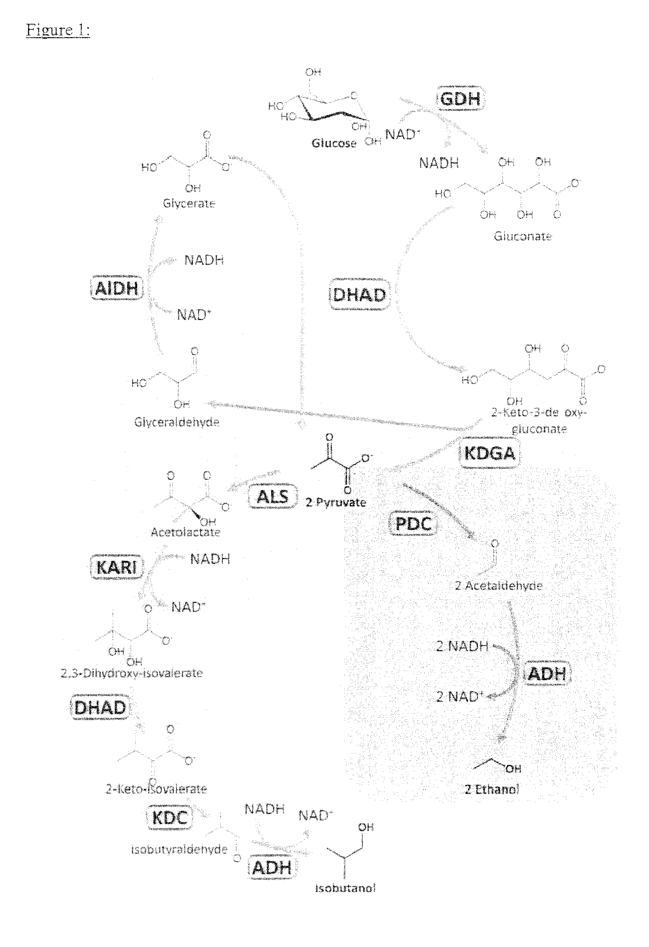

Figure 1 Schematic representation of cell-free reaction pathways to ethanol and isobutanol via minimized reaction cascades. In the first part of the reaction (top box) glucose is converted into two molecules of pyruvate. Depending on the desired final product and the enzymes applied, pyruvate can be either directed to ethanol (lower right box) or isobutanol synthesis (lower left box) in the second part of the reaction cascade. For clarity protons and molecules of CO2 and H2O that are acquired or released in the reactions are not shown.
Figure 2: Cell-free synthesis of ethanol. a: Intermediates in concentrations > 5 mM; closed circles: glucose concentration, open circles: gluconate concentration, closed triangles: ethanol. b: Intermediates in concentrations < 5 mM; closed circles, dashed line: KDG, open circles, dashed line: pyruvate, closed triangles, dashed line: glycerate, open triangles, dashed line: acetaldehyde. (Note that the concentration of glucose, gluconate and KDG was duplicated to allow for a better comparison with ethanol concentration (1 mol glucose is converted to 2 mol ethanol). All data points represent average values from three independent experiments.)

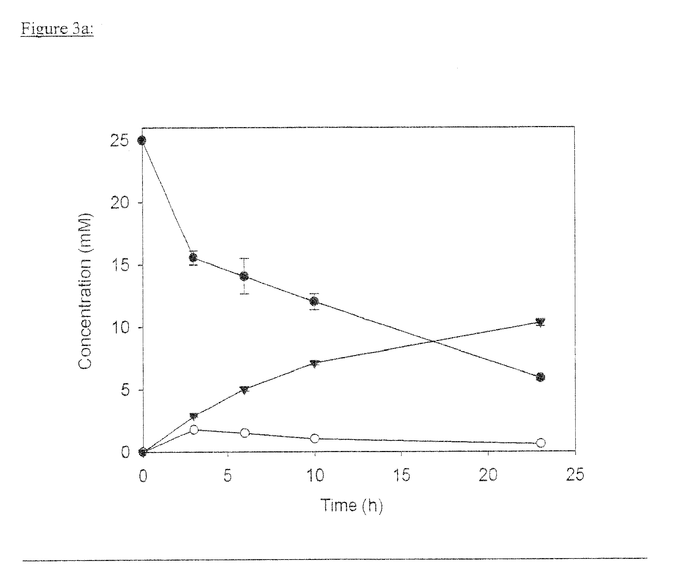

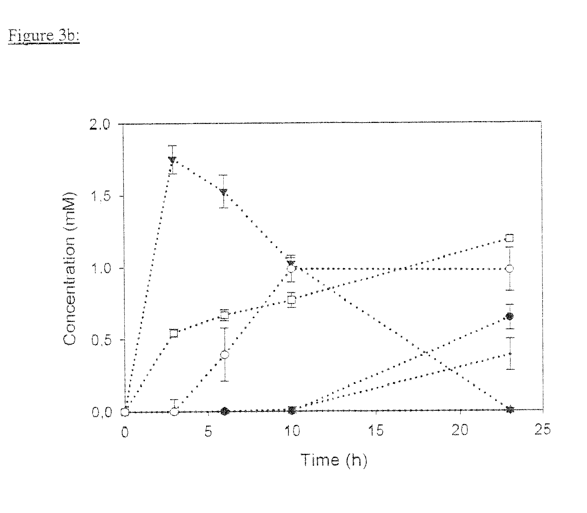

Figure 3: Cell-free synthesis of isobutanol. a: Intermediates in concentrations > 2 mM; closed circles: glucose concentration, open circles: gluconate concentration, closed triangles: isobutanol. b: Intermediates in concentrations < 2 mM; closed circles, dashed line: KDG, dots, dashed line: pyruvate, closed triangles, dashed line: glycerate, open squares, dashed line: isobutyraldehyde; open circles, dashed line: KIV. DHIV could not be detected at all. All data points represent average values from three independent experiments.

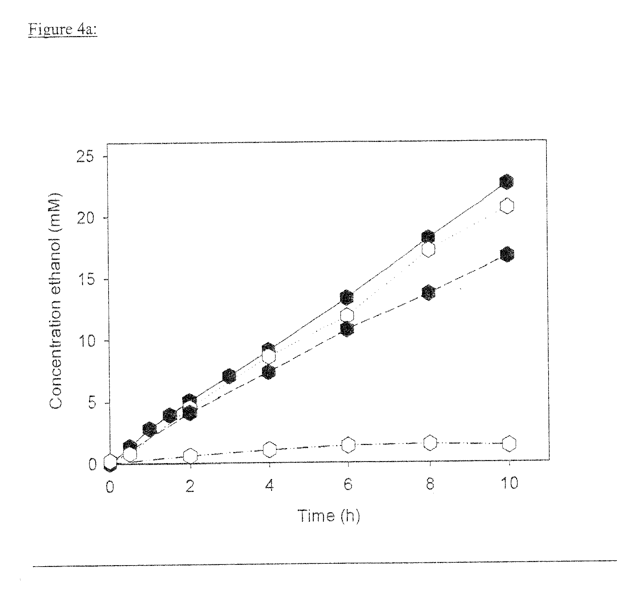

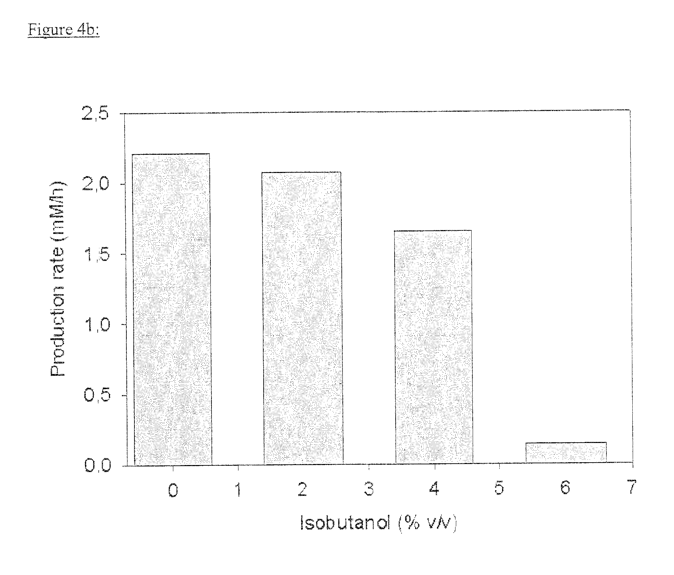

Figure 4: Ethanol production at different isobutanol concentrations. Closed diamonds, straight line: 0% isobutanol; open diamonds, dotted line: 2% isobutanol; closed diamonds, dashed line: 4% isobutanol; open diamonds, dashed-dotted line: 6% isobutanol. b: ethanol production rate (mM/h) plotted against isobutanol concentration.

### Examples

The present invention is further defined in the following examples. It should be understood that these examples are given by way of illustration only and are not limiting the scope of the invention. From the above discussion and these examples, a person skilled in the art can ascertain the essential characteristics of this invention and without departing from the spirit and scope thereof, can make various changes and modifications of the invention to adapt it to various uses and conditions.

Substrate and product concentrations in the herein described experiments are comparably low. For allowing easy product separation, for more economic processes, the product concentration may be increased above the solubility limit, which for example for isobutanol is 1.28 M at 20 °C (ca. 95 g/l). The product solubility can also be lowered by increasing process temperature and adjusting salt concentrations.

In one embodiment, 1 mol glucose or galactose is converted to 1 mol isobutanol in the described system, therefore substrate concentrations are to be chosen according to the desired end concentration (e.g. 230 g/l glucose or galactose) or higher.

Furthermore, a continuously running process comprising constant substrate feed (glucose syrup) and product removal (organic phase) is further advantageous, given that enzymes and cofactors are retained, e.g. by immobilization.

### Example 1 (Ethanol synthesis)

One general example of the feasibility of the cell-free synthesis toolbox, glucose or galactose was converted to pyruvate using the enzyme cascade of conversion of glucose or galactose to pyruvate with four enzymes, comprising glucose dehydrogenase (GDH), gluconate/glycerate/ dihydroxyacid dehydratase (DHAD), 2-keto-3-deoxygluconate aldolase (KDGA) and glyceraldehyde dehydrogenase (ALDH). The ALDH used in this example is defined by SEQ ID NO 10 as established in Example 4.

In a subsequent two-step reaction pyruvate was converted to acetaldehyde and then to ethanol by action of pyruvate decarboxylase (PDC) (J. Mol. Catal. B-Enzym. 2009, 61, 30-35)and alcohol dehydrogenase (ADH) (Protein Eng. 1998, 11, 925-930). The PDC from Zymomonas mobilis was selected due to its relatively high thermal tolerance and activity. Despite its mesophilic origin, Z. m. PDC is thermostable up to 50 °C (see table 10) which is in accord with the temperature range of more thermostable enzymes. Consequently, experiments were carried out at 50 °C. The six required enzymes were recombinantly expressed in E. coli and subjected to different purification regimes. Using this set of enzymes, together with 5 mM NAD, 25 mM glucose was converted to 28.7 mM ethanol (molar yield of 57.4 %) in 19 h **(****Figure 2****).** Based on the initial substrate and cofactor concentrations these results clearly demonstrate successful recycling of NAD and NADH, and, since the overall product yield exceeds 50 %, that glyceraldehyde resulting from 2-keto-3-deoxygluconate cleavage was successfully redirected towards pyruvate. Next to ethanol and glucose, reaction intermediates such as gluconate, 2-keto-3-deoxygluconate, pyruvate, glycerate and acetaldehyde were monitored during the course of the reaction. Especially for gluconate, the substrate of DHAD, a temporary accumulation of up to 8 mM was detected during the first 10 h of the reaction. In contrast, glycerate and acetaldehyde concentrations did not exceed 4 mM, while pyruvate was not detectable.

While residual intermediates generally accumulated at the end, gluconate maximum was measured between 8 and 10 h during the course of the reaction. Notably, undesired side-products such as lactate and acetate were not detected, indicating that the selected enzymes did provide the necessary substrate specificity. Although the enzyme-catalyzed reaction was not completed over the course of the experiment, the cumulative mass of all detectable intermediates and product gives a yield in excess of 80 %.

**Table 1:**

| **Enzyme** | **EC** | **Source organism / Seq ID** | **Activity^{a}, 50°C (U/mg)** | **Half-life, 50°C (h)** | **T-Optimum (°C)** | **E₅₀ (% v/v)** | **I₅₀ (% v/v)** |
|---|---|---|---|---|---|---|---|
| GDH | 11147 | *S solfatancus* / *Seq ID 02* | 15 | >24 | 70 | 30 (45 °C) | 9 (45 °C) |
| DHAD | 421.39 | *S solfatancus* / *Seq ID 04* | 066, 0011, 038 | 17 | 70 | 15 (50 °C) | 4 (50 °C) |
| KDGA | 42114 | *S acidocaldanus* / *Se ID 06* | 4 | >24 | 99^{[1]} | 15 (60°C) | >12 (60°C)^{b} |
| ALDH | 1.2 1.3 | *T acidophilum°* / *Seq ID 10* | 1 | 12 | 63^{[2]} | 13 (60 °C) | 3 (50 °C) |
| PDC | 4111 | *Z mobilis* / *Seq ID 20* | 64 | 22 | 50 | 20 (50 °C) | 8 (45°C) |
| ADH | 1111 | *G stearothermophilus* / *Seq ID 18* | 210, 83 | >24 | >60^{[3]} | 25 (50 °C) | 5 (50 °C) |

Table 1: Enzymes used in the cell-free synthesis of ethanol. a: activity for natural substrates, DHAD for gluconate, glycerate and dihydroxyisovalerate, ADH for acetaldehyde and isobutyraldehyde (resp.) as substrates; b: above solubility, c: enzyme was engineered, E50: Ethanol concentration which causes loss of 50% activity. I50: Isobutanol concentration which causes loss of 50% activity; n.d.: not determined. ([1] S. Wolterink-van Loo, A. van Eerde, M. A. J. Siemerink, J. Akerboom, B. W. Dijkstra, J. van der Cost, Biochem. J. 2007, 403, 421-430; [2] M. Reher, P. Schonheit, FEBS Lett. 2006, 580, 1198-1204; [3] G. Fiorentino, R. Cannio, M. Rossi, S. Bartolucci, Protein Eng. 1998, 11, 925-930.)

### Example 2 (Isobutanol synthesis)

This example demonstrates the successful conversion of pyruvate to isobutanol using only four additional enzymes (see **Fig. 2****, Table 2)** in a completely cell-free environment. Initially, two pyruvate molecules are joined by acetolactate synthase (ALS) (FEMS Microbiol. Lett. 2007, 272, 30-34) to yield acetolactate, which is further converted by ketolacid reductoisomerase (KARI) (Accounts Chem. Res. 2001, 34, 399-408) resulting in the natural DHAD substrate dihydroxyisovalerate. DHAD then converts dihydroxyisovalerate into 2-ketoisovalerate.

**Table 2:**

| **Enzyme** | **EC** | **Source organism** | **Activity^{a}, 50°C (U/mg)** | **Half-life, 50°C (h)** | **T-Optimum (°C)** | **E₅₀ (% v/v)** | **I₅₀ (% *v*/*v)*** |
|---|---|---|---|---|---|---|---|
| GDH | 11147 | S *solfatancus* / *Seq ID 02* | 15 | >24 | 70 | 30 (45 °C) | 9 (45 °C) |
| DHAD | 42139 | *S salfatancus* / *Seq ID 04* | 066, 0011, 038 | 17 | 70 | 15 (50 °C) | 4 (50 °C) |
| KDGA | 42114 | *S acidocaldanus* / *Seq ID 06* | 4 | >24 | 99^{[1]} | 15 (60 °C) | >12 (60 °C)^{b} |
| ALDH | 1213 | *T acidophilum°* / *Seq ID 10* | 1 | 12 | 63^{[2]} | 13 (60°C) | 3 (50 °C) |
| | | | | | | | |
| ADH | 1111 | *G stearothermophilus* / *Seq ID 18* | 210, 83 | >24 | >60^{[3]} | 25 (50 °C) | 5 (50 °C) |
| ALS | 2216 | *B subtilis* / *Seq ID 12* | 30 | 12 | 37^{[4]} | n d | 4 (50°C) |
| KARI | 11186 | *M ruber* / *Seq ID 14* | 07 | 34 | 55 | n d | 8 (40 °C) |
| KDC | 41172 | *L lactis* / *Seq ID 16* | 150 | >24 | 50^{[5]} | nd | 4 (45 °C) |

Table 2: Enzymes used in the cell-free synthesis of isobutanol. a: activity for natural substrates, DHAD for gluconate, glycerate and dihydroxyisovalerate, ADH for acetaldehyde and isobutyraldehyde (resp.) as substrates; b: above solubility, c: enzyme was engineered, E50: Ethanol concentration which causes loss of 50% activity. I50: Isobutanol concentration which causes loss of 50% activity; n.d.: not determined. ([1] S. Wolterink-van Loo, A. van Eerde, M. A. J. Siemerink, J. Akerboom, B. W. Dijkstra, J. van der Oost, Biochem. J. 2007, 403, 421-430; [2] M. Reher, P. Schonheit, FEBS Lett. 2006, 580, 1198-1204; [3] G. Fiorentino, R. Cannio, M. Rossi, S. Bartolucci, Protein Eng. 1998, 11, 925-930; [4] F. Wiegeshoff, M. A. Marahiel, FEMS Microbiol. Lett. 2007, 272, 30-34; and [5]D. Gocke, C. L. Nguyen, M. Pohl, T. Stillger, L. Walter, M. Mueller, Adv. Synth. Catal. 2007, 349, 1425-1435.)

The enzymes 2-ketoacid decarboxylase (KDC) (J. Mol. Catal. B-Enzym. 2009, 61, 30-35) and an ADH (Protein Eng. 1998, 11, 925-930) produce the final product isobutanol via isobutyraldehyde. Again the substrate ambiguity of DHAD is exploited to minimize the total number of enzymes required.

In analogy to ethanol production, the enzymes of the general pyruvate synthesis route differ from the following three biocatalysts with respect to thermal stability, solvent tolerance and activity profiles (Table 2). To allow experimental comparison, reaction conditions remained the same as described previously. The activity of the enzymes was adjusted to to 0.12 U per mM glucose in the reaction for the GDH, to 0.6 U for the DHAD and to 0.2 U for the remaining enzymes. Measurements indicated that 19.1 mM glucose was converted to 10.3 mM isobutanol within 23 h, which corresponds to a molar yield of 53 % (Fig. 4). During the first 10 h of the reaction, product formation rate was 0.7 mM/h, which is similar to the ethanol formation rate of 2.2 mM/h (2 mol of ethanol instead of 1 mol of isobutanol is produced from 1 mol glucose). In contrast to the ethanol synthesis, only a minor accumulation of the DHAD substrates gluconate and glycerate was detected, resulting in a maximum of 1.8 mM for each of these intermediates. Additional reaction intermediates such as 2-keto-3-deoxygluconate, pyruvate, 2-ketoisovalerate, and isobutyraldehyde were measured at low concentrations (maximum 1.2 mM) but slowly increased towards the end of the measurement. Again substrate conversion was not completed within the monitored time. As with cell-free ethanol biosynthesis, quantification of all detectable intermediates gave a yield of 80 %.

In analogy to isobutanol production with glucose, galactose was used as a substrate. To allow experimental comparison, reaction conditions remained the same. Measurements indicated that galactose was converted to 7,5 mM isobutanol within 23 h, which corresponds to a molar yield of 38 %.

### Example 3 (Solvent tolerance)

A key characteristic of cell-free systems is their pronounced tolerance against higher alcohols. To evaluate solvent tolerance of the artificial enzyme cascade, glucose conversion to ethanol was conducted as in Example 1 in the presence of increasing isobutanol concentrations **(****Fig. 4****).**

In contrast to microbial cells, where minor isobutanol concentrations (ca. 1% v/v) already result in loss of productivity, presumably through loss of membrane integrity, cell-free ethanol productivity and reaction kinetics were not significantly affected by isobutanol concentrations up to 4 % (v/v). Only in the presence of 6 % (v/v) isobutanol, ethanol productivity rapidly declined (1.4 mM ethanol in 8 h). This demonstrates that cell-free processes have the potential to tolerate much higher solvent concentrations than equivalent whole-cell systems. Based on the current data ALDH has the lowest solvent tolerance, as 3 % (v/v) isobutanol already induce adverse effects on activity. In contrast, KDGA remains completely active even in a two-phase isobutanol/water system, which forms spontaneously at product titers above 12 % (v/v) (see table 2). As shown for an engineered transaminase, which remains active in a reaction medium containing 50 % DMSO, such short- comings can be addressed by engineering of the respective protein. In comparison, there is neither a successful example nor a straight-forward technology in place to engineer an entire cell for solvent tolerance. It is expected, that all enzymes utilized in the cell-free pathways can be engineered to be as solvent tolerant as KDGA or can be replaced by a stable naturally occurring equivalent, so that isobutanol production is achieved in a two phase system. Product recovery by a simple phase separation would significantly simplify the downstream processing (Ind. Eng. Chem. Res. 2009, 48, 7325-7336) and, while conceivable with a cell-free system, it is highly unlikely to be realized by microbial fermentation.

### Example 4 (Directed evolution of TaALDH)

### Generation of Ta-aldh libraries by random mutagenesis:

Random mutations were introduced into Ta-aldh gene by PCR under error prone conditions according to the protocol of Jaeger et al. (Applied Microbiology and Biotechnology, 2001. 55(5): p. 519-530). Mutated Ta-aldh genes were purified, cut, ligated (via Xbal and Bsal) into pCBR-Chis and used to transform E. coli BL21 (DE3) to create expression library. Ta-aldh libraries created by random mutagenesis were calculated to have 1.3-3 base pair changes per Ta-aldh gene. This calculation was based on the reference that a concentration of 0,1 mmol/l MnCl2 in the PCR reaction leads to a mutation rate of about 1-2 bases per 1000 bases. The Ta-aldh gene contains 1515 bases.

The following primers, reaction conditions and temperature program were used in the PCR reaction:
• Primers:

| | |
|---|---|
| Fw-Mut (65°C) | GAATTGTGAGCGGATAACAATTCCC |
| Rev-Mut (65°C) | CTTTGTTAGCAGCCGGATCTC |

• ₀ PCR mixture:

| | | |
|---|---|---|
| 10x Taq buffer (NH4) Fermentas® | 5 µl | (1x) |
| dNTP-Mix (10 mmol/l) | 1 µl | (0,2 mM) |
| MnC12 (1 mmol/l) | 5 µl | (0,1 mM) |
| MgCl2 (25 mmol/l) | 8 µl | (4 mM) |
| Fw Mut Primer (c = 10 pmol/µl) | 2.5 µl | (0,5 mM) |
| Rev Mut Primer (c = 10 pmol/µl) | 2.5 µl | (0,5 mM) |
| Template (pCBR-taALDH-CH) (50 ng/µl) | 10 µl | (500 ng) |
| sterile dest. H2O | 15 µl | |
| Taq-Polymerase (5 U/µl) Fermentas® | 1 µl | (2,5 U) |
| Total volume | 50 µl | |

• Temperature program:

| Step | Denaturation | Annealing | Extension |
|---|---|---|---|
| 1 | 95°C, 5 min | | |
| 2 (25x) | 95°C, 45 s | 55°C, 45 s | 72°C, 3 min |
| 3 | | | 72°C, 5 min |

Followed by purification with MN Gelextraction kit.

### Generation of Ta-ALDH libraries by site directed mutagenesis

TaALDH-variants with improved properties were found by screening method described below. Mutations in TaALDH were detected by sequencing Ta-aldh gene (GATC Biotech, Cologne, Germany). Base triplets coding for beneficial amino acid changes were isolated and saturated by quickchange PCR according to the protocol of Wang and Malcolm (Biotechniques, 1999. 26(4): p. 680-68). Mutations were inserted into pCBR- Ta-ALDH -Chis using degenerative primer pairs. Quickchange PCR product was purified from pCBR-Ta-aldh-Chis template and used to transform E. coli BL21 (DE3) to create expression library.

| **quickchange** | | | | | | |
|---|---|---|---|---|---|---|
| **wang und malcolm** | | | | | | |
| | **Stammlsg** | | **PCR-conc** | PCR-vol | **PCR-vol** | |
| **Template DNA ca.** | 20 | ng/µL | 1 | ng/µL | 1 | µL |
| fw-/rev-Primer | 1 | pmol/µL (µM) | 0.25 | pmol/µl (µM) | 5 | µL |
| **dNTP-Mix** | 10 | mM | 0,2 | mM | 0,4 | µL |
| **HF-Puffer** | 5 | x | 1 | x | 4 | µL |
| **Fusion-Polymerase** | 2 | U/µL | 0,04 | U/µL | 0,4 | µL |
| **dH₂O in µl** | | | | | 9,2 | µL |

### First 10 cycles:

| Cycles | Temperature | Time |
|---|---|---|
| 1x | 98 °C | 3 min |
| 10x | 98 °C | 10 sec |
| | 65 °C (dep. Primer Annealing) | 30 sec |
| | 72 °C | 3 min (15 - 30sec/kb) |
| 1x | 72 °C | 10 min |

15µL each from sample using fw-Primer and rev-Primer were pooled.

### Second 25 cycles (same conditions as above):

| Cycles | Temperature | Time |
|---|---|---|
| 1x | 98 °C | 3 min |
| 25x | 98 °C | 10 sec |
| | 65 °C (dep. Primer Annealing) | 30 sec |
| | 72 °C | 3 min (15 - 30sec/kb) |
| 1x | 72 °C | 10 min |

### Primerlist:

Saturation mutagenesis on amino acid position 34:

| | |
|---|---|
| Fw-F34 (71 °C) | CGGTCAGGTTATTGGTCGTNNKGAAGCAGCAACCCGTG |
| Rev-F34 (71 °C) | CACGGGTTGCTGCTTCMNNACGACCAATAACCTGACCG |

Saturation mutagenesis on amino acid position 405:

| | |
|---|---|
| Fw-S405 (64°C) | GTATGATCTGGCCAATGATNNKAAATATGGTCTGGCCAG |
| Rev-S405 (64°C) | CTGGCCAGACCATATTTMNNATCATTGGCCAGATCATAC |

Saturation mutagenesis on amino acid position 271:

| | |
|---|---|
| Fw-W271 (60°C) | GAAAACCCTGCTGNNKGCAAAATATTGGAATG |
| Rev-W271 (60°C) | CATTCCAATATTTTGCMNNCAGCAGGGTTTTC |

Saturation mutagenesis on amino acid position 399:

| | |
|---|---|
| Fw-Y399 (59°C) | CGTGGAAGAAATGNNKGATCTGGCCAAT |
| Rev-Y399 (59°C) | ATTGGCCAGATCMNNCATTTCTTCCACG |

### Quickchange PCR for specific variants:

Variant F34L

| | |
|---|---|
| Fw-F34L (75°C) | GGTCAGGTTATTGGTCGTTTAGAAGCAGCAACCCGTG |
| Rev-F34L (75°C) | CACGGGTTGCTGCTTCTAAACGACCAATAACCTGACC |

Variant F34M

| | |
|---|---|
| Fw-F34M (68°C) | GTCAGGTTATTGGTCGTATGGAAGCAGCAACCCGT |
| Rev-F34M (68°C) | ACGGGTTGCTGCTTCCATACGACCAATAACCTGAC |

Variant W271 S

| | |
|---|---|
| Fw-W271S (65°C) | GAAAACCCTGCTGTCGGCAAAATATTGGAATG |
| Rev-W271 S (65°C) | CATTCCAATATTTTGCCGACAGCAGGGTTTTC |

Variant Y399C

| | |
|---|---|
| Fw-Y399C (63°C) | CGTGGAAGAAATGTGTGATCTGGCCAATG |
| Rev-Y399C (63°C) | CATTGGCCAGATCACACATTTCTTCCACG |

Variant S405C

| | |
|---|---|
| Fw-S405C (73°C) | GTATGATCTGGCCAATGATTGCAAATATGGTCTGGCC |
| Rev-S405C (73°C) | GGCCAGACCATATTTGCAATCATTGGCCAGATCATAC |

Variant S405N

| | |
|---|---|
| Fw-S405N (68°C) | TGATCTGGCCAATGATAACAAATATGGTCTGGCCA |
| Rev-S405N (68°C) | TGGCCAGACCATATTTGTTATCATTGGCCAGATCA |

### Screening for improved TaALDH variants

Colonies of E. coli BL21 (DE3) containing Ta-ALDH library were transferred into 96-deepwell plates (Gainer BioOne) containing Zym5052 autoinduction medium (Protein Expression and Purification, 2005. 41(1): p. 207-234). Cultures were grown over night at 37 °C, 1000 rpm. Cells were harvested by centrifugation at 5000 g at 2 °C for 2 min and lysed with B-Per® protein extraction reagent (Thermo Fisher Scentific, Rockford, USA). After incubation for 60 min at 50 °C, insoluble cell debris and was removed by centrifugation at 5000 g at 20 °C for 30 min. Supernatants containing variants of TaALDH were tested for relative activity under standard conditions: 2 mM NAD, 1 mM D-glyceraldehyde in 50 mM HEPES (pH 7) at 50 °C. After 20 min NADH formation was detected spectrophotometrically at 340nm. Superior NADH formation compared to wild type TaALDH indicated an improved relative activity of TaALDH variant.

Activity in solvents of Ta-ALDH variants was tested under standard assay conditions containing additional solvent. After 20 min NADH formation was detected and compared to relative activity.

Change in cofactor acceptance of Ta-ALDH variants was tested under standard assay conditions but with 10 mM NAD. After 20 min NADH formation was detected and compared to relative activity.

### Determination of specific activity of TaALDH Variants

The Ta-aldh gene was cloned in pCBR-Chis expression vector as described above. Mutations F34L, F34M, Y399C, S405C and S405N were inserted into Ta-aldh gene by Quickchange PCR as described above. For recombinant expression, E. coli BL21 (DE3) was transformed with pCBR-Ta-aldh-Chis, pCBR-Ta-aldh-f34l-s405c-Chis, pCBR-Ta-aldh-f34m-s405n-Chis or pCBR-Ta-aldh-f34m-y399c-s405n-Chis. Each of the four variants was produced using the following protocol:

Large amounts of a TaALDH-Variant were produced with fed-batch fermentation in a 40 L Biostat Cplus bioreactor (Sartorius Stedim, Goettingen, Germany). Defined media was supplemented with 30 µg/ml kanamycin. After inoculation cells were grown at 30° C for 24 h and induced with 0.3 mM IPTG. Enzyme expression was performed at 30° C for 3 h. One fermentation produced 300 g cells (wet weight). Cells were harvested and lysed with Basic-Z Cell Disruptor (Constant Systems, Northants, UK) in loading buffer (200 mM NaCl; 20 mM Imidazol; 2.5 mM MgCl2; 50 mM NaPi, pH 6.2). After heat treatment at 50 °C for 30 min, cell debris and protein aggregates were separated from soluble fraction by centrifugation at 30,000 g at 20 °C for 30 min (Sorvall RC6+, SS-34 rotor, Thermo Scientific).

Soluble fraction of His-tagged TaALDH-Variant was further purified by Ni-NTA chromatography using ÄKTA UPC-900 FPLC-system (GE Healthcare, Freiburg, Germany). Supernatant was loaded on HiTrap FF-column and washed with two column volumes of loading buffer. Highly purified and concentrated TaALDH was fractioned after elution in imidazole buffer (200 mM NaCl; 500 mM Imidazol; 50 mM NaPi, pH 6.2). Buffer was changed to 20 mM (NH4)HCO3 with HiPrep 26/10 Desalting column and TaALDH was lyophilized with an Alpha 2-4 LD Plus freeze dryer (Martin Christ GmbH, Osterode am Harz, Germany).

TaALDH activity was determined spectrophotometrically at 50°C by measuring the rate of cofactor reduction at 340 nm in flat bottom microtiter plates (Grainer BioOne) with a Fluorostar Omega Photometer (BMG Labtech GmbH, Ortenberg, Germany). One unit of activity was defined as reduction of 1 µmol of cofactor per minute. Reaction mixtures (total volume 0.2 mL) contained 1 mM D-Glyceraldehyde and 2 mM NAD and appropriate amounts of enzyme in 100 mM HEPES pH 7.

The values "relative to wt" are the mU/mL values normalized to the wildtype control value.

**Table 3:**

| | | **specific activity (U/mg) at 2 mM NAD** | **2 mM NAD (mU/mL)** | **Error** | **relative to wt** |
|---|---|---|---|---|---|
| **wildtype (wt)** | Seq ID NO. 8 | 0,2 | 2,89 | 0,44 | 1,0 |
| **F34L** | SEQ ID NO: 57 | | 9,09 | 0,92 | 3,1 |
| **S405C** | SEQ ID NO: 59 | | 19,29 | 0,85 | 6,7 |
| **F34L S405C** | SEQ ID NO: 61 | 1 | 15,23 | 3,41 | 5,3 |
| **F34M S405N** | SEQ ID NO: 63 | 1,2 | 25,87 | 2,32 | 9,0 |
| **F34M W271S S405N** | | | 125,38 | 4,32 | 43,4 |
| **W271S** | SEQ ID NO: 65 | | 58,35 | 1,58 | 20,2 |
| **F34M Y399C S405N** | Seq ID NO. 10 | 1,2 | 130,81 | 5,79 | 45,3 |
| **Y399R** | SEQ ID NO: 67 | | 3,76 | 0,14 | 1,3 |
| **F34M W271S Y399C S405N** | SEQ ID NO: 69 | | 3,00 | 0,52 | 1,0 |

**Table 4:**

| | | **10 mM NAD (mU/mL)** | **Error** | **relative to wt** | **10 mM NAD / 2 mM NAD (mU/mL)** |
|---|---|---|---|---|---|
| **wildtype (wt)** | Seq ID NO. 8 | 11,92 | 1,82 | 1,0 | 1,0 |
| **F34L** | SEQ ID NO: 57 | 31,72 | 3,03 | 2,7 | 0,8 |
| **S405C** | SEQ ID NO: 59 | 66,82 | 3,00 | 5,6 | 0,8 |
| **F34L S405C** | SEQID NO: 61 | 46,83 | 9,92 | 3,9 | 0,7 |
| **F34M S405N** | SEQ ID NO: 63 | 74,31 | 6,79 | 6,2 | 0,7 |
| **F34M W271S S405N** | | 279,58 | 9,86 | 23,5 | 0,5 |
| **W271S** | SEQ ID NO: 65 | 181,89 | 5,93 | 15,3 | 0,8 |
| **F34M Y399C S405N** | Seq ID NO: 10 | 396,91 | 15,22 | 33,3 | 0,7 |
| **Y399R** | SEQ ID NO: 67 | 11,56 | 0,51 | 1,0 | 0,7 |
| **F34M W271S Y399C S405N** | SEQ ID NO: 69 | 8,08 | 1,82 | 0,7 | 0,7 |

**Table 5:**

| | | **3 % isobutanol, 2 mM NAD (mU/mL)** | **Error** | **relative to wt** | **Stab. rel to wt** |
|---|---|---|---|---|---|
| **wild** | Seq ID NO. 8 | 1,60 | 0,29 | 1,0 | 1,0 |
| **F34L** | SEQ ID NO: 57 | 4,08 | 0,13 | 2,6 | 0,8 |
| **S405C** | SEQ ID NO: 59 | 12,65 | 0,76 | 7,9 | 1,2 |
| **F34L S405C** | SEQ ID NO: 61 | 8,17 | 2,54 | 5,1 | 1,0 |
| **F34M S405N** | SEQ ID NO: 63 | 15,62 | 1,42 | 9,8 | 1,1 |
| **F34M W271S S405N** | | 43,86 | 1,74 | 27,5 | 0,6 |
| **W271S** | SEQ ID NO: 65 | 20,80 | 1,10 | 13,0 | 0,6 |
| **F34M Y399C S405N** | Seq ID NO. 10 | 74,77 | 4,25 | 46,9 | 1,0 |
| **Y399R** | SEQ ID NO: 67 | 2,16 | 0,01 | 1,4 | 1,0 |
| **F34M W271S Y399C S405N** | SEQ ID NO: 69 | 1,30 | 0,57 | 0,8 | 0,8 |

### Reagents:

Restriction enzymes, Klenow fragment, T4 ligase and T4 kinase were purchased from New England Biolabs (Frankfurt, Germany). Phusion polymerase was from Finnzymes (Espoo, Finland), desoxynucleotides from Rapidozym (Berlin, Germany). All enzymes were used according to the manufacturers' recommendations, applying the provided buffer solutions. Oligonucleotides were ordered from Thermo Scientific (Ulm, Germany). Full-length genes were synthesized by Geneart (Regensburg, Germany), with optimized E. coli codon usage, and delivered in the company's standard plasmids. Porcine heart lactate dehydrogenase (LDH) was bought from Serva (Heidelberg, Germany), Aspergillus niger glucose oxidase and horseradish peroxidase from Sigma-Aldrich (Munich, Germany). All chemicals were, unless otherwise stated, purchased in analytical grade from Sigma-Aldrich, Carl Roth GmbH (Karlsruhe, Germany), Serva Electrophoresis GmbH and Merck KGaA (Darmstadt, Germany).

### Strains and Plasmids:

E. coli BL21 (DE3) (F- ompT hsdSB (rB- mB-) gal dcm (DE3)) was purchased from Novagen (Nottingham, UK), E. coli XL1-Blue (recA1 endA1 gyrA96 thi-1 hsdR17 supE44 relA1 lac [F' proAB laclqZΔM15 Tn10 (Tetr)]) from Stratagene (Waldbronn, Germany). pET28a-DNA was provided by Novagen.

### Vector construction:

Plasmids pCBR, pCBRHisN and pCBRHisC were constructed on the basis of pET28a (Novagen). DNA-sequences (see Table 6) for the corresponding new multiple cloning sites were synthesized (Geneart, Regensburg, Germany) and cloned into pET28a via XbaI/BamHI (pCBR), NdeI/EcoRI (pCBRHisN) or XbaI/Bpu1102I (pCBRHisC), thereby replacing the existing multiple cloning site with a new restriction site containing a BfuAI- and a Bsal-sequence and, in case of pCBR and pCBRHisN, a stop codon.

**Table 6: Vector multiple cloning sites**

| Name | DNA-Sequence (5' → 3') |
|---|---|
| pCBR | ATATATATATTCTAGAAATAATTTTGTTTAACTTTAAGAA GGAGATATACATATGATGCAGGTATATATATATTAATAG AGACCTCCTCGGATCCATATATATAT |
| pCBRHisN | ATATATATATCATATGATGCAGGTATATATATATTAATAG AGACCTCCTCGAATTCATATATATAT |
| pCBRHisC | ATATATATATTCTAGAAATAATTTTGTTTAACTTTAAGAA GGAGATATACATATGATGCAGGTATATATATATATAGCGGG AGACCTGTGCTGGGCAGCAGCCACCACCACCACCACC ACTAATGAGATCCGGCTGCTAACAAAGCCCGAAAGGAA GCTGAGTTGGCTGCTGCCACCGCTGAGCATATATATAT |

The three new vectors allow the simultaneous cloning of any gene using the same restriction sites, enabling the user to express the respective gene without or with an N- or C-terminal His-tag, whereby a stop codon must not be attached at the 3'-end of the gene. Vector-DNA was first restricted with Bsal, followed by blunt end generation with Klenow fragment. Afterwards, the linearized plasmids were digested with BfuAl, generating a 5'-overhang. Genes were amplified using the Geneart vectors as templates and the corresponding oligonucleotides (Table 7).

**Table 7: Oligonucleotides**

| Oligonucleotide Name | Gene amplified | Oligonucleotide Sequence (5' → 3') |
|---|---|---|
| SsGDH_for | *S. solfataricus* Glucose dehydrogenase | CAGCAAGGTCTCACATAT GAAAGCCATTATTGTGAA ACCTCCG |
| SsGDH_rev | *S. solfataricus* Glucose dehydrogenase | TTCCCACAGAATACGAAT TTTGATTTCGC |
| SsDHAD_for | *S. solfataricus* Dihydroxyacid dehydratase | CAGCAAGGTCTCACATAT GCCTGCAAAACTGAATAG CCC |
| SsDHAD_rev | S. *solfataricus* Dihydroxyacid dehydratase | TGCCGGACGGGTAACTG C |
| SaKDGA_for | *S. acidocaldarius* KDG aldolase | CAGCAAGGTCTCACATAT GGAAATTATTAGCCCGAT |
| | | TATTACCC |
| SaKDGA_rev | *S acidocaldanus* KDG aldolase | ATGAACCAGTTCCTGAAT TTTGCG |
| TaALDH_for | *T acidophilum* Glyceraldehyde dehydrogenase | CAGCAAGGTCTCACATAT GGATACCAAACTGTATAT TGATGGC |
| TaALDH_rev | *T acidophilum* Glyceraldehyde dehydrogenase | CTGAAACAGGTCATCACG AACG |
| MrKARI_for | *M ruber Ketolacid* reductoisomerase | CAGCAACGTCTCGCATAT GAAGATTTACTACGACCA GGACGCAG |
| MrKARI_rev | *M ruber* Ketolacid reductoisomerase | GCTACCGACCTCTTCCTT CGTGAAC |

After PCR, DNA fragments were digested with Bsal, 3'-phosphorylated (T4 kinase) and subsequently ligated into the appropriate vectors. In some cases, phosphorylation could be replaced by digestion using Psil. Plasmids were transformed into E. coli as described elsewhere (Molecular Cloning: A Laboratory Manual Cold Spring Harbor Laboratory Press Cold Spring Habor, NY, 1989). Sequence analysis was performed by GATC Biotech (Konstanz, Germany). pET28a-HisN-LIKdcA was cloned according to Gocke et al. (Adv. Synth. Catal. 2007, 349, 1425-1435)

### Enzyme expression:

Enzyme expression was performed using E. coli BL21 (DE3) or BL21 Rosetta(DE3)-pLysS as host strains, either in shaking flask cultures or in a 10 L Biostat Cplus bioreactor (Sartorius Stedim, Goettingen, Germany). All media were supplemented with 30-50 µg/ml kanamycin. GDH and DHAD were expressed in LB medium, acetolactate synthase in TB medium. After inoculation cells were grown at 37 °C to an optical density at 600 nm of 0.6, induced with 1 mM IPTG and the temperature lowered to 16-20 °C for 16-24 h expression. KDGA and ALDH were expressed according to the fed-batch cultivation method of Neubauer et al. (Biotechnol. Bioeng. 1995, 47, 139-146) at 37 °C. After inoculation cells were grown for 24 h and induced with 1 mM IPTG. Enzyme expression was performed for 24 or 30 h, respectively. KDC expression was performed for 22 h at 30 °C in batch mode using Zyp-5052 (Protein Expression Purif. 2005, 41, 207-234) as a medium. KARI was expressed in a batch fermentation using TB medium. Cells were grown at 37 °C to an optical density of 5.2 and induced by the addition of 0.5 mM IPTG. Afterwards, expression was performed for 24 h at 20 °C.

### Enzyme purification:

All protein purification steps were performed using an ÄKTA UPC-900 FPLC-system (GE Healthcare, Freiburg, Germany), equipped with HiTrap FF-, HiPrep 26/10 Desalting- and HiTrap Q-Sepharose FF-columns (GE Healthcare). Cell lysates were prepared with a Basic-Z Cell Disruptor (Constant Systems, Northants, UK), cell debris was removed by centrifugation at 35.000 g and 4 °C for 30 min (Sorvall RC6+, SS-34 rotor, Thermo Scientific). For lyophilization an Alpha 2-4 LD Plus freeze dryer (Martin Christ GmbH, Osterode am Harz, Germany) was used. GDH and DHAD were purified by heat denaturation (30 minutes at 70 °C, respectively). GDH was subsequently freeze-dried (SpeedVac Plus, Thermo Scientific), DHAD concentrated using a stirred Amicon cell (Milipore, Darmstadt, Germany) and either stored at -80 °C or directly applied to experiments. KDGA, ALDH and KDC were purified as previously described (Biochem. J. 2007, 403, 421-430; FEBS Lett. 2006, 580, 1198-1204; Adv. Synth. Catal. 2007, 349, 1425-1435) and stored as lyophilisates. ALS and KARI were purified via IMAC using 25 or 50 mM HEPES, pH 7. Elution was achieved with 500 mM imidazol. Enzymes were desalted and stored as a liquid stock (ALS) or lyophilisate (KARI).

### Protein determination:

Protein concentration was determined with the Roti-Nanoquant reagent (Carl Roth GmbH) according to the manufacturer's recommendations using bovine serum albumin as a standard.

### SDS-PAGE:

Protein samples were analyzed as described by Laemmli ( Nature 1970, 227, 680-685) using a Mini-Protean system from Biorad (Munich, Germany).

### Enzyme assays:

All photometrical enzyme assays were performed in microtiter plate format using a Thermo Scientific Multiskan or Varioskan photometer. When necessary, reaction mixtures were incubated in a waterbath (Julabo, Seelbach, Germany) for accurate temperature control. Buffers were prepared according to Stoll (Guide to Protein Purification, Vol. 466, Elsevier Academic Press Inc, San Diego, 2009, pp. 43-56), adjusting the pH to the corresponding temperature. Reactions using NAD or NADH as coenzymes were followed at 340 nm (molar extinction coefficient NADH = 6.22 L mmol-1 cm-1) Reaction mixtures (total volume 0.2 mL) contained 1 mM D-Glyceraldehyde and 2 mM NAD and appropriate amounts of enzyme in 100 mM HEPES pH 7. (J. Mol. Catal. B-Enzym. 2009, 61, 30-35). One unit of enzyme activity is defined as the amount of enzyme necessary to convert 1 µmol substrate per minute. In addition to the standard reaction conditions described below, enzyme activity was tested under reaction conditions (100 mM HEPES, pH 7, 2.5 mM MgCl2, 0.1 mM thiamine pyrophosphate) prior to alcohol synthesis experiments.

**GDH activity:** GDH activity was assayed at 50 °C by oxidizing D-glucose to gluconate, whereby the coenzyme NAD is reduced to NADH. Assay mixture contained 50 mM HEPES (pH 7), 2 mM NAD and 50 mM D-glucose. (J. Biol. Chem. 2006, 281, 14796-14804)

**DHAD activity:** DHAD activity was measured by an indirect assay. The assay mixture containing DHAD, 20 mM substrate and 100 mM HEPES (pH 7) was incubated at 50 °C. Afterwards the conversion of glycerate to pyruvate, gluconate to 2-keto-3-deoxygluconate or 2,3-dihydroxy-isovalerate to 2-ketoisovalerate, respectively, was determined via HPLC as described below.

**KDGA activity:** KDGA activity was followed in cleavage direction at 50 °C. Reaction mixture contained 50 mM HEPES (pH 7), 0.1 mM thiamine pyrophosphate, 2.5 mM MgCl2, 20 U PDC and 10 mM KDG. KDG cleavage was followed by HPLC as described below.

**ALDH activity:** ALDH activity was assayed at 50 °C by oxidizing D-glyceraldeyde to glycerate, whereby the coenzyme NAD is reduced to NADH. Assay mixture contained 50 mM HEPES (pH 7), 2.5 mM MgCl2, 2 mM NAD and 1 mM glyceraldehyde. (FEBS Lett. 2006, 580, 1198-1204)

**ALS activity:** ALS activity was determined by following pyruvate consumption at 50 °C. Reaction mixtures contained 25 mM HEPES (pH 7), 0.1 mM thiamine-pyrophosphate, 2.5 mM MgCl2, 15 mM sodium pyruvate. Pyruvate concentration in the samples was determined via lactate dehydrogenase as described elsewhere. (Biochem. J. 2007, 403, 421-430) **KARI activity:** KARI activity was assayed by following the NADH consumption connected to the conversion of acetolactate to 2,3-dihydroxy isovalerate at 50 °C. Assay mixture contained 5 mM acetolactate, 0.3 mM NADH, 10 mM MgCl2 and 50 mM HEPES, pH 7.

**KDC activity:** KDC activity was assayed by following the decarboxylation of 2-ketoisovalerate to isobutyraldehyde at 50 °C and 340 nm. Assay mixture contained 50 mM HEPES (pH 7), 0.1 mM thiamine-pyrophosphate, 2.5 mM MgCl2 and 60 mM 2-ketoisovalerate. Decarboxylation rate was calculated using the molar extinction coefficient of 2-ketoisovalerate (ε = 0.017 L mmol-1 cm-1). (J. Mol. Catal. B-Enzym. 2009, 61, 30-35)

**ADH activity:** ADH activity was determined by following the NADH-dependent reduction of isobutyraldehyde to isobutanol at 50 °C. Assay mixture contained 10 mM HEPES (pH 7.2), 5 mM isobutanal and 0.3 mM NADH.

**Glucose analysis:** Glucose oxidase was used for the quantification of glucose. Assay mixture contained 20 mM potassium phosphate (pH 6), 0.75 mM 2,2-azino-bis(3-ethylbenzthiazoline)-6-sulfonic acid (ABTS), 2 U glucose oxidase and 0.1 U peroxidase. After the addition of samples the reaction mixture was incubated for 30 min at 30 °C and the extinction at 418 and 480 nm measured. Assay calibration was performed using defined glucose standard solutions. (J. Clin. Chem. Clin. Biochem. 1979, 17, 1-7)

### GC-FID analysis:

Isobutyraldehyde and isobutanol or acetaldehyde and ethanol were quantified by GC-FID using a Thermo Scientific Trace GC Ultra, equipped with a flame ionization detector and a Headspace Tri Plus autosampler. Alcohol and aldehyde compounds were separated by a StabilWax column (30 m, 0.25 mm internal diameter, 0.25 µm film thickness; Restek, Bellefonte, USA), whereby helium (0.8 or 1.2 ml min-1) was used as the carrier gas. The oven temperature was programmed to be held at 50 °C for 2 min, raised with a gradient 10 °C min-1 to 150 °C and held for 1 min. Injector and detector were kept at 200 °C. Samples were incubated prior to injection at 40 °C for 15 min. Injection was done in the split mode with a flow of 10 ml min-1, injecting 700 µl using headspace mode.

### HPLC analysis:

Gluconate, 2-keto-3-deoxygluconate, pyruvate, glycerate, 2,3-dihydroxyisovalerate and 2-ketoisovalerate were separated and quantified by HPLC, using an Ultimate-3000 HPLC system (Dionex, Idstein, Germany), equipped with autosampler and a diode-array detector. Chromatographic separation of gluconate, 2-keto-3-deoxygluconate, pyruvate and glycerate was achieved on a Metrosep A Supp10-250/40 column (250 mm, particle size 4.6 µm; Metrohm, Filderstadt, Germany) at 65 °C by isocratic elution with 12 mM ammonium bicarbonate (pH 10), followed by a washing step with 30 mM sodium carbonate (pH 10.4). Mobile phase flow was adjusted to 0.2 ml min-1. 2,3-dihydroxyisovalerate and 2-ketoisovalerate were separated using a Nucleogel Sugar 810H column (300 mm, 7.8 mm internal diameter; Macherey-Nagel, Dueren, Germany) at 60 °C by isocratic elution with 3 mM H2SO4 (pH 2.2). Mobile phase flow was adjusted to 0.6 ml min-1. Sample volume was 10 µl in each case. System calibration was performed using external standards of each of the abovementioned intermediates. Samples were prepared by filtration (10 kDa MWCO, modified PES; VWR, Darmstadt, Germany) and diluted.

### Alcohol biosynthesis:

All reactions were set up in 20 ml GC vials. Reaction mixtures contained 100 mM HEPES (pH 7 at 50 °C), 0.1 mM thiamine-pyrophosphate, 2.5 mM MgCl2, 25 mM D-glucose and 5 mM NAD. Enzymes were added as follows: GDH: 6 U, DHAD: 20 U for ethanol synthesis and 30 U for isobutanol synthesis, all other enzymes: 10 U. Control reactions were performed either without enzymes or without D-glucose. Reaction mixtures were placed in a water bath at 50 °C and gently stirred at 100 rpm.

## Claims

1. A process for the production of a target organic compound from glucose, galactose or a mixture of glucose and galactose or a glucose-containing oligomer or polymer and/or a galactose-containing oligomer or polymer by a cell-free enzyme system, comprising the conversion of glucose and/or galactose to pyruvate as an intermediate product; wherein said process comprises the following steps:
(1) oxidation of glucose and/or galactose to gluconate and/or galactonate
(2) conversion of gluconate and/or galactonate to pyruvate and glyceraldehyde
(3) oxidation of glyceraldehyde to glycerate
(4) conversion of glycerate to pyruvate
(5) conversion of pyruvate from steps (2) and (4) to the target compound
wherein steps (1) and (3) are enzymatically catalyzed with one or more enzymes and said steps involve the use of said enzyme(s) to reduce a single cofactor which is added and/or present for electron transport; and wherein step (5) comprises an enzymatically catalyzed reaction involving the reduced form of the cofactor of steps (1) and (3); and wherein the process is performed at a temperature of at least 40°C over a period of at least 30 minutes.

2. The process of claim 1, wherein the temperature of the process is maintained in a range from 40-80°C, preferably in a range from 45°-70°C, more preferably in a range from 50°-60°C, and most preferred at or greater than 50°.

3. The process of claim 1 or claim 2, wherein the process is maintained at the given temperature for at least 1 hour, preferably at least 3 hours, more preferably at least 12 hours, even more preferably for at least 24 hours, most preferred for at least 48 hours, and most highly preferred for at least 72 hours.

4. The process of any one of the above claims, wherein step (1) comprises the use of a single dehydrogenase for the oxidation of glucose and/or galactose to gluconate and/or galactonate.

5. The process of any one of the above claims, wherein step (2) comprises the conversion of gluconate to 2-keto-3-deoxygluconate and of 2-keto-3-deoxygluconate to glyceraldehyde and pyruvate and / or the conversion of galaconate to 2-keto-3-deoxygalactonate and of 2-keto-3-deoxygalactonate to glyceraldehyde and pyruvate.

6. The process of any one of the above claims, wherein step (2) comprises the use of dehydroxy acid dehydratase and keto-3-deoxygluconate aldolase.

7. The process of any one of the above claims, wherein step (3) comprises the use of a dehydrogenase for the oxidation of glyceraldehyde to glycerate.

8. The process of any one of the above claims, wherein steps (1) and (3) are carried out with the use of a single dehydrogenase.

9. The process of any one of the above claims, wherein no net production of ATP occurs.

10. The process of any one of the above claims, wherein no ATPase or arsenate is added.

11. The process of any one of the above claims, wherein said process occurs without ATP and/or ADP as cofactors.

12. The process of any one of the above claims, wherein the single cofactor is selected from the group consisting of NAD/NADH, NADP/NADPH, and FAD/FADH2.

13. The process of claim 11, wherein the single cofactor is NAD/NADH.

14. The process of any one of the above claims, wherein the enzyme activity of each enzymatically catalyzed reaction step is adjusted so that it is the same or greater than the activity of any preceding enzymatically catalyzed reaction step.

15. The process of any one of the above claims, wherein the specific enzymatic activity when using glyceraldehyde as a substrate is at least 100 fold greater than using either acetaldehyde or isobutyraldehyde as a substrate, more preferably at least 500 fold greater, even more preferably at least 800 fold greater, most preferably at least 1000 fold greater.

16. The process of any one of the above claims, whereby
- for the conversion of glucose and/or galactose to pyruvate only enzymes selected from the group of dehydrogenases, dehydratases, and aldolases are used; and
- wherein one or more of said enzymes is selected from each group.

17. The process of any one of the above claims, wherein the conversion of glucose and/or galactose to pyruvate consists of the use of one or two dehydrogenases, one or two dehydratases, and one aldolase.

18. The process of any one of the above claims, wherein the conversion of glucose and/or galactose to pyruvate consists of the use of two dehydrogenases, one dehydratase, and one aldolase.

19. The process of any one of the above claims, wherein one of the enzyme combinations included in any one of the tables P-1, P-2-a, P-2-b, P-2-c, P-2-d, P-3-a, P-3-b, and P-3-c are employed for the conversion of glucose and/or galactose to pyruvate.

20. The process of any one of the above claims, wherein the enzymes used for the conversion of glucose and/or galactose to pyruvate are selected from the group consisting of Glucose dehydrogenase GDH (EC 1.1.1.47), Sulfolobus solfataricus, NP 344316.1, Seq ID 02; and Dihydroxy acid dehydratase DHAD (EC 4.2.1.9), Sulfolobus solfataricus, NP 344419.1, Seq ID 04; Gluconate dehydratase (EC 4.2.1.39), Sulfolobus solfataricus, NP_344505; Gluconate dehydratase (EC 4.2.1.39), Sulfolobus solfataricus, NP_344505, Mutation I9L; Gluconate dehydratase ilvEDD (EC 4.2.1.39), Achromobacter xylsoxidans; Gluconate dehydratase ilvEDD (EC 4.2.1.39), Metallosphaera sedula DSM 5348; Gluconate dehydratase ilvEDD (EC 4.2.1.39), Thermoplasma acidophilum DSM 1728; Gluconate dehydratase ilvEDD (EC 4.2.1.39), Thermoplasma acidophilum DSM 1728; 2-Keto-3-deoxygluconate aldolase KDGA (EC 4.1.2.14), Sulfolobus solfataricus, NP 344504.1; 2-Keto-3-deoxygluconate aldolase KDGA (EC 4.1.2.14), Sulfolobus acidocaldaricus, Seq ID 06; Aldehyde Dehydrogenase ALDH (EC 1.2.1.3), Flavobacterium frigidimaris, BAB96577.1; Aldehyde Dehydrogenase ALDH (EC 1.2.1.3), Thermoplasma acidophilum, Seq ID 08; Aldehyde Dehydrogenase ALDH (EC 1.2.1.3), Thermoplasma acidophilum, Mutations F34M + Y399C + S405N, Seq ID 10; Glycerate kinase (EC 2.7.1.), Sulfolobus solfataricus, NP_342180.1; Glycerate 2-kinase (EC 2.7.1.165), Sulfolobus tokodaii, Uniprot Q96YZ3.1; Enolase (EC 4.2.1.11), Sulfolobus solfataricus, NP 342405.1; Pyruvate Kinase (EC 2.7.1.40), Sulfolobus solfataricus, NP 342465.1; Glycerate dehydrogenase/hydroxypyruvate reductase (EC 1.1.1.29/1.1.1.81), Picrophilus torridus, YP_023894.1; Serine-pyruvate transaminase (EC 2.6.1.51), Sulfolobus solfataricus, NCBI Gen ID: NP_343929.1; L-serine ammonia-lyase (EC 4.3.1.17), EC 4.3.1.17, Thermus thermophilus, YP_144295.1 and YP_144005.1; and Alanine dehydrogenase (EC 1.4.1.1), Thermus thermophilus, NCBI-Gen ID: YP_005739.1.

21. The process of any one of the above claims, wherein the conversion of glucose to pyruvate consists of the conversion of one mole of glucose to two moles of pyruvate.

22. The process of any one of the above claims, wherein pyruvate is further converted to a target chemical, and wherein during such conversion 1 molecule NADH is converted to 1 molecule NAD per molecule pyruvate, and wherein such target chemical is preferably selected from ethanol, isobutanol, n-butanol and 2-butanol.

23. The process of any one of the above claims, wherein one of the enzyme combinations included in any one of the tables E-1, I-1, N-1, T-1, T-2, and T-2a are employed for the conversion of pyruvate to the respective target chemical.

24. The process of any one of the above claims, wherein the target chemical is ethanol and the enzymes used for the conversion of pyruvate to ethanol are selected from the group consisting of Pyruvate decarboxylase PDC (EC 4.1.1.1), Zymomonas mobilis, Seq ID 20 and Alcohol dehydrogenase ADH (EC 1.1.1.1), Geobacillus stearothermophilus, Seq ID 18.

25. The process of any of claims 1 to 24, wherein the target chemical is isobutanol and the enzymes used for the conversion of pyruvate to isobutanol are selected from the group consisting of Acetolactate synthase ALS (EC 2.2.1.6), Bacillus subtilis, Seq ID 12; Acetolactate synthase ALS (EC 2.2.1.6), Sulfolobus solfataricus, NCBI-GenID: NP_342102.1; Acetolactate synthetase ALS (EC: 2.2.1.6), Thermotoga maritima, NCBI-GeneID: NP_228358.1; Ketol-acid reductoisomerase KARI (EC 1.1.1.86), Meiothermus ruber, Seq ID 14; Ketol-acid reductoisomerase KARI (EC 1.1.1.86), Sulfolobus solfataricus, NCBI-GenID: NP_342100.1; Ketol-acid reductoisomerase KARI (EC. 1.1.1.86), Thermotoga maritima, NCBI-GeneID: NP_228360.1; Branched-chain-2-oxo acid decarboxylase KDC (EC 4.1.1.72), Lactococcus lactis, Seq ID 16; α-Ketoisovalerate decarboxylase KDC, (EC 4.1.1.-), Lactococcus lactis, NCBI-GeneID: CAG34226.1; Dihydroxy acid dehydratase DHAD (EC 4.2.1.9), Sulfolobus solfataricus, NP 344419.1, Seq ID 04; Dihydroxy-acid dehydratase DHAD, (EC: 4.2.1.9), Thermotoga maritima, NCBI-GeneID: NP_228361.1; Alcohol dehydrogenase ADH (EC 1.1.1.1), Geobacillus stearothermophilus, Seq ID 18; Alcohol dehydrogenase ADH (EC 1.1.1.1), Flavobacterium frigidimaris, NCBI-GenID: BAB91411.1; and Alcohol dehydrogenase ADH (EC: 1.1.1.1), S. cerevisiae.

26. The process of any one of the above claims, wherein the product is removed from the reaction continuously or in a batch mode, preferably by extraction, perstraction, distillation, adsorption, gas stripping, pervaporation, membrane extraction or reverse osmosis.

27. The process of any one of the above claims, wherein the solvent tolerance of the used enzymes for the respective target chemical is preferably better than 1% (w/w), more preferably better than 4% (w/w), even more preferably better than 6% (w/w) and most preferably better than 10% (w/w).

28. The process of any one of the above claims, wherein the enzyme or enzymes involved in steps (1) and/or (3) is/are optimized for the single cofactor by having a higher specific activity to said cofactor.

29. The process of claim 28, wherein the optimized enzyme has a sequence identity of at least 50%, preferably 70%, more preferably 80%, even more preferably 90%, even more preferably 95%, most preferably 97%, most highly preferred 99% as compared to either SEQ ID NO. 8 or SEQ ID NO. 2 and has an improved specific activity to said cofactor as compared to SEQ ID NO. 8 or SEQ ID NO. 2, respectively.

30. The process of claim 28 or claim 29, wherein the enzyme has a specific activity of 0.4 U/mg or more to said cofactor at 50°C and pH 7.0 with glyceraldehyde/glycerate as substrates at 1 mM and the said cofactor at 2 mM in a total reaction volume of 0.2 ml.

31. The process of claim 30, wherein the specific activity is 0.6 U/mg or more, preferably 0.8 U/mg or more, more preferably 1.0 U/mg or more, most preferably 1.2 U/mg or more, and most highly preferred 1.5 U/mg or more.

32. The process of claim 31, wherein the specific activity is measured in the presence of 3% isobutanol.

33. The process of any one of the above claims, wherein the enzyme involved in steps (1) and/or (3) is aldehyde dehydrogenase.

34. The process of claim 33, wherein the aldehyde dehydrogenase belongs to the structural class EC 1.1.1.47.

35. The process of any one of the above claims, wherein the aldehyde dehydrogenase is selected from the group consisting of SEQ ID NO: 10, SEQ ID NO 57, SEQ ID NO 59, SEQ ID NO 61, SEQ ID NO 63, SEQ ID NO 65, SEQ ID NO 67, SEQ ID NO 69.
